# EUROPEAN PATENT APPLICATION

(11) **EP 4 056 596 A1**
(43) Date of publication of application: **14.09.2022**
(21) Application number: 21770764.5
(22) Date of filing: 19.03.2021
(51) Int. Cl.: C07K 19/00, C12N 15/62, C12N 15/63, A61K 38/17, A61K 39/395, A61P 35/00, G01N 33/574

(54) **BISPECIFIC FUSION PROTEIN AND APPLICATION THEREOF**

(30) Priority: 20.03.2020 CN 202010199036
(71) Applicant: RemeGen Co., Ltd., Shandong 264006 (CN)
(72) Inventor: FANG, Jianmin, Beijing Middle Road, Yantai Development Zone, Yantai District, China (Shandong) Pilot Free Trade Zone Yantai, Shandong 264006 (CN)
(74) Representative: HGF
(86) International application number: PCT/CN2021/081659
(87) International publication number: WO 2021/185337

(57) **Abstract**

Provided is a bispecific fusion protein having a novel structure. A second binding structural domain is inserted into an IgG hinged region in a full length by means of an optional peptide joint. The fusion protein has the same expression and production advantages as those of IgG, does not influence the binding activity of an Fab region of the fusion protein and further improves the stability and obtains a higher half life. In addition, the binding activity of the second binding structural domain to a target binding site is significantly improved with respect to the binding activity of a monomer corresponding to a soluble natural binding fragment to a corresponding target.

SEQUENCE LISTING

<110>  RemeGen Co., Ltd.

<120>  BISPECIFIC FUSION PROTEIN AND APPLICATION THEREOF

<130>  OP0021-EP-KR-SG-0084

<160>  137

<170>  PatentIn version 3.3

<210>  1
<211>  7
<212>  PRT
<213>  Artificial sequence

<220>
<223>  Heavy chain variable region CDR1

<400>  1

Gly Phe Ser Leu Ser Arg Tyr
1               5

<210>  2
<211>  10
<212>  PRT
<213>  Artificial sequence

<220>
<223>  Heavy chain variable region CDR1

<400>  2

Gly Phe Ser Leu Ser Arg Tyr Ser Val His
1               5                   10

<210>  3
<211>  5
<212>  PRT
<213>  Artificial sequence

<220>
<223>  Heavy chain variable region CDR1

<400>  3

Arg Tyr Ser Val His
1               5

<210>  4
<211>  6
<212>  PRT
<213>  Artificial sequence

<220>
<223>  Heavy chain variable region CDR1

<400>  4

Ser Arg Tyr Ser Val His
1               5


<210>  5
<211>  8
<212>  PRT
<213>  Artificial sequence

<220>
<223>  Heavy chain variable region CDR1

<400>  5

Gly Phe Ser Leu Ser Arg Tyr Ser
1               5


<210>  6
<211>  5
<212>  PRT
<213>  Artificial sequence

<220>
<223>  Heavy chain variable region CDR2

<400>  6

Trp Gly Val Gly Thr
1               5


<210>  7
<211>  9
<212>  PRT
<213>  Artificial sequence

<220>
<223>  Heavy chain variable region CDR2

<400>  7

Met Ile Trp Gly Val Gly Thr Thr Asp
1               5


<210>  8
<211>  16
<212>  PRT
<213>  Artificial sequence

<220>

<223>  Heavy chain variable region CDR2

<400>  8

Met Ile Trp Gly Val Gly Thr Thr Asp Tyr Asn Ser Ala Leu Lys Ser
1               5                   10                  15

<210>  9
<211>  12
<212>  PRT
<213>  Artificial sequence

<220>
<223>  Heavy chain variable region CDR2

<400>  9

Trp Leu Gly Met Ile Trp Gly Val Gly Thr Thr Asp
1               5                   10

<210>  10
<211>  7
<212>  PRT
<213>  Artificial sequence

<220>
<223>  Heavy chain variable region CDR2

<400>  10

Ile Trp Gly Val Gly Thr Thr
1               5

<210>  11
<211>  10
<212>  PRT
<213>  Artificial sequence

<220>
<223>  Heavy chain variable region CDR3

<400>  11

Asn Trp Gly Thr Ala Asp Tyr Phe Asp Tyr
1               5                   10

<210>  12
<211>  10
<212>  PRT
<213>  Artificial sequence

<220>
<223>  Heavy chain variable region CDR3

<400>  12

Asn Trp Gly Thr Ala Asp Tyr Phe Asp Tyr
1               5                   10



<210>  13
<211>  10
<212>  PRT
<213>  Artificial sequence

<220>
<223>  Heavy chain variable region CDR3

<400>  13

Asn Trp Gly Thr Ala Asp Tyr Phe Asp Tyr
1               5                   10



<210>  14
<211>  11
<212>  PRT
<213>  Artificial sequence

<220>
<223>  Heavy chain variable region CDR3

<400>  14

Ala Arg Asn Trp Gly Thr Ala Asp Tyr Phe Asp
1               5                   10



<210>  15
<211>  12
<212>  PRT
<213>  Artificial sequence

<220>
<223>  Heavy chain variable region CDR3

<400>  15

Ala Arg Asn Trp Gly Thr Ala Asp Tyr Phe Asp Tyr
1               5                   10



<210>  16
<211>  15
<212>  PRT
<213>  Artificial sequence

<220>
<223>  Light chain variable region CDR1

<400>  16

Arg Ala Ser Lys Ser Val His Thr Ser Gly Tyr Ser Tyr Met His
1               5                   10                  15


<210>  17
<211>  15
<212>  PRT
<213>  Artificial sequence

<220>
<223>  Light chain variable region CDR1

<400>  17

Arg Ala Ser Lys Ser Val His Thr Ser Gly Tyr Ser Tyr Met His
1               5                   10                  15


<210>  18
<211>  15
<212>  PRT
<213>  Artificial sequence

<220>
<223>  Light chain variable region CDR1

<400>  18

Arg Ala Ser Lys Ser Val His Thr Ser Gly Tyr Ser Tyr Met His
1               5                   10                  15


<210>  19
<211>  11
<212>  PRT
<213>  Artificial sequence

<220>
<223>  Light chain variable region CDR1

<400>  19

His Thr Ser Gly Tyr Ser Tyr Met His Trp Tyr
1               5                   10


<210>  20
<211>  10
<212>  PRT
<213>  Artificial sequence

<220>
<223>  Light chain variable region CDR1

<400>  20

Lys Ser Val His Thr Ser Gly Tyr Ser Tyr
1               5                   10

<210>  21
<211>  7
<212>  PRT
<213>  Artificial sequence

<220>
<223>  Light chain variable region CDR2

<400>  21

Leu Ala Ser Asn Leu Glu Ser
1               5


<210>  22
<211>  7
<212>  PRT
<213>  Artificial sequence

<220>
<223>  Light chain variable region CDR2

<400>  22

Leu Ala Ser Asn Leu Glu Ser
1               5


<210>  23
<211>  7
<212>  PRT
<213>  Artificial sequence

<220>
<223>  Light chain variable region CDR2

<400>  23

Leu Ala Ser Asn Leu Glu Ser
1               5


<210>  24
<211>  10
<212>  PRT
<213>  Artificial sequence

<220>
<223>  Light chain variable region CDR2

<400>  24

Leu Leu Ile Tyr Leu Ala Ser Asn Leu Glu
1               5                   10

<210>  25
<211>  3
<212>  PRT
<213>  Artificial sequence

<220>
<223>  Light chain variable region CDR2

<400>  25

Leu Ala Ser
1


<210>  26
<211>  9
<212>  PRT
<213>  Artificial sequence

<220>
<223>  Light chain variable region CDR3

<400>  26

Gln His Ser Gly Glu Leu Pro Tyr Thr
1               5


<210>  27
<211>  9
<212>  PRT
<213>  Artificial sequence

<220>
<223>  Light chain variable region CDR3

<400>  27

Gln His Ser Gly Glu Leu Pro Tyr Thr
1               5


<210>  28
<211>  9
<212>  PRT
<213>  Artificial sequence

<220>
<223>  Light chain variable region CDR3

<400>  28

Gln His Ser Gly Glu Leu Pro Tyr Thr
1               5


<210>  29
<211>  8

<212>  PRT
<213>  Artificial sequence

<220>
<223>  Light chain variable region CDR3

<400>  29

Gln His Ser Gly Glu Leu Pro Tyr
1               5


<210>  30
<211>  9
<212>  PRT
<213>  Artificial sequence

<220>
<223>  Light chain variable region CDR3

<400>  30

Gln His Ser Gly Glu Leu Pro Tyr Thr
1               5


<210>  31
<211>  137
<212>  PRT
<213>  Artificial sequence

<220>
<223>  Human TGF-βRII

<400>  31

Thr Ile Pro Pro His Val Gln Lys Ser Val Asn Asn Asp Met Ile Val
1               5                   10                  15


Thr Asp Asn Asn Gly Ala Val Lys Phe Pro Gln Leu Cys Lys Phe Cys
            20                  25                  30


Asp Val Arg Phe Ser Thr Cys Asp Asn Gln Lys Ser Cys Met Ser Asn
        35                  40                  45


Cys Ser Ile Thr Ser Ile Cys Glu Lys Pro Gln Glu Val Cys Val Ala
    50                  55                  60


Val Trp Arg Lys Asn Asp Glu Asn Ile Thr Leu Glu Thr Val Cys His
65                  70                  75                  80


Asp Pro Lys Leu Pro Tyr His Asp Phe Ile Leu Glu Asp Ala Ala Ser
                85                  90                  95

Pro Lys Cys Ile Met Lys Glu Lys Lys Lys Pro Gly Glu Thr Phe Phe
            100                 105                 110

Met Cys Ser Cys Ser Ser Asp Glu Cys Asn Asp Asn Ile Ile Phe Ser
        115                 120                 125

Glu Glu Tyr Asn Thr Ser Asn Pro Asp
    130                 135

<210>  32
<211>  198
<212>  PRT
<213>  Artificial sequence

<220>
<223>  CD80  ECD

<400>  32

Val Ile His Val Thr Lys Glu Val Lys Glu Val Ala Thr Leu Ser Cys
1               5                   10                  15

Gly His Asn Val Ser Val Glu Glu Leu Ala Gln Thr Arg Ile Tyr Trp
            20                  25                  30

Gln Lys Glu Lys Lys Met Val Leu Thr Met Met Ser Gly Asp Met Asn
        35                  40                  45

Ile Trp Pro Glu Tyr Lys Asn Arg Thr Ile Phe Asp Ile Thr Asn Asn
    50                  55                  60

Leu Ser Ile Val Ile Leu Ala Leu Arg Pro Ser Asp Glu Gly Thr Tyr
65                  70                  75                  80

Glu Cys Val Val Leu Lys Tyr Glu Lys Asp Ala Phe Lys Arg Glu His
                85                  90                  95

Leu Ala Glu Val Thr Leu Ser Val Lys Ala Asp Phe Pro Thr Pro Ser
            100                 105                 110

Ile Ser Asp Phe Glu Ile Pro Thr Ser Asn Ile Arg Arg Ile Ile Cys
        115                 120                 125

Ser Thr Ser Gly Gly Phe Pro Glu Pro His Leu Ser Trp Leu Glu Asn
    130                 135                 140

Gly Glu Glu Leu Asn Ala Ile Asn Thr Thr Val Ser Gln Asp Pro Glu
145                 150                 155                 160

Thr Glu Leu Tyr Ala Val Ser Ser Lys Leu Asp Phe Asn Met Thr Thr
                165                 170                 175

Asn His Ser Phe Met Cys Leu Ile Lys Tyr Gly His Leu Arg Val Asn
            180                 185                 190

Gln Thr Phe Asn Trp Asn
        195

<210>  33
<211>  202
<212>  PRT
<213>  Artificial sequence

<220>
<223>  VEGFR

<400>  33

Gly Arg Pro Phe Val Glu Met Tyr Ser Glu Ile Pro Glu Ile Ile His
1               5                   10                  15

Met Thr Glu Gly Arg Glu Leu Val Ile Pro Cys Arg Val Thr Ser Pro
            20                  25                  30

Asn Ile Thr Val Thr Leu Lys Lys Phe Pro Leu Asp Thr Leu Ile Pro
        35                  40                  45

Asp Gly Lys Arg Ile Ile Trp Asp Ser Arg Lys Gly Phe Ile Ile Ser
    50                  55                  60

Asn Ala Thr Tyr Lys Glu Ile Gly Leu Leu Thr Cys Glu Ala Thr Val
65                  70                  75                  80

Asn Gly His Leu Tyr Lys Thr Asn Tyr Leu Thr His Arg Gln Thr Asn
                85                  90                  95

Thr Ile Ile Asp Val Val Leu Ser Pro Ser His Gly Ile Glu Leu Ser
            100                 105                 110

Val Gly Glu Lys Leu Val Leu Asn Cys Thr Ala Arg Thr Glu Leu Asn
        115                 120                 125

Val Gly Ile Asp Phe Asn Trp Glu Tyr Pro Ser Ser Lys His Gln His
    130                 135                 140

Lys Lys Leu Val Asn Arg Asp Leu Lys Thr Gln Ser Gly Ser Glu Met
145                 150                 155                 160

Lys Lys Phe Leu Ser Thr Leu Thr Ile Asp Gly Val Thr Arg Ser Asp
                165                 170                 175

Gln Gly Leu Tyr Thr Cys Ala Ala Ser Ser Gly Leu Met Thr Lys Lys
            180                 185                 190

Asn Ser Thr Phe Val Arg Val His Glu Pro
        195                 200

<210>  34
<211>  7
<212>  PRT
<213>  Artificial sequence

<220>
<223>  Heavy chain variable region CDR1

<400>  34

Gly Ile Thr Phe Ser Asn Ser
1               5

<210>  35
<211>  10
<212>  PRT
<213>  Artificial sequence

<220>
<223>  Heavy chain variable region CDR1

<400>  35

Gly Ile Thr Phe Ser Asn Ser Gly Met His
1               5                   10

<210>  36
<211>  5
<212>  PRT
<213>  Artificial sequence

<220>
<223>  Heavy chain variable region CDR1

<400>  36

Asn Ser Gly Met His
1               5



<210>  37
<211>  6
<212>  PRT
<213>  Artificial sequence

<220>
<223>  Heavy chain variable region CDR1

<400>  37

Ser Asn Ser Gly Met His
1               5



<210>  38
<211>  8
<212>  PRT
<213>  Artificial sequence

<220>
<223>  Heavy chain variable region CDR1

<400>  38

Gly Ile Thr Phe Ser Asn Ser Gly
1               5



<210>  39
<211>  6
<212>  PRT
<213>  Artificial sequence

<220>
<223>  Heavy chain variable region CDR2

<400>  39

Trp Tyr Asp Gly Ser Lys
1               5



<210>  40
<211>  10
<212>  PRT
<213>  Artificial sequence

<220>
<223>  Heavy chain variable region CDR2

<400>  40

Val Ile Trp Tyr Asp Gly Ser Lys Arg Tyr
1               5                   10

<210>  41
<211>  17
<212>  PRT
<213>  Artificial sequence

<220>
<223>  Heavy chain variable region CDR2

<400>  41

Val Ile Trp Tyr Asp Gly Ser Lys Arg Tyr Tyr Ala Asp Ser Val Lys
1               5                   10                  15

Gly

<210>  42
<211>  13
<212>  PRT
<213>  Artificial sequence

<220>
<223>  Heavy chain variable region CDR2

<400>  42

Trp Val Ala Val Ile Trp Tyr Asp Gly Ser Lys Arg Tyr
1               5                   10

<210>  43
<211>  8
<212>  PRT
<213>  Artificial sequence

<220>
<223>  Heavy chain variable region CDR2

<400>  43

Ile Trp Tyr Asp Gly Ser Lys Arg
1               5

<210>  44
<211>  4
<212>  PRT
<213>  Artificial sequence

<220>
<223>  Heavy chain variable region CDR3

<400>  44

Asn Asp Asp Tyr
1



<210>  45
<211>  4
<212>  PRT
<213>  Artificial sequence

<220>
<223>  Heavy chain variable region CDR3

<400>  45

Asn Asp Asp Tyr
1



<210>  46
<211>  4
<212>  PRT
<213>  Artificial sequence

<220>
<223>  Heavy chain variable region CDR3

<400>  46

Asn Asp Asp Tyr
1



<210>  47
<211>  5
<212>  PRT
<213>  Artificial sequence

<220>
<223>  Heavy chain variable region CDR3

<400>  47

Ala Thr Asn Asp Asp
1               5



<210>  48
<211>  6
<212>  PRT
<213>  Artificial sequence

<220>
<223>  Heavy chain variable region CDR3

<400>  48

Ala Thr Asn Asp Asp Tyr
1               5

<210>  49
<211>  11
<212>  PRT
<213>  Artificial sequence

<220>
<223>  Light chain variable region CDR1

<400>  49

Arg Ala Ser Gln Ser Val Ser Ser Tyr Leu Ala
1               5                   10

<210>  50
<211>  11
<212>  PRT
<213>  Artificial sequence

<220>
<223>  Light chain variable region CDR1

<400>  50

Arg Ala Ser Gln Ser Val Ser Ser Tyr Leu Ala
1               5                   10

<210>  51
<211>  11
<212>  PRT
<213>  Artificial sequence

<220>
<223>  Light chain variable region CDR1

<400>  51

Arg Ala Ser Gln Ser Val Ser Ser Tyr Leu Ala
1               5                   10

<210>  52
<211>  7
<212>  PRT
<213>  Artificial sequence

<220>
<223>  Light chain variable region CDR1

<400>  52

Ser Ser Tyr Leu Ala Trp Tyr
1               5

<210>  53
<211>  6
<212>  PRT
<213>  Artificial sequence

<220>
<223>  Light chain variable region CDR1

<400>  53

Gln Ser Val Ser Ser Tyr
1               5


<210>  54
<211>  7
<212>  PRT
<213>  Artificial sequence

<220>
<223>  Light chain variable region CDR2

<400>  54

Asp Ala Ser Asn Arg Ala Thr
1               5


<210>  55
<211>  7
<212>  PRT
<213>  Artificial sequence

<220>
<223>  Light chain variable region CDR2

<400>  55

Asp Ala Ser Asn Arg Ala Thr
1               5


<210>  56
<211>  7
<212>  PRT
<213>  Artificial sequence

<220>
<223>  Light chain variable region CDR2

<400>  56

Asp Ala Ser Asn Arg Ala Thr
1               5

<210>  57
<211>  10
<212>  PRT
<213>  Artificial sequence

<220>
<223>  Light chain variable region CDR2

<400>  57

Leu Leu Ile Tyr Asp Ala Ser Asn Arg Ala
1               5                   10


<210>  58
<211>  3
<212>  PRT
<213>  Artificial sequence

<220>
<223>  Light chain variable region CDR2

<400>  58

Asp Ala Ser
1


<210>  59
<211>  9
<212>  PRT
<213>  Artificial sequence

<220>
<223>  Light chain variable region CDR3

<400>  59

Gln Gln Ser Ser Asn Trp Pro Arg Thr
1               5


<210>  60
<211>  9
<212>  PRT
<213>  Artificial sequence

<220>
<223>  Light chain variable region CDR3

<400>  60

Gln Gln Ser Ser Asn Trp Pro Arg Thr
1               5


<210>  61
<211>  9

<212>  PRT
<213>  Artificial sequence

<220>
<223>  Light chain variable region CDR3

<400>  61

Gln Gln Ser Ser Asn Trp Pro Arg Thr
1               5


<210>  62
<211>  8
<212>  PRT
<213>  Artificial sequence

<220>
<223>  Light chain variable region CDR3

<400>  62

Gln Gln Ser Ser Asn Trp Pro Arg
1               5


<210>  63
<211>  9
<212>  PRT
<213>  Artificial sequence

<220>
<223>  Light chain variable region CDR3

<400>  63

Gln Gln Ser Ser Asn Trp Pro Arg Thr
1               5


<210>  64
<211>  113
<212>  PRT
<213>  Artificial sequence

<220>
<223>  Heavy chain variable region amino acid sequence

<400>  64

<210> 65
<211> 107
<212> PRT
<213> Artificial sequence

<220>
<223> Light chain variable region amino acid sequence

<400> 65

<210>  66
<211>  120
<212>  PRT
<213>  Artificial sequence

<220>
<223>  Heavy chain variable region amino acid sequence

<400>  66

Gln Val Gln Leu Gln Glu Ser Gly Pro Gly Leu Val Lys Pro Ser Glu
1               5                   10                  15

Thr Leu Ser Leu Thr Cys Thr Val Ser Gly Phe Ser Leu Ser Arg Tyr
            20                  25                  30

Ser Val His Trp Ile Arg Gln Pro Pro Gly Lys Gly Leu Glu Trp Leu
        35                  40                  45

Gly Met Ile Trp Gly Val Gly Thr Thr Asp Tyr Asn Ser Ala Leu Lys
    50                  55                  60

Ser Arg Leu Thr Ile Ser Lys Asp Thr Ser Lys Asn Gln Phe Ser Leu
65                  70                  75                  80

Lys Leu Ser Ser Val Thr Ala Ala Asp Thr Ala Val Tyr Tyr Cys Ala
                85                  90                  95

Arg Asn Trp Gly Thr Ala Asp Tyr Phe Asp Tyr Trp Gly Gln Gly Thr
            100                 105                 110

Thr Val Thr Val Ser Ser Ala Ser
        115                 120

<210>  67
<211>  113
<212>  PRT
<213>  Artificial sequence

<220>
<223>  Light chain variable region amino acid sequence

<400>  67

Asp Ile Val Leu Thr Gln Ser Pro Ala Ser Leu Ala Val Ser Pro Gly
1               5                   10                  15

Gln Arg Ala Thr Ile Thr Cys Arg Ala Ser Lys Ser Val His Thr Ser
            20                  25                  30

Gly Tyr Ser Tyr Met His Trp Tyr Gln Gln Lys Pro Gly Gln Pro Pro
        35                  40                  45

Lys Leu Leu Ile Tyr Leu Ala Ser Asn Leu Glu Ser Gly Val Pro Ala
    50                  55                  60

Arg Phe Ser Gly Ser Gly Ser Gly Thr Asp Phe Thr Leu Thr Ile Asn
65                  70                  75                  80

Pro Val Glu Ala Asn Asp Thr Ala Asn Tyr Tyr Cys Gln His Ser Gly
                85                  90                  95

Glu Leu Pro Tyr Thr Phe Gly Gly Gly Thr Lys Val Glu Ile Lys Arg
            100                 105                 110

Thr

<210> 68
<211> 668
<212> PRT
<213> Artificial sequence

<220>
<223> Hib-PDC heavy chain amino acid sequence

<400> 68

Gln Val Gln Leu Val Glu Ser Gly Gly Gly Val Val Gln Pro Gly Arg
1               5                   10                  15

Ser Leu Arg Leu Asp Cys Lys Ala Ser Gly Ile Thr Phe Ser Asn Ser
            20                  25                  30

Gly Met His Trp Val Arg Gln Ala Pro Gly Lys Gly Leu Glu Trp Val
        35                  40                  45

Ala Val Ile Trp Tyr Asp Gly Ser Lys Arg Tyr Tyr Ala Asp Ser Val
    50                  55                  60

Lys Gly Arg Phe Thr Ile Ser Arg Asp Asn Ser Lys Asn Thr Leu Phe
65                  70                  75                  80

Leu Gln Met Asn Ser Leu Arg Ala Glu Asp Thr Ala Val Tyr Tyr Cys
                85                  90                  95

Ala Thr Asn Asp Asp Tyr Trp Gly Gln Gly Thr Leu Val Thr Val Ser
            100                 105                 110

Ser Ala Ser Thr Lys Gly Pro Ser Val Phe Pro Leu Ala Pro Cys Ser
        115                 120                 125

Arg Ser Thr Ser Glu Ser Thr Ala Ala Leu Gly Cys Leu Val Lys Asp
    130                 135                 140

Tyr Phe Pro Glu Pro Val Thr Val Ser Trp Asn Ser Gly Ala Leu Thr
145                 150                 155                 160

Ser Gly Val His Thr Phe Pro Ala Val Leu Gln Ser Ser Gly Leu Tyr
                165                 170                 175

Ser Leu Ser Ser Val Val Thr Val Pro Ser Ser Ser Leu Gly Thr Lys
            180                 185                 190

Thr Tyr Thr Cys Asn Val Asp His Lys Pro Ser Asn Thr Lys Val Asp
        195                 200                 205

Lys Arg Val Glu Ser Gly Gly Gly Gly Ser Gly Gly Gly Gly Ser Gly
    210                 215                 220

Gly Gly Gly Ser Val Ile His Val Thr Lys Glu Val Lys Glu Val Ala
225                 230                 235                 240

Thr Leu Ser Cys Gly His Asn Val Ser Val Glu Glu Leu Ala Gln Thr
                245                 250                 255

Arg Ile Tyr Trp Gln Lys Glu Lys Lys Met Val Leu Thr Met Met Ser
            260                 265                 270

Gly Asp Met Asn Ile Trp Pro Glu Tyr Lys Asn Arg Thr Ile Phe Asp
        275                 280                 285

Ile Thr Asn Asn Leu Ser Ile Val Ile Leu Ala Leu Arg Pro Ser Asp
    290                 295                 300

Glu Gly Thr Tyr Glu Cys Val Val Leu Lys Tyr Glu Lys Asp Ala Phe
305                 310                 315                 320

Lys Arg Glu His Leu Ala Glu Val Thr Leu Ser Val Lys Ala Asp Phe
                325                 330                 335

Pro Thr Pro Ser Ile Ser Asp Phe Glu Ile Pro Thr Ser Asn Ile Arg
            340                 345                 350

Arg Ile Ile Cys Ser Thr Ser Gly Gly Phe Pro Glu Pro His Leu Ser
        355                 360                 365

Trp Leu Glu Asn Gly Glu Glu Leu Asn Ala Ile Asn Thr Thr Val Ser
    370                 375                 380

Gln Asp Pro Glu Thr Glu Leu Tyr Ala Val Ser Ser Lys Leu Asp Phe
385                 390                 395                 400

Asn Met Thr Thr Asn His Ser Phe Met Cys Leu Ile Lys Tyr Gly His
                405                 410                 415

Leu Arg Val Asn Gln Thr Phe Asn Trp Asn Gly Gly Gly Gly Ser Gly
            420                 425                 430

Gly Gly Gly Ser Gly Gly Gly Gly Ser Lys Tyr Gly Pro Pro Cys Pro
        435                 440                 445

Pro Cys Pro Ala Pro Glu Phe Leu Gly Gly Pro Ser Val Phe Leu Phe
    450                 455                 460

Pro Pro Lys Pro Lys Asp Thr Leu Met Ile Ser Arg Thr Pro Glu Val
465                 470                 475                 480

Thr Cys Val Val Val Asp Val Ser Gln Glu Asp Pro Glu Val Gln Phe
                485                 490                 495

Asn Trp Tyr Val Asp Gly Val Glu Val His Asn Ala Lys Thr Lys Pro
            500                 505                 510

Arg Glu Glu Gln Phe Asn Ser Thr Tyr Arg Val Val Ser Val Leu Thr
        515                 520                 525

Val Leu His Gln Asp Trp Leu Asn Gly Lys Glu Tyr Lys Cys Lys Val
    530                 535                 540

Ser Asn Lys Gly Leu Pro Ser Ser Ile Glu Lys Thr Ile Ser Lys Ala
545                 550                 555                 560

Lys Gly Gln Pro Arg Glu Pro Gln Val Tyr Thr Leu Pro Pro Ser Gln
                565                 570                 575

Glu Glu Met Thr Lys Asn Gln Val Ser Leu Thr Cys Leu Val Lys Gly
            580                 585                 590

Phe Tyr Pro Ser Asp Ile Ala Val Glu Trp Glu Ser Asn Gly Gln Pro
        595                 600                 605

Glu Asn Asn Tyr Lys Thr Thr Pro Pro Val Leu Asp Ser Asp Gly Ser
    610                 615                 620

Phe Phe Leu Tyr Ser Arg Leu Thr Val Asp Lys Ser Arg Trp Gln Glu
625                 630                 635                 640

Gly Asn Val Phe Ser Cys Ser Val Met His Glu Ala Leu His Asn His
                645                 650                 655

Tyr Thr Gln Lys Ser Leu Ser Leu Ser Leu Gly Lys
            660                 665


<210>  69
<211>  214
<212>  PRT
<213>  Artificial sequence

<220>
<223>  Hib-PDC light chain amino acid sequence

<400>  69

Glu Ile Val Leu Thr Gln Ser Pro Ala Thr Leu Ser Leu Ser Pro Gly
1               5                   10                  15

Glu Arg Ala Thr Leu Ser Cys Arg Ala Ser Gln Ser Val Ser Ser Tyr
            20                  25                  30

Leu Ala Trp Tyr Gln Gln Lys Pro Gly Gln Ala Pro Arg Leu Leu Ile
        35                  40                  45

Tyr Asp Ala Ser Asn Arg Ala Thr Gly Ile Pro Ala Arg Phe Ser Gly
    50                  55                  60

Ser Gly Ser Gly Thr Asp Phe Thr Leu Thr Ile Ser Ser Leu Glu Pro
65                  70                  75                  80

Glu Asp Phe Ala Val Tyr Tyr Cys Gln Gln Ser Ser Asn Trp Pro Arg
                85                  90                  95

Thr Phe Gly Gln Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210>  70
<211>  672
<212>  PRT
<213>  Artificial sequence

<220>
<223>  Hib-PDV heavy chain amino acid sequence

<400>  70

Gln Val Gln Leu Val Glu Ser Gly Gly Gly Val Val Gln Pro Gly Arg
1               5                   10                  15

Ser Leu Arg Leu Asp Cys Lys Ala Ser Gly Ile Thr Phe Ser Asn Ser
            20                  25                  30

Gly Met His Trp Val Arg Gln Ala Pro Gly Lys Gly Leu Glu Trp Val
        35                  40                  45

Ala Val Ile Trp Tyr Asp Gly Ser Lys Arg Tyr Tyr Ala Asp Ser Val
    50                  55                  60

Lys Gly Arg Phe Thr Ile Ser Arg Asp Asn Ser Lys Asn Thr Leu Phe
65                  70                  75                  80

Leu Gln Met Asn Ser Leu Arg Ala Glu Asp Thr Ala Val Tyr Tyr Cys
                85                  90                  95

Ala Thr Asn Asp Asp Tyr Trp Gly Gln Gly Thr Leu Val Thr Val Ser
            100                 105                 110

Ser Ala Ser Thr Lys Gly Pro Ser Val Phe Pro Leu Ala Pro Cys Ser
        115                 120                 125

Arg Ser Thr Ser Glu Ser Thr Ala Ala Leu Gly Cys Leu Val Lys Asp
    130                 135                 140

Tyr Phe Pro Glu Pro Val Thr Val Ser Trp Asn Ser Gly Ala Leu Thr
145                 150                 155                 160

Ser Gly Val His Thr Phe Pro Ala Val Leu Gln Ser Ser Gly Leu Tyr
                165                 170                 175

Ser Leu Ser Ser Val Val Thr Val Pro Ser Ser Ser Leu Gly Thr Lys
            180                 185                 190

Thr Tyr Thr Cys Asn Val Asp His Lys Pro Ser Asn Thr Lys Val Asp
        195                 200                 205

Lys Arg Val Glu Ser Gly Gly Gly Gly Ser Gly Gly Gly Gly Ser Gly
    210                 215                 220

Gly Gly Gly Ser Gly Arg Pro Phe Val Glu Met Tyr Ser Glu Ile Pro
225                 230                 235                 240

Glu Ile Ile His Met Thr Glu Gly Arg Glu Leu Val Ile Pro Cys Arg
                245                 250                 255

Val Thr Ser Pro Asn Ile Thr Val Thr Leu Lys Lys Phe Pro Leu Asp
        260                 265                 270

Thr Leu Ile Pro Asp Gly Lys Arg Ile Ile Trp Asp Ser Arg Lys Gly
    275                 280                 285

Phe Ile Ile Ser Asn Ala Thr Tyr Lys Glu Ile Gly Leu Leu Thr Cys
    290                 295                 300

Glu Ala Thr Val Asn Gly His Leu Tyr Lys Thr Asn Tyr Leu Thr His
305                 310                 315                 320

Arg Gln Thr Asn Thr Ile Ile Asp Val Val Leu Ser Pro Ser His Gly
                325                 330                 335

Ile Glu Leu Ser Val Gly Glu Lys Leu Val Leu Asn Cys Thr Ala Arg
        340                 345                 350

Thr Glu Leu Asn Val Gly Ile Asp Phe Asn Trp Glu Tyr Pro Ser Ser
    355                 360                 365

Lys His Gln His Lys Lys Leu Val Asn Arg Asp Leu Lys Thr Gln Ser
    370                 375                 380

Gly Ser Glu Met Lys Lys Phe Leu Ser Thr Leu Thr Ile Asp Gly Val
385                 390                 395                 400

Thr Arg Ser Asp Gln Gly Leu Tyr Thr Cys Ala Ala Ser Ser Gly Leu
                405                 410                 415

Met Thr Lys Lys Asn Ser Thr Phe Val Arg Val His Glu Pro Gly Gly
        420                 425                 430

Gly Gly Ser Gly Gly Gly Gly Ser Gly Gly Gly Gly Ser Lys Tyr Gly
    435                 440                 445

Pro Pro Cys Pro Pro Cys Pro Ala Pro Glu Phe Leu Gly Gly Pro Ser
    450                 455                 460

Val Phe Leu Phe Pro Pro Lys Pro Lys Asp Thr Leu Met Ile Ser Arg
465                 470                 475                 480

Thr Pro Glu Val Thr Cys Val Val Val Asp Val Ser Gln Glu Asp Pro
                485                 490                 495

Glu Val Gln Phe Asn Trp Tyr Val Asp Gly Val Glu Val His Asn Ala
            500                 505                 510

Lys Thr Lys Pro Arg Glu Glu Gln Phe Asn Ser Thr Tyr Arg Val Val
        515                 520                 525

Ser Val Leu Thr Val Leu His Gln Asp Trp Leu Asn Gly Lys Glu Tyr
    530                 535                 540

Lys Cys Lys Val Ser Asn Lys Gly Leu Pro Ser Ser Ile Glu Lys Thr
545                 550                 555                 560

Ile Ser Lys Ala Lys Gly Gln Pro Arg Glu Pro Gln Val Tyr Thr Leu
                565                 570                 575

Pro Pro Ser Gln Glu Glu Met Thr Lys Asn Gln Val Ser Leu Thr Cys
            580                 585                 590

Leu Val Lys Gly Phe Tyr Pro Ser Asp Ile Ala Val Glu Trp Glu Ser
        595                 600                 605

Asn Gly Gln Pro Glu Asn Asn Tyr Lys Thr Thr Pro Pro Val Leu Asp
    610                 615                 620

Ser Asp Gly Ser Phe Phe Leu Tyr Ser Arg Leu Thr Val Asp Lys Ser
625                 630                 635                 640

Arg Trp Gln Glu Gly Asn Val Phe Ser Cys Ser Val Met His Glu Ala
                645                 650                 655

Leu His Asn His Tyr Thr Gln Lys Ser Leu Ser Leu Ser Leu Gly Lys
            660                 665                 670

<210> 71
<211> 214
<212> PRT
<213> Artificial sequence

<220>
<223> Hib-PDV light chain amino acid sequence

<400> 71

Glu Ile Val Leu Thr Gln Ser Pro Ala Thr Leu Ser Leu Ser Pro Gly
1               5                   10                  15

Glu Arg Ala Thr Leu Ser Cys Arg Ala Ser Gln Ser Val Ser Ser Tyr
            20                  25                  30

Leu Ala Trp Tyr Gln Gln Lys Pro Gly Gln Ala Pro Arg Leu Leu Ile
        35                  40                  45

Tyr Asp Ala Ser Asn Arg Ala Thr Gly Ile Pro Ala Arg Phe Ser Gly
    50                  55                  60

Ser Gly Ser Gly Thr Asp Phe Thr Leu Thr Ile Ser Ser Leu Glu Pro
65                  70                  75                  80

Glu Asp Phe Ala Val Tyr Tyr Cys Gln Gln Ser Ser Asn Trp Pro Arg
                85                  90                  95

Thr Phe Gly Gln Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210>  72
<211>  615
<212>  PRT
<213>  Artificial sequence

<220>
<223>  Hib-PLT heavy chain amino acid sequence

<400>  72

Gln Val Gln Leu Gln Glu Ser Gly Pro Gly Leu Val Lys Pro Ser Glu
1               5                   10                  15

Thr Leu Ser Leu Thr Cys Thr Val Ser Gly Phe Ser Leu Ser Arg Tyr
            20                  25                  30

Ser Val His Trp Ile Arg Gln Pro Pro Gly Lys Gly Leu Glu Trp Leu
        35                  40                  45

Gly Met Ile Trp Gly Val Gly Thr Thr Asp Tyr Asn Ser Ala Leu Lys
    50                  55                  60

Ser Arg Leu Thr Ile Ser Lys Asp Thr Ser Lys Asn Gln Phe Ser Leu
65                  70                  75                  80

Lys Leu Ser Ser Val Thr Ala Ala Asp Thr Ala Val Tyr Tyr Cys Ala
                85                  90                  95

Arg Asn Trp Gly Thr Ala Asp Tyr Phe Asp Tyr Trp Gly Gln Gly Thr
            100                 105                 110

Thr Val Thr Val Ser Ser Ala Ser Thr Lys Gly Pro Ser Val Phe Pro
        115                 120                 125

Leu Ala Pro Ser Ser Lys Ser Thr Ser Gly Gly Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Gln Thr Tyr Ile Cys Asn Val Asn His Lys Pro Ser
        195                 200                 205

Asn Thr Lys Val Asp Lys Lys Val Glu Pro Lys Ser Cys Asp Lys Gly
    210                 215                 220

Gly Gly Gly Ser Gly Gly Gly Gly Ser Gly Gly Gly Gly Ser Thr Ile
225                 230                 235                 240

Pro Pro His Val Gln Lys Ser Val Asn Asn Asp Met Ile Val Thr Asp
                245                 250                 255

Asn Asn Gly Ala Val Lys Phe Pro Gln Leu Cys Lys Phe Cys Asp Val
            260                 265                 270

Arg Phe Ser Thr Cys Asp Asn Gln Lys Ser Cys Met Ser Asn Cys Ser
        275                 280                 285

Ile Thr Ser Ile Cys Glu Lys Pro Gln Glu Val Cys Val Ala Val Trp
    290                 295                 300

Arg Lys Asn Asp Glu Asn Ile Thr Leu Glu Thr Val Cys His Asp Pro
305                 310                 315                 320

Lys Leu Pro Tyr His Asp Phe Ile Leu Glu Asp Ala Ala Ser Pro Lys
                325                 330                 335

Cys Ile Met Lys Glu Lys Lys Lys Pro Gly Glu Thr Phe Phe Met Cys
            340                 345                 350

Ser Cys Ser Ser Asp Glu Cys Asn Asp Asn Ile Ile Phe Ser Glu Glu
        355                 360                 365

Tyr Asn Thr Ser Asn Pro Asp Gly Gly Gly Gly Ser Gly Gly Gly Gly
    370                 375                 380

Ser Gly Gly Gly Gly Ser Thr His Thr Cys Pro Pro Cys Pro Ala Pro
385                 390                 395                 400

Glu Leu Leu Gly Gly Pro Ser Val Phe Leu Phe Pro Pro Lys Pro Lys
                405                 410                 415

Asp Thr Leu Met Ile Ser Arg Thr Pro Glu Val Thr Cys Val Val Val
            420                 425                 430

Asp Val Ser His Glu Asp Pro Glu Val Lys Phe Asn Trp Tyr Val Asp
        435                 440                 445

Gly Val Glu Val His Asn Ala Lys Thr Lys Pro Arg Glu Glu Gln Tyr
    450                 455                 460

Asn Ser Thr Tyr Arg Val Val Ser Val Leu Thr Val Leu His Gln Asp
465                 470                 475                 480

Trp Leu Asn Gly Lys Glu Tyr Lys Cys Lys Val Ser Asn Lys Ala Leu
                485                 490                 495

Pro Ala Pro Ile Glu Lys Thr Ile Ser Lys Ala Lys Gly Gln Pro Arg
            500                 505                 510

Glu Pro Gln Val Tyr Thr Leu Pro Pro Ser Arg Glu Glu Met Thr Lys
        515                 520                 525

Asn Gln Val Ser Leu Thr Cys Leu Val Lys Gly Phe Tyr Pro Ser Asp
    530                 535                 540

Ile Ala Val Glu Trp Glu Ser Asn Gly Gln Pro Glu Asn Asn Tyr Lys
545                 550                 555                 560

Thr Thr Pro Pro Val Leu Asp Ser Asp Gly Ser Phe Phe Leu Tyr Ser
                565                 570                 575

Lys Leu Thr Val Asp Lys Ser Arg Trp Gln Gln Gly Asn Val Phe Ser
            580                 585                 590

Cys Ser Val Met His Glu Ala Leu His Asn His Tyr Thr Gln Lys Ser
        595                 600                 605

Leu Ser Leu Ser Pro Gly Lys
    610                 615

<210>  73
<211>  218
<212>  PRT
<213>  Artificial sequence

<220>
<223>  Hib-PLT light chain amino acid sequence

<400>  73

Asp Ile Val Leu Thr Gln Ser Pro Ala Ser Leu Ala Val Ser Pro Gly
1               5                   10                  15

Gln Arg Ala Thr Ile Thr Cys Arg Ala Ser Lys Ser Val His Thr Ser
            20                  25                  30

Gly Tyr Ser Tyr Met His Trp Tyr Gln Gln Lys Pro Gly Gln Pro Pro
        35                  40                  45

Lys Leu Leu Ile Tyr Leu Ala Ser Asn Leu Glu Ser Gly Val Pro Ala
    50                  55                  60

Arg Phe Ser Gly Ser Gly Ser Gly Thr Asp Phe Thr Leu Thr Ile Asn
65                  70                  75                  80

Pro Val Glu Ala Asn Asp Thr Ala Asn Tyr Tyr Cys Gln His Ser Gly
                85                  90                  95

Glu Leu Pro Tyr Thr Phe Gly Gly Gly Thr Lys Val Glu Ile Lys Arg
            100                 105                 110

Thr Val Ala Ala Pro Ser Val Phe Ile Phe Pro Pro Ser Asp Glu Gln
        115                 120                 125

Leu Lys Ser Gly Thr Ala Ser Val Val Cys Leu Leu Asn Asn Phe Tyr
    130                 135                 140

Pro Arg Glu Ala Lys Val Gln Trp Lys Val Asp Asn Ala Leu Gln Ser
145                 150                 155                 160

Gly Asn Ser Gln Glu Ser Val Thr Glu Gln Asp Ser Lys Asp Ser Thr
                165                 170                 175

Tyr Ser Leu Ser Ser Thr Leu Thr Leu Ser Lys Ala Asp Tyr Glu Lys
            180                 185                 190

His Lys Val Tyr Ala Cys Glu Val Thr His Gln Gly Leu Ser Ser Pro
        195                 200                 205

Val Thr Lys Ser Phe Asn Arg Gly Glu Cys
    210                 215

<210>  74
<211>  2007
<212>  DNA
<213>  Artificial sequence

<220>
<223>  Hib-PDC heavy chain nucleic acid sequence

<400>  74
caggtgcagc tcgtggagag cgggggaggc gtcgtccagc ctggccgtag cctgcggctg      60

gactgtaagg ctagcggaat cacgttcagc aacagcggaa tgcactgggt cagacaggct     120

cctggcaaag gcctcgaatg ggtcgccgtg atctggtacg acgggagcaa gagatactac     180

gcagattcag tgaagggccg tttcacaatc agccgtgaca actcgaagaa cacactgttc     240

ctgcagatga acagcctgag ggcagaagac acagcagtct actactgcgc tacaaatgac     300

gactactggg gccagggaac actggtcacc gtgagctcag cttccaccaa gggcccatcc     360

gtcttccccc tggcgccctg ctccaggagc acctccgaga gcacagccgc cctgggctgc     420

ctggtcaagg actacttccc cgaaccggtg acggtgtcgt ggaactcagg cgccctgacc     480

agcggcgtgc acaccttccc ggctgtccta cagtcctcag gactctactc cctcagcagc     540

gtggtgaccg tgccctccag cagcttgggc acgaagacct acacctgcaa cgtagatcac     600

aagcccagca acaccaaggt ggacaagaga gttgagtccg gtggcggcgg aagcggcggc     660

ggaggcagcg gcggaggcgg cagcgtgatc catgtcacga aggaggtcaa agaagtggcc     720

accctcagct gcggtcacaa cgtcagcgtg gaagagttgg cccagaccag aatctactgg     780

cagaaggaga agaagatggt cttgaccatg atgagcggcg acatgaacat ctggccagag     840

tacaagaata gaactatctt cgacatcacc aataacctga gcatcgtgat cctcgctttg     900

cgtccgagcg acgaaggcac ctacgagtgc gtagtgctaa agtacgagaa agacgccttc     960

aagcgggagc acctggcaga agtaaccctg agcgtgaagg cagacttccc aacgcctagc    1020

atctccgact tcgagatccc cacaagcaac atccggcgga tcatctgtag cacctccggg    1080

ggtttccccg agcctcacct gtcctggctc gagaacggcg aggagctgaa cgccatcaac    1140

accaccgtga gccaggaccc cgagacagaa ctgtacgccg tgagctccaa gctcgacttc    1200

aacatgacca caaatcacag cttcatgtgc ctcatcaagt acggacacct gcgggtgaat    1260

cagacgttca actggaacgg cggggggggt agcggcggcg ggggctcagg tggggggggc    1320

tcaaaatatg gtcccccatg cccaccatgc ccagcacctg agttcctggg gggaccatca    1380

gtcttcctgt tccccccaaa acccaaggac actctcatga tctcccggac ccctgaggtc    1440

acgtgcgtgg tggtggacgt gagccaggaa gaccccgagg tccagttcaa ctggtacgtg    1500

gatggcgtgg aggtgcataa tgccaagaca aagccgcggg aggagcagtt caacagcacg    1560

taccgtgtgg tcagcgtcct caccgtcctg caccaggact ggctgaacgg caaggagtac    1620

aagtgcaagg tctccaacaa aggcctcccg tcctccatcg agaaaaccat ctccaaagcc 1680

aaagggcagc cccgagagcc acaggtgtac accctgcccc catcccagga ggagatgacc 1740

aagaaccagg tcagcctgac ctgcctggtc aaaggcttct accccagcga catcgccgtg 1800

gagtgggaga gcaatgggca gccggagaac aactacaaga ccacgcctcc cgtgctggac 1860

tccgacggct ccttcttcct ctacagcagg ctaaccgtgg acaagagcag gtggcaggag 1920

gggaatgtct tctcatgctc cgtgatgcat gaggctctgc acaaccacta cacacagaag 1980

agcctctccc tgtctctggg taaatag 2007

<210> 75
<211> 645
<212> DNA
<213> Artificial sequence

<220>
<223> Hib-PDC light chain nucleic acid sequence

<400> 75
gagatcgtgc tgacacagag cccagccact ctgtcactgt ccccaggaga aagggctact 60

ctgtcttgcc gggcaagcca gtctgtctcc agctacctgg cctggtatca gcagaagccc 120

ggacaggctc ctagactgct gatctacgac gcaagtaaca gagccaccgg catccccgca 180

cgcttcagtg gctcaggctc cggaacagac tttactctga ccatctctag tctggagcct 240

gaagatttcg ccgtgtacta ttgtcagcag agctctaatt ggcctagaac cttcggccag 300

ggcaccaaag tcgagatcaa gcgtacggtg gctgcaccat ctgtcttcat cttcccgcca 360

tctgatgagc agttgaaatc tggaactgcc tctgttgtgt gcctgctgaa taacttctat 420

cccagagagg ccaaagtaca gtggaaggtg gataacgccc tccaatcggg taactcccag 480

gagagtgtca cagagcagga cagcaaggac agcacctaca gcctcagcag caccctgacg 540

ctgagcaaag cagactacga gaaacacaaa gtctacgcct gcgaagtcac ccatcagggc 600

ctgagctcgc ccgtcacaaa gagcttcaac aggggagagt gttag 645

<210> 76
<211> 2019
<212> DNA
<213> Artificial sequence

<220>
<223> Hib-PDV heavy chain nucleic acid sequence

<400> 76
caggtgcagc tcgtggagag cgggggaggc gtcgtccagc ctggccgtag cctgcggctg 60

gactgtaagg ctagcggaat cacgttcagc aacagcggaa tgcactgggt cagacaggct 120
cctggcaaag gcctcgaatg ggtcgccgtg atctggtacg acgggagcaa gagatactac 180
gcagattcag tgaagggccg tttcacaatc agccgtgaca actcgaagaa cacactgttc 240
ctgcagatga acagcctgag ggcagaagac acagcagtct actactgcgc tacaaatgac 300
gactactggg gccagggaac actggtcacc gtgagctcag cttccaccaa gggcccatcc 360
gtcttccccc tggcgccctg ctccaggagc acctccgaga gcacagccgc cctgggctgc 420
ctggtcaagg actacttccc cgaaccggtg acggtgtcgt ggaactcagg cgccctgacc 480
agcggcgtgc acaccttccc ggctgtccta cagtcctcag gactctactc cctcagcagc 540
gtggtgaccg tgccctccag cagcttgggc acgaagacct acacctgcaa cgtagatcac 600
aagcccagca acaccaaggt ggacaagaga gttgagtccg gtggcggcgg aagcggcggc 660
ggaggcagcg gcggaggcgg cagcggtaga ccattcgtag agatgtacag cgaaatcccc 720
gaaatcatcc acatgactga aggaagggag ctcgtcatcc cctgccgggt tacgtcacct 780
aacatcactg ttactctgaa gaagttccca ctcgacactt tgatccctga tggaaaacgc 840
atcatctggg acagcagaaa gggcttcatc atctcaaatg caacgtacaa agaaatcgga 900
ctcctgacct gtgaagcaac agtcaatgga catttgtata agacaaacta tctcacacat 960
cgacagacca atacaatcat cgatgtggtt ctgagcccgt ctcatggaat cgaactatct 1020
gttggagaaa agctcgtcct gaattgtaca gcaagaactg aactgaatgt gggaatcgac 1080
ttcaactggg aatacccttc ttcgaagcat cagcataaga aactcgtaaa ccgagaccta 1140
aagacccagt ctgggagcga gatgaagaaa ttcttgagca ccctgactat cgatggtgta 1200
acccggagcg accagggatt gtacacctgt gcagcatcca gcgggctgat gaccaagaag 1260
aacagcacat tcgtcagggt ccatgaaccc ggcggggggg gtagcggcgg cgggggctca 1320
ggtggggggg gctcaaaata tggtccccca tgcccaccat gcccagcacc tgagttcctg 1380
gggggaccat cagtcttcct gttcccccca aaacccaagg acactctcat gatctcccgg 1440
acccctgagg tcacgtgcgt ggtggtggac gtgagccagg aagaccccga ggtccagttc 1500
aactggtacg tggatggcgt ggaggtgcat aatgccaaga caaagccgcg ggaggagcag 1560
ttcaacagca cgtaccgtgt ggtcagcgtc ctcaccgtcc tgcaccagga ctggctgaac 1620
ggcaaggagt acaagtgcaa ggtctccaac aaaggcctcc cgtcctccat cgagaaaacc 1680
atctccaaag ccaaagggca gccccgagag ccacaggtgt acaccctgcc cccatcccag 1740
gaggagatga ccaagaacca ggtcagcctg acctgcctgg tcaaaggctt ctaccccagc 1800

gacatcgccg tggagtggga gagcaatggg cagccggaga acaactacaa gaccacgcct 1860

cccgtgctgg actccgacgg ctccttcttc ctctacagca ggctaaccgt ggacaagagc 1920

aggtggcagg aggggaatgt cttctcatgc tccgtgatgc atgaggctct gcacaaccac 1980

tacacacaga agagcctctc cctgtctctg ggtaaatag 2019

<210> 77
<211> 645
<212> DNA
<213> Artificial sequence

<220>
<223> Hib-PDV light chain nucleic acid sequence

<400> 77
gagatcgtgc tgacacagag cccagccact ctgtcactgt ccccaggaga aagggctact 60

ctgtcttgcc gggcaagcca gtctgtctcc agctacctgg cctggtatca gcagaagccc 120

ggacaggctc ctagactgct gatctacgac gcaagtaaca gagccaccgg catccccgca 180

cgcttcagtg gctcaggctc cggaacagac tttactctga ccatctctag tctggagcct 240

gaagatttcg ccgtgtacta ttgtcagcag agctctaatt ggcctagaac cttcggccag 300

ggcaccaaag tcgagatcaa gcgtacggtg gctgcaccat ctgtcttcat cttcccgcca 360

tctgatgagc agttgaaatc tggaactgcc tctgttgtgt gcctgctgaa taacttctat 420

cccagagagg ccaaagtaca gtggaaggtg gataacgccc tccaatcggg taactcccag 480

gagagtgtca cagagcagga cagcaaggac agcacctaca gcctcagcag caccctgacg 540

ctgagcaaag cagactacga gaaacacaaa gtctacgcct gcgaagtcac ccatcagggc 600

ctgagctcgc ccgtcacaaa gagcttcaac aggggagagt gttag 645

<210> 78
<211> 1848
<212> DNA
<213> Artificial sequence

<220>
<223> Hib-PLT heavy chain nucleic acid sequence

<400> 78
caggtgcagc tgcaggagtc cggaccagga ctggtgaagc catccgagac cctgagcctg 60

acctgtacag tgtccggctt cagcctgtct aggtacagcg tgcactggat cagacagcca 120

cctggcaagg gactggagtg gctgggcatg atctggggcg tgggcaccac agactacaac 180

tctgctctga agtccagact gaccatcagc aaggatacat ctaagaatca gttcagcctg 240

aagctgtcca gcgtgaccgc cgctgacaca gccgtgtact attgcgctcg caactggggc 300

accgccgact acttcgacta ttggggccag ggcaccacag tgacagtgtc ttccgctagc 360

accaagggcc catcggtctt ccccctggca ccctcctcca agagcacctc tgggggcaca 420

gcggccctgg gctgcctggt caaggactac ttccccgaac cggtgacggt gtcgtggaac 480

tcaggcgccc tgaccagcgg cgtgcacacc ttcccggctg tcctacagtc ctcaggactc 540

tactccctca gcagcgtggt gaccgtgccc tccagcagct tgggcaccca gacctacatc 600

tgcaacgtga atcacaagcc cagcaacacc aaggtggaca agaaagttga gcccaaatct 660

tgtgacaaag gcgggggagg cagcggcggc ggaggaagcg ggggcggagg tagcaccatc 720

cctccacacg tgcagaagag cgtgaacaat gacatgatcg tcaccgacaa caacggcgcc 780

gtcaagttcc cccagctgtg caaattctgc gacgtgaggt tctccacgtg cgacaaccag 840

aagagctgta tgagcaactg cagcatcaca tccatctgcg aaaaacccca ggaagtgtgc 900

gtcgccgtct ggcggaagaa cgacgagaac atcacactgg agaccgtgtg ccacgacccc 960

aaactgccct accacgactt catcctggag gacgccgcca gcccaaagtg catcatgaaa 1020

gagaagaaga agccgggcga gactttcttc atgtgctcct gcagctccga cgagtgcaac 1080

gataatatca tcttcagcga agaatacaac acatctaacc cagacggagg gggcggatcc 1140

gggggcggcg gaagcggcgg ggggggcagc actcacacat gcccaccgtg cccagcacct 1200

gaactcctgg ggggaccgtc agtcttcctc ttccccccaa aacccaagga caccctcatg 1260

atctcccgga cccctgaggt cacatgcgtg gtggtggacg tgagccacga agaccctgag 1320

gtcaagttca actggtacgt ggacggcgtg gaggtgcata atgccaagac aaagccgcgg 1380

gaggagcagt acaacagcac gtaccgtgtg gtcagcgtcc tcaccgtcct gcaccaggac 1440

tggctgaatg gcaaggagta caagtgcaag gtctccaaca aagccctccc agcccccatc 1500

gagaaaacca tctccaaagc caaagggcag ccccgagaac cacaggtgta caccctgccc 1560

ccatcccggg aggagatgac caagaaccag gtcagcctga cctgcctggt caaaggcttc 1620

tatcccagcg acatcgccgt ggagtgggag agcaatgggc agccggagaa caactacaag 1680

accacgcctc ccgtgctgga ctccgacggc tccttcttcc tctacagcaa gctcaccgtg 1740

gacaagagca ggtggcagca ggggaacgtc ttctcatgct ccgtgatgca tgaggctctg 1800

cacaaccact acacgcagaa gagcctctcc ctgtctccgg gtaaatga 1848

<210> 79
<211> 657

<212>  DNA
<213>  Artificial sequence

<220>
<223>  Hib-PLT light chain nucleic acid sequence

<400>  79
gatatcgtgc tgacccagtc tccagcttcc ctggccgtgt ccccaggaca gagggccacc      60

atcacatgtc gggcttccaa gagcgtgcac acaagcggct actcttatat gcattggtac     120

cagcagaagc ccggccagcc ccctaagctg ctgatctatc tggcttccaa cctggagagc     180

ggagtgccag ctaggttctc tggctccggc agcggcaccg actttaccct gacaatcaat     240

cctgtggagg ccaacgatac agctaattac tattgccagc actccggaga gctgccatac     300

accttcggcg gaggcacaaa ggtggagatc aagcgtacgg tggctgcacc atctgtcttc     360

atcttcccgc catctgatga gcagttgaaa tctggaactg cctctgttgt gtgcctgctg     420

aataacttct atcccagaga ggccaaagta cagtggaagg tggataacgc cctccaatcg     480

ggtaactccc aggagagtgt cacagagcag gacagcaagg acagcaccta cagcctcagc     540

agcaccctga cgctgagcaa agcagactac gagaaacaca aagtctacgc ctgcgaagtc     600

acccatcagg gcctgagctc gcccgtcaca aagagcttca acaggggaga gtgttag       657


<210>  80
<211>  16
<212>  PRT
<213>  Artificial sequence

<220>
<223>  Peptide linker

<400>  80

Ala Lys Thr Thr Pro Lys Leu Glu Glu Gly Glu Phe Ser Glu Ala Arg
1               5                   10                  15


<210>  81
<211>  17
<212>  PRT
<213>  Artificial sequence

<220>
<223>  Peptide linker

<400>  81

Ala Lys Thr Thr Pro Lys Leu Glu Glu Gly Glu Phe Ser Glu Ala Arg
1               5                   10                  15

Val

<210> 82
<211> 9
<212> PRT
<213> Artificial sequence

<220>
<223> Peptide linker

<400> 82

Ala Lys Thr Thr Pro Lys Leu Gly Gly
1               5

<210> 83
<211> 10
<212> PRT
<213> Artificial sequence

<220>
<223> Peptide linker

<400> 83

Ser Ala Lys Thr Thr Pro Lys Leu Gly Gly
1               5                   10

<210> 84
<211> 6
<212> PRT
<213> Artificial sequence

<220>
<223> Peptide linker

<400> 84

Ser Ala Lys Thr Thr Pro
1               5

<210> 85
<211> 6
<212> PRT
<213> Artificial sequence

<220>
<223> Peptide linker

<400> 85

Arg Ala Asp Ala Ala Pro
1               5

<210>  86
<211>  9
<212>  PRT
<213>  Artificial sequence

<220>
<223>  Peptide linker

<400>  86

Arg Ala Asp Ala Ala Pro Thr Val Ser
1               5


<210>  87
<211>  12
<212>  PRT
<213>  Artificial sequence

<220>
<223>  Peptide linker

<400>  87

Arg Ala Asp Ala Ala Ala Ala Gly Gly Pro Gly Ser
1               5                   10


<210>  88
<211>  7
<212>  PRT
<213>  Artificial sequence

<220>
<223>  Peptide linker

<400>  88

Arg Ala Asp Ala Ala Ala Ala
1               5


<210>  89
<211>  18
<212>  PRT
<213>  Artificial sequence

<220>
<223>  Peptide linker

<400>  89

Ser Ala Lys Thr Thr Pro Lys Leu Glu Glu Gly Glu Phe Ser Glu Ala
1               5                   10                  15

Arg Val

<210> 90
<211> 5
<212> PRT
<213> Artificial sequence

<220>
<223> Peptide linker

<400> 90

Ala Asp Ala Ala Pro
1               5

<210> 91
<211> 11
<212> PRT
<213> Artificial sequence

<220>
<223> Peptide linker

<400> 91

Asp Ala Ala Pro Thr Val Ser Ile Phe Pro Pro
1               5                   10

<210> 92
<211> 5
<212> PRT
<213> Artificial sequence

<220>
<223> Peptide linker

<400> 92

Thr Val Ala Ala Pro
1               5

<210> 93
<211> 12
<212> PRT
<213> Artificial sequence

<220>
<223> Peptide linker

<400> 93

Thr Val Ala Ala Pro Ser Val Phe Ile Phe Pro Pro
1               5                   10

<210>  94
<211>  6
<212>  PRT
<213>  Artificial sequence

<220>
<223>  Peptide linker

<400>  94

Gln Pro Lys Ala Ala Pro
1               5


<210>  95
<211>  13
<212>  PRT
<213>  Artificial sequence

<220>
<223>  Peptide linker

<400>  95

Gln Pro Lys Ala Ala Pro Ser Val Thr Leu Phe Pro Pro
1               5                   10


<210>  96
<211>  6
<212>  PRT
<213>  Artificial sequence

<220>
<223>  Peptide linker

<400>  96

Ala Lys Thr Thr Pro Pro
1               5


<210>  97
<211>  13
<212>  PRT
<213>  Artificial sequence

<220>
<223>  Peptide linker

<400>  97

Ala Lys Thr Thr Pro Pro Ser Val Thr Pro Leu Ala Pro
1               5                   10

<210>  98
<211>  6
<212>  PRT
<213>  Artificial sequence

<220>
<223>  Peptide linker

<400>  98

Ala Lys Thr Thr Ala Pro
1               5


<210>  99
<211>  13
<212>  PRT
<213>  Artificial sequence

<220>
<223>  Peptide linker

<400>  99

Ala Lys Thr Thr Ala Pro Ser Val Tyr Pro Leu Ala Pro
1               5                   10


<210>  100
<211>  6
<212>  PRT
<213>  Artificial sequence

<220>
<223>  Peptide linker

<400>  100

Ala Ser Thr Lys Gly Pro
1               5


<210>  101
<211>  13
<212>  PRT
<213>  Artificial sequence

<220>
<223>  Peptide linker

<400>  101

Ala Ser Thr Lys Gly Pro Ser Val Phe Pro Leu Ala Pro
1               5                   10


<210>  102
<211>  15

<212>  PRT
<213>  Artificial sequence

<220>
<223>  Peptide linker

<400>  102

Gly Glu Asn Lys Val Glu Tyr Ala Pro Ala Leu Met Ala Leu Ser
1               5                   10                  15


<210>  103
<211>  15
<212>  PRT
<213>  Artificial sequence

<220>
<223>  Peptide linker

<400>  103

Gly Pro Ala Lys Glu Leu Thr Pro Leu Lys Glu Ala Lys Val Ser
1               5                   10                  15


<210>  104
<211>  15
<212>  PRT
<213>  Artificial sequence

<220>
<223>  Peptide linker

<400>  104

Gly His Glu Ala Ala Ala Val Met Gln Val Gln Tyr Pro Ala Ser
1               5                   10                  15


<210>  105
<211>  16
<212>  PRT
<213>  Artificial sequence

<220>
<223>  Peptide linker

<400>  105

Gly Gly Gly Gly Ser Gly Gly Gly Gly Ser Gly Gly Gly Gly Ser Ala
1               5                   10                  15


<210>  106
<211>  448
<212>  PRT
<213>  Artificial sequence

<220>
<223>  Heavy chain amino acid sequence of Atezolizumab

<400>  106

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Asp Ser
            20                  25                  30

Trp Ile His Trp Val Arg Gln Ala Pro Gly Lys Gly Leu Glu Trp Val
        35                  40                  45

Ala Trp Ile Ser Pro Tyr Gly Gly Ser Thr Tyr Tyr Ala Asp Ser Val
    50                  55                  60

Lys Gly Arg Phe Thr Ile Ser Ala Asp Thr Ser Lys Asn Thr Ala Tyr
65                  70                  75                  80

Leu Gln Met Asn Ser Leu Arg Ala Glu Asp Thr Ala Val Tyr Tyr Cys
                85                  90                  95

Ala Arg Arg His Trp Pro Gly Gly Phe Asp Tyr Trp Gly Gln Gly Thr
            100                 105                 110

Leu Val Thr Val Ser Ser Ala Ser Thr Lys Gly Pro Ser Val Phe Pro
        115                 120                 125

Leu Ala Pro Ser Ser Lys Ser Thr Ser Gly Gly Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Gln Thr Tyr Ile Cys Asn Val Asn His Lys Pro Ser
        195                 200                 205

Asn Thr Lys Val Asp Lys Lys Val Glu Pro Lys Ser Cys Asp Lys Thr
    210                 215                 220

His Thr Cys Pro Pro Cys Pro Ala Pro Glu Leu Leu Gly Gly Pro Ser
225                 230                 235                 240

Val Phe Leu Phe Pro Pro Lys Pro Lys Asp Thr Leu Met Ile Ser Arg
                245                 250                 255

Thr Pro Glu Val Thr Cys Val Val Val Asp Val Ser His Glu Asp Pro
            260                 265                 270

Glu Val Lys Phe Asn Trp Tyr Val Asp Gly Val Glu Val His Asn Ala
        275                 280                 285

Lys Thr Lys Pro Arg Glu Glu Gln Tyr Ala Ser Thr Tyr Arg Val Val
    290                 295                 300

Ser Val Leu Thr Val Leu His Gln Asp Trp Leu Asn Gly Lys Glu Tyr
305                 310                 315                 320

Lys Cys Lys Val Ser Asn Lys Ala Leu Pro Ala Pro Ile Glu Lys Thr
                325                 330                 335

Ile Ser Lys Ala Lys Gly Gln Pro Arg Glu Pro Gln Val Tyr Thr Leu
            340                 345                 350

Pro Pro Ser Arg Glu Glu Met Thr Lys Asn Gln Val Ser Leu Thr Cys
        355                 360                 365

Leu Val Lys Gly Phe Tyr Pro Ser Asp Ile Ala Val Glu Trp Glu Ser
    370                 375                 380

Asn Gly Gln Pro Glu Asn Asn Tyr Lys Thr Thr Pro Pro Val Leu Asp
385                 390                 395                 400

Ser Asp Gly Ser Phe Phe Leu Tyr Ser Lys Leu Thr Val Asp Lys Ser
                405                 410                 415

Arg Trp Gln Gln Gly Asn Val Phe Ser Cys Ser Val Met His Glu Ala
            420                 425                 430

Leu His Asn His Tyr Thr Gln Lys Ser Leu Ser Leu Ser Pro Gly Lys
        435                 440                 445

<210>  107
<211>  214
<212>  PRT
<213>  Artificial sequence

<220>
<223>  Light chain amino acid sequence of Atezolizumab

<400>  107

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Val Ser Thr Ala
            20                  25                  30

Val Ala Trp Tyr Gln Gln Lys Pro Gly Lys Ala Pro Lys Leu Leu Ile
        35                  40                  45

Tyr Ser Ala Ser Phe Leu Tyr Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Leu Tyr His Pro Ala
                85                  90                  95

Thr Phe Gly Gln Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210>  108
<211>  450
<212>  PRT
<213>  Artificial sequence

<220>
<223>  Heavy chain amino acid sequence of Avelumab

<400>  108

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ile Met Met Trp Val Arg Gln Ala Pro Gly Lys Gly Leu Glu Trp Val
        35                  40                  45

Ser Ser Ile Tyr Pro Ser Gly Gly Ile Thr Phe Tyr Ala Asp Thr Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Ile Lys Leu Gly Thr Val Thr Thr Val Asp Tyr Trp Gly Gln
            100                 105                 110

Gly Thr Leu Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Lys Val Glu Pro Lys Ser Cys Asp
    210                 215                 220

Lys Thr His Thr Cys Pro Pro Cys Pro Ala Pro Glu Leu Leu Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Asn Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Ala Leu Pro Ala Pro Ile Glu
                325                 330                 335

Lys Thr Ile Ser Lys Ala Lys Gly Gln Pro Arg Glu Pro Gln Val Tyr
            340                 345                 350

Thr Leu Pro Pro Ser Arg Asp Glu Leu Thr Lys Asn Gln Val Ser Leu
        355                 360                 365

Thr Cys Leu Val Lys Gly Phe Tyr Pro Ser Asp Ile Ala Val Glu Trp
    370                 375                 380

Glu Ser Asn Gly Gln Pro Glu Asn Asn Tyr Lys Thr Thr Pro Pro Val
385                 390                 395                 400

Leu Asp Ser Asp Gly Ser Phe Phe Leu Tyr Ser Lys Leu Thr Val Asp
                405                 410                 415

Lys Ser Arg Trp Gln Gln Gly Asn Val Phe Ser Cys Ser Val Met His
            420                 425                 430

Glu Ala Leu His Asn His Tyr Thr Gln Lys Ser Leu Ser Leu Ser Pro
        435                 440                 445

Gly Lys
    450

<210>  109
<211>  216
<212>  PRT
<213>  Artificial sequence

<220>
<223>  Light chain amino acid sequence of Avelumab

<400>  109

Gln Ser Ala Leu Thr Gln Pro Ala Ser Val Ser Gly Ser Pro Gly Gln
1               5                   10                  15

Ser Ile Thr Ile Ser Cys Thr Gly Thr Ser Ser Asp Val Gly Gly Tyr
            20                  25                  30

Asn Tyr Val Ser Trp Tyr Gln Gln His Pro Gly Lys Ala Pro Lys Leu
        35                  40                  45

Met Ile Tyr Asp Val Ser Asn Arg Pro Ser Gly Val Ser Asn Arg Phe
    50                  55                  60

Ser Gly Ser Lys Ser Gly Asn Thr Ala Ser Leu Thr Ile Ser Gly Leu
65                  70                  75                  80

Gln Ala Glu Asp Glu Ala Asp Tyr Tyr Cys Ser Ser Tyr Thr Ser Ser
                85                  90                  95

Ser Thr Arg Val Phe Gly Thr Gly Thr Lys Val Thr Val Leu Gly Gln
            100                 105                 110

Pro Lys Ala Asn Pro Thr Val Thr Leu Phe Pro Pro Ser Ser Glu Glu
        115                 120                 125

Leu Gln Ala Asn Lys Ala Thr Leu Val Cys Leu Ile Ser Asp Phe Tyr
    130                 135                 140

Pro Gly Ala Val Thr Val Ala Trp Lys Ala Asp Gly Ser Pro Val Lys
145                 150                 155                 160

Ala Gly Val Glu Thr Thr Lys Pro Ser Lys Gln Ser Asn Asn Lys Tyr
                165                 170                 175

Ala Ala Ser Ser Tyr Leu Ser Leu Thr Pro Glu Gln Trp Lys Ser His
            180                 185                 190

Arg Ser Tyr Ser Cys Gln Val Thr His Glu Gly Ser Thr Val Glu Lys
        195                 200                 205

Thr Val Ala Pro Thr Glu Cys Ser
    210                 215

<210>  110
<211>  451
<212>  PRT
<213>  Artificial sequence

<220>
<223>  Heavy chain amino acid sequence of Durvalumab

<400>  110

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Arg Tyr
            20                  25                  30

Trp Met Ser Trp Val Arg Gln Ala Pro Gly Lys Gly Leu Glu Trp Val
        35                  40                  45

Ala Asn Ile Lys Gln Asp Gly Ser Glu Lys Tyr Tyr Val Asp Ser Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Ala Arg Glu Gly Gly Trp Phe Gly Glu Leu Ala Phe Asp Tyr Trp Gly
            100                 105                 110

Gln Gly Thr Leu Val Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
        195                 200                 205

Lys Pro Ser Asn Thr Lys Val Asp Lys Arg Val Glu Pro Lys Ser Cys
    210                 215                 220

Asp Lys Thr His Thr Cys Pro Pro Cys Pro Ala Pro Glu Phe Glu Gly
225                 230                 235                 240

Gly Pro Ser Val Phe Leu Phe Pro Pro Lys Pro Lys Asp Thr Leu Met
                245                 250                 255

Ile Ser Arg Thr Pro Glu Val Thr Cys Val Val Val Asp Val Ser His
            260                 265                 270

Glu Asp Pro Glu Val Lys Phe Asn Trp Tyr Val Asp Gly Val Glu Val
        275                 280                 285

His Asn Ala Lys Thr Lys Pro Arg Glu Glu Gln Tyr Asn Ser Thr Tyr
    290                 295                 300

Arg Val Val Ser Val Leu Thr Val Leu His Gln Asp Trp Leu Asn Gly
305                 310                 315                 320

Lys Glu Tyr Lys Cys Lys Val Ser Asn Lys Ala Leu Pro Ala Ser Ile
                325                 330                 335

Glu Lys Thr Ile Ser Lys Ala Lys Gly Gln Pro Arg Glu Pro Gln Val
            340                 345                 350

Tyr Thr Leu Pro Pro Ser Arg Glu Glu Met Thr Lys Asn Gln Val Ser
        355                 360                 365

Leu Thr Cys Leu Val Lys Gly Phe Tyr Pro Ser Asp Ile Ala Val Glu
    370                 375                 380

Trp Glu Ser Asn Gly Gln Pro Glu Asn Asn Tyr Lys Thr Thr Pro Pro
385                 390                 395                 400

Val Leu Asp Ser Asp Gly Ser Phe Phe Leu Tyr Ser Lys Leu Thr Val
                405                 410                 415

Asp Lys Ser Arg Trp Gln Gln Gly Asn Val Phe Ser Cys Ser Val Met
            420                 425                 430

His Glu Ala Leu His Asn His Tyr Thr Gln Lys Ser Leu Ser Leu Ser
        435                 440                 445

Pro Gly Lys
    450

<210>  111
<211>  215
<212>  PRT
<213>  Artificial sequence

<220>
<223>  Light chain amino acid sequence of Durvalumab

<400>  111

Glu Ile Val Leu Thr Gln Ser Pro Gly Thr Leu Ser Leu Ser Pro Gly
1               5                   10                  15

Glu Arg Ala Thr Leu Ser Cys Arg Ala Ser Gln Arg Val Ser Ser Ser
            20                  25                  30

Tyr Leu Ala Trp Tyr Gln Gln Lys Pro Gly Gln Ala Pro Arg Leu Leu
        35                  40                  45

Ile Tyr Asp Ala Ser Ser Arg Ala Thr Gly Ile Pro Asp Arg Phe Ser
    50                  55                  60

Gly Ser Gly Ser Gly Thr Asp Phe Thr Leu Thr Ile Ser Arg Leu Glu
65                  70                  75                  80

Pro Glu Asp Phe Ala Val Tyr Tyr Cys Gln Gln Tyr Gly Ser Leu Pro
                85                  90                  95

Trp Thr Phe Gly Gln Gly Thr Lys Val Glu Ile Lys Arg Thr Val Ala
            100                 105                 110

Ala Pro Ser Val Phe Ile Phe Pro Pro Ser Asp Glu Gln Leu Lys Ser
        115                 120                 125

Gly Thr Ala Ser Val Val Cys Leu Leu Asn Asn Phe Tyr Pro Arg Glu
    130                 135                 140

Ala Lys Val Gln Trp Lys Val Asp Asn Ala Leu Gln Ser Gly Asn Ser
145                 150                 155                 160

Gln Glu Ser Val Thr Glu Gln Asp Ser Lys Asp Ser Thr Tyr Ser Leu
                165                 170                 175

Ser Ser Thr Leu Thr Leu Ser Lys Ala Asp Tyr Glu Lys His Lys Val
            180                 185                 190

Tyr Ala Cys Glu Val Thr His Gln Gly Leu Ser Ser Pro Val Thr Lys
        195                 200                 205

Ser Phe Asn Arg Gly Glu Cys
    210                 215

<210>  112
<211>  440
<212>  PRT
<213>  Artificial sequence

<220>
<223>  Heavy chain amino acid sequence of Nivolumab

<400>  112

Gln Val Gln Leu Val Glu Ser Gly Gly Gly Val Val Gln Pro Gly Arg
1               5                   10                  15

Ser Leu Arg Leu Asp Cys Lys Ala Ser Gly Ile Thr Phe Ser Asn Ser
            20                  25                  30

Gly Met His Trp Val Arg Gln Ala Pro Gly Lys Gly Leu Glu Trp Val
        35                  40                  45

Ala Val Ile Trp Tyr Asp Gly Ser Lys Arg Tyr Tyr Ala Asp Ser Val
    50                  55                  60

Lys Gly Arg Phe Thr Ile Ser Arg Asp Asn Ser Lys Asn Thr Leu Phe
65                  70                  75                  80

Leu Gln Met Asn Ser Leu Arg Ala Glu Asp Thr Ala Val Tyr Tyr Cys
                85                  90                  95

Ala Thr Asn Asp Asp Tyr Trp Gly Gln Gly Thr Leu Val Thr Val Ser
            100                 105                 110

Ser Ala Ser Thr Lys Gly Pro Ser Val Phe Pro Leu Ala Pro Cys Ser
        115                 120                 125

Arg Ser Thr Ser Glu Ser Thr Ala Ala Leu Gly Cys Leu Val Lys Asp
    130                 135                 140

Tyr Phe Pro Glu Pro Val Thr Val Ser Trp Asn Ser Gly Ala Leu Thr
145                 150                 155                 160

Ser Gly Val His Thr Phe Pro Ala Val Leu Gln Ser Ser Gly Leu Tyr
                165                 170                 175

Ser Leu Ser Ser Val Val Thr Val Pro Ser Ser Ser Leu Gly Thr Lys
            180                 185                 190

Thr Tyr Thr Cys Asn Val Asp His Lys Pro Ser Asn Thr Lys Val Asp
        195                 200                 205

Lys Arg Val Glu Ser Lys Tyr Gly Pro Pro Cys Pro Pro Cys Pro Ala
    210                 215                 220

Pro Glu Phe Leu Gly Gly Pro Ser Val Phe Leu Phe Pro Pro Lys Pro
225                 230                 235                 240

Lys Asp Thr Leu Met Ile Ser Arg Thr Pro Glu Val Thr Cys Val Val
                245                 250                 255

Val Asp Val Ser Gln Glu Asp Pro Glu Val Gln Phe Asn Trp Tyr Val
            260                 265                 270

Asp Gly Val Glu Val His Asn Ala Lys Thr Lys Pro Arg Glu Glu Gln
        275                 280                 285

Phe Asn Ser Thr Tyr Arg Val Val Ser Val Leu Thr Val Leu His Gln
    290                 295                 300

Asp Trp Leu Asn Gly Lys Glu Tyr Lys Cys Lys Val Ser Asn Lys Gly
305                 310                 315                 320

Leu Pro Ser Ser Ile Glu Lys Thr Ile Ser Lys Ala Lys Gly Gln Pro
                325                 330                 335

Arg Glu Pro Gln Val Tyr Thr Leu Pro Pro Ser Gln Glu Glu Met Thr
            340                 345                 350

Lys Asn Gln Val Ser Leu Thr Cys Leu Val Lys Gly Phe Tyr Pro Ser
        355                 360                 365

Asp Ile Ala Val Glu Trp Glu Ser Asn Gly Gln Pro Glu Asn Asn Tyr
    370                 375                 380

Lys Thr Thr Pro Pro Val Leu Asp Ser Asp Gly Ser Phe Phe Leu Tyr
385                 390                 395                 400

Ser Arg Leu Thr Val Asp Lys Ser Arg Trp Gln Glu Gly Asn Val Phe
                405                 410                 415

Ser Cys Ser Val Met His Glu Ala Leu His Asn His Tyr Thr Gln Lys
            420                 425                 430

Ser Leu Ser Leu Ser Leu Gly Lys
        435                 440

<210>  113
<211>  214
<212>  PRT
<213>  Artificial sequence

<220>

<223>  Light chain amino acid sequence of Nivolumab

<400>  113

Glu Ile Val Leu Thr Gln Ser Pro Ala Thr Leu Ser Leu Ser Pro Gly
1               5                   10                  15

Glu Arg Ala Thr Leu Ser Cys Arg Ala Ser Gln Ser Val Ser Ser Tyr
            20                  25                  30

Leu Ala Trp Tyr Gln Gln Lys Pro Gly Gln Ala Pro Arg Leu Leu Ile
        35                  40                  45

Tyr Asp Ala Ser Asn Arg Ala Thr Gly Ile Pro Ala Arg Phe Ser Gly
    50                  55                  60

Ser Gly Ser Gly Thr Asp Phe Thr Leu Thr Ile Ser Ser Leu Glu Pro
65                  70                  75                  80

Glu Asp Phe Ala Val Tyr Tyr Cys Gln Gln Ser Ser Asn Trp Pro Arg
                85                  90                  95

Thr Phe Gly Gln Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210>  114
<211>  447
<212>  PRT
<213>  Artificial sequence

<220>
<223>  Pembrolizumab heavy chain amino acid sequence

<400>  114

Gln Val Gln Leu Val Gln Ser Gly Val Glu Val Lys Lys Pro Gly Ala
1               5                   10                  15

Ser Val Lys Val Ser Cys Lys Ala Ser Gly Tyr Thr Phe Thr Asn Tyr
            20                  25                  30

Tyr Met Tyr Trp Val Arg Gln Ala Pro Gly Gln Gly Leu Glu Trp Met
        35                  40                  45

Gly Gly Ile Asn Pro Ser Asn Gly Gly Thr Asn Phe Asn Glu Lys Phe
    50                  55                  60

Lys Asn Arg Val Thr Leu Thr Thr Asp Ser Ser Thr Thr Thr Ala Tyr
65                  70                  75                  80

Met Glu Leu Lys Ser Leu Gln Phe Asp Asp Thr Ala Val Tyr Tyr Cys
                85                  90                  95

Ala Arg Arg Asp Tyr Arg Phe Asp Met Gly Phe Asp Tyr Trp Gly Gln
            100                 105                 110

Gly Thr Thr Val Thr Val Ser Ser Ala Ser Thr Lys Gly Pro Ser Val
        115                 120                 125

Phe Pro Leu Ala Pro Cys Ser Arg Ser Thr Ser Glu Ser Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Lys Thr Tyr Thr Cys Asn Val Asp His Lys
        195                 200                 205

Pro Ser Asn Thr Lys Val Asp Lys Arg Val Glu Ser Lys Tyr Gly Pro
    210                 215                 220

Pro Cys Pro Pro Cys Pro Ala Pro Glu Phe Leu Gly Gly Pro Ser Val
225                 230                 235                 240

Phe Leu Phe Pro Pro Lys Pro Lys Asp Thr Leu Met Ile Ser Arg Thr
                245                 250                 255

Pro Glu Val Thr Cys Val Val Val Asp Val Ser Gln Glu Asp Pro Glu
            260                 265                 270

Val Gln Phe Asn Trp Tyr Val Asp Gly Val Glu Val His Asn Ala Lys
        275                 280                 285

Thr Lys Pro Arg Glu Glu Gln Phe Asn Ser Thr Tyr Arg Val Val Ser
    290                 295                 300

Val Leu Thr Val Leu His Gln Asp Trp Leu Asn Gly Lys Glu Tyr Lys
305                 310                 315                 320

Cys Lys Val Ser Asn Lys Gly Leu Pro Ser Ser Ile Glu Lys Thr Ile
                325                 330                 335

Ser Lys Ala Lys Gly Gln Pro Arg Glu Pro Gln Val Tyr Thr Leu Pro
            340                 345                 350

Pro Ser Gln Glu Glu Met Thr Lys Asn Gln Val Ser Leu Thr Cys Leu
        355                 360                 365

Val Lys Gly Phe Tyr Pro Ser Asp Ile Ala Val Glu Trp Glu Ser Asn
    370                 375                 380

Gly Gln Pro Glu Asn Asn Tyr Lys Thr Thr Pro Pro Val Leu Asp Ser
385                 390                 395                 400

Asp Gly Ser Phe Phe Leu Tyr Ser Arg Leu Thr Val Asp Lys Ser Arg
                405                 410                 415

Trp Gln Glu Gly Asn Val Phe Ser Cys Ser Val Met His Glu Ala Leu
            420                 425                 430

His Asn His Tyr Thr Gln Lys Ser Leu Ser Leu Ser Leu Gly Lys
        435                 440                 445

<210>  115
<211>  218
<212>  PRT
<213>  Artificial sequence

<220>
<223>  Pembrolizumab light chain amino acid sequence

<400>  115

Glu Ile Val Leu Thr Gln Ser Pro Ala Thr Leu Ser Leu Ser Pro Gly
1               5                   10                  15

Glu Arg Ala Thr Leu Ser Cys Arg Ala Ser Lys Gly Val Ser Thr Ser
            20                  25                  30

Gly Tyr Ser Tyr Leu His Trp Tyr Gln Gln Lys Pro Gly Gln Ala Pro
        35                  40                  45

Arg Leu Leu Ile Tyr Leu Ala Ser Tyr Leu Glu Ser Gly Val Pro Ala
    50                  55                  60

Arg Phe Ser Gly Ser Gly Ser Gly Thr Asp Phe Thr Leu Thr Ile Ser
65                  70                  75                  80

Ser Leu Glu Pro Glu Asp Phe Ala Val Tyr Tyr Cys Gln His Ser Arg
                85                  90                  95

Asp Leu Pro Leu Thr Phe Gly Gly Gly Thr Lys Val Glu Ile Lys Arg
            100                 105                 110

Thr Val Ala Ala Pro Ser Val Phe Ile Phe Pro Pro Ser Asp Glu Gln
        115                 120                 125

Leu Lys Ser Gly Thr Ala Ser Val Val Cys Leu Leu Asn Asn Phe Tyr
    130                 135                 140

Pro Arg Glu Ala Lys Val Gln Trp Lys Val Asp Asn Ala Leu Gln Ser
145                 150                 155                 160

Gly Asn Ser Gln Glu Ser Val Thr Glu Gln Asp Ser Lys Asp Ser Thr
                165                 170                 175

Tyr Ser Leu Ser Ser Thr Leu Thr Leu Ser Lys Ala Asp Tyr Glu Lys
            180                 185                 190

His Lys Val Tyr Ala Cys Glu Val Thr His Gln Gly Leu Ser Ser Pro
        195                 200                 205

Val Thr Lys Ser Phe Asn Arg Gly Glu Cys
    210                 215

<210>  116
<211>  448
<212>  PRT
<213>  Artificial sequence

<220>
<223>  Ipilimumab heavy chain amino acid sequence

<400>  116

Gln Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Ser Ser Tyr
            20                  25                  30

Thr Met His Trp Val Arg Gln Ala Pro Gly Lys Gly Leu Glu Trp Val
        35                  40                  45

Thr Phe Ile Ser Tyr Asp Gly Asn Asn Lys Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Ile Tyr Tyr Cys
                85                  90                  95

Ala Arg Thr Gly Trp Leu Gly Pro Phe Asp Tyr Trp Gly Gln Gly Thr
            100                 105                 110

Leu Val Thr Val Ser Ser Ala Ser Thr Lys Gly Pro Ser Val Phe Pro
        115                 120                 125

Leu Ala Pro Ser Ser Lys Ser Thr Ser Gly Gly Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Gln Thr Tyr Ile Cys Asn Val Asn His Lys Pro Ser
        195                 200                 205

Asn Thr Lys Val Asp Lys Arg Val Glu Pro Lys Ser Cys Asp Lys Thr
    210                 215                 220

His Thr Cys Pro Pro Cys Pro Ala Pro Glu Leu Leu Gly Gly Pro Ser
225                 230                 235                 240

Val Phe Leu Phe Pro Pro Lys Pro Lys Asp Thr Leu Met Ile Ser Arg
                245                 250                 255

Thr Pro Glu Val Thr Cys Val Val Val Asp Val Ser His Glu Asp Pro
            260                 265                 270

Glu Val Lys Phe Asn Trp Tyr Val Asp Gly Val Glu Val His Asn Ala
        275                 280                 285

Lys Thr Lys Pro Arg Glu Glu Gln Tyr Asn Ser Thr Tyr Arg Val Val
    290                 295                 300

Ser Val Leu Thr Val Leu His Gln Asp Trp Leu Asn Gly Lys Glu Tyr
305                 310                 315                 320

Lys Cys Lys Val Ser Asn Lys Ala Leu Pro Ala Pro Ile Glu Lys Thr
                325                 330                 335

Ile Ser Lys Ala Lys Gly Gln Pro Arg Glu Pro Gln Val Tyr Thr Leu
            340                 345                 350

Pro Pro Ser Arg Asp Glu Leu Thr Lys Asn Gln Val Ser Leu Thr Cys
        355                 360                 365

Leu Val Lys Gly Phe Tyr Pro Ser Asp Ile Ala Val Glu Trp Glu Ser
    370                 375                 380

Asn Gly Gln Pro Glu Asn Asn Tyr Lys Thr Thr Pro Pro Val Leu Asp
385                 390                 395                 400

Ser Asp Gly Ser Phe Phe Leu Tyr Ser Lys Leu Thr Val Asp Lys Ser
                405                 410                 415

Arg Trp Gln Gln Gly Asn Val Phe Ser Cys Ser Val Met His Glu Ala
            420                 425                 430

Leu His Asn His Tyr Thr Gln Lys Ser Leu Ser Leu Ser Pro Gly Lys
        435                 440                 445

<210>  117
<211>  215
<212>  PRT
<213>  Artificial sequence

<220>
<223>  Ipilimumab light chain amino acid sequence

<400>  117

Glu Ile Val Leu Thr Gln Ser Pro Gly Thr Leu Ser Leu Ser Pro Gly
1               5                   10                  15

Glu Arg Ala Thr Leu Ser Cys Arg Ala Ser Gln Ser Val Gly Ser Ser
            20                  25                  30

Tyr Leu Ala Trp Tyr Gln Gln Lys Pro Gly Gln Ala Pro Arg Leu Leu
        35                  40                  45

Ile Tyr Gly Ala Phe Ser Arg Ala Thr Gly Ile Pro Asp Arg Phe Ser
    50                  55                  60

Gly Ser Gly Ser Gly Thr Asp Phe Thr Leu Thr Ile Ser Arg Leu Glu
65                  70                  75                  80

Pro Glu Asp Phe Ala Val Tyr Tyr Cys Gln Gln Tyr Gly Ser Ser Pro
                85                  90                  95

Trp Thr Phe Gly Gln Gly Thr Lys Val Glu Ile Lys Arg Thr Val Ala
            100                 105                 110

Ala Pro Ser Val Phe Ile Phe Pro Pro Ser Asp Glu Gln Leu Lys Ser
        115                 120                 125

Gly Thr Ala Ser Val Val Cys Leu Leu Asn Asn Phe Tyr Pro Arg Glu
    130                 135                 140

Ala Lys Val Gln Trp Lys Val Asp Asn Ala Leu Gln Ser Gly Asn Ser
145                 150                 155                 160

Gln Glu Ser Val Thr Glu Gln Asp Ser Lys Asp Ser Thr Tyr Ser Leu
                165                 170                 175

Ser Ser Thr Leu Thr Leu Ser Lys Ala Asp Tyr Glu Lys His Lys Val
            180                 185                 190

Tyr Ala Cys Glu Val Thr His Gln Gly Leu Ser Ser Pro Val Thr Lys
        195                 200                 205

Ser Phe Asn Arg Gly Glu Cys
    210                 215

<210> 118
<211> 444
<212> PRT
<213> Artificial sequence

<220>
<223> Cemiplimab heavy chain amino acid sequence

<400> 118

Glu Val Gln Leu Leu Glu Ser Gly Gly Val Leu Val Gln Pro Gly Gly
1               5                   10                  15

Ser Leu Arg Leu Ser Cys Ala Ala Ser Gly Phe Thr Phe Ser Asn Phe
            20                  25                  30

Gly Met Thr Trp Val Arg Gln Ala Pro Gly Lys Gly Leu Glu Trp Val
        35                  40                  45

Ser Gly Ile Ser Gly Gly Gly Arg Asp Thr Tyr Phe Ala Asp Ser Val
    50                  55                  60

Lys Gly Arg Phe Thr Ile Ser Arg Asp Asn Ser Lys Asn Thr Leu Tyr

65                  70                  75                  80

Leu Gln Met Asn Ser Leu Lys Gly Glu Asp Thr Ala Val Tyr Tyr Cys
                85                  90                  95

Val Lys Trp Gly Asn Ile Tyr Phe Asp Tyr Trp Gly Gln Gly Thr Leu
            100                 105                 110

Val Thr Val Ser Ser Ala Ser Thr Lys Gly Pro Ser Val Phe Pro Leu
        115                 120                 125

Ala Pro Cys Ser Arg Ser Thr Ser Glu Ser Thr Ala Ala Leu Gly Cys
    130                 135                 140

Leu Val Lys Asp Tyr Phe Pro Glu Pro Val Thr Val Ser Trp Asn Ser
145                 150                 155                 160

Gly Ala Leu Thr Ser Gly Val His Thr Phe Pro Ala Val Leu Gln Ser
                165                 170                 175

Ser Gly Leu Tyr Ser Leu Ser Ser Val Val Thr Val Pro Ser Ser Ser
            180                 185                 190

Leu Gly Thr Lys Thr Tyr Thr Cys Asn Val Asp His Lys Pro Ser Asn
        195                 200                 205

Thr Lys Val Asp Lys Arg Val Glu Ser Lys Tyr Gly Pro Pro Cys Pro
    210                 215                 220

Pro Cys Pro Ala Pro Glu Phe Leu Gly Gly Pro Ser Val Phe Leu Phe
225                 230                 235                 240

Pro Pro Lys Pro Lys Asp Thr Leu Met Ile Ser Arg Thr Pro Glu Val
                245                 250                 255

Thr Cys Val Val Val Asp Val Ser Gln Glu Asp Pro Glu Val Gln Phe
            260                 265                 270

Asn Trp Tyr Val Asp Gly Val Glu Val His Asn Ala Lys Thr Lys Pro
        275                 280                 285

Arg Glu Glu Gln Phe Asn Ser Thr Tyr Arg Val Val Ser Val Leu Thr
    290                 295                 300

Val Leu His Gln Asp Trp Leu Asn Gly Lys Glu Tyr Lys Cys Lys Val
305                 310                 315                 320

Ser Asn Lys Gly Leu Pro Ser Ser Ile Glu Lys Thr Ile Ser Lys Ala
                325                 330                 335

Lys Gly Gln Pro Arg Glu Pro Gln Val Tyr Thr Leu Pro Pro Ser Gln
            340                 345                 350

Glu Glu Met Thr Lys Asn Gln Val Ser Leu Thr Cys Leu Val Lys Gly
        355                 360                 365

Phe Tyr Pro Ser Asp Ile Ala Val Glu Trp Glu Ser Asn Gly Gln Pro
    370                 375                 380

Glu Asn Asn Tyr Lys Thr Thr Pro Pro Val Leu Asp Ser Asp Gly Ser
385                 390                 395                 400

Phe Phe Leu Tyr Ser Arg Leu Thr Val Asp Lys Ser Arg Trp Gln Glu
                405                 410                 415

Gly Asn Val Phe Ser Cys Ser Val Met His Glu Ala Leu His Asn His
            420                 425                 430

Tyr Thr Gln Lys Ser Leu Ser Leu Ser Leu Gly Lys
        435                 440

<210>  119
<211>  214
<212>  PRT
<213>  Artificial sequence

<220>
<223>  Cemiplimab light chain amino acid sequence

<400>  119

Asp Ile Gln Met Thr Gln Ser Pro Ser Ser Leu Ser Ala Ser Val Gly
1               5                   10                  15

Asp Ser Ile Thr Ile Thr Cys Arg Ala Ser Leu Ser Ile Asn Thr Phe
            20                  25                  30

Leu Asn Trp Tyr Gln Gln Lys Pro Gly Lys Ala Pro Asn Leu Leu Ile
        35                  40                  45

Tyr Ala Ala Ser Ser Leu His Gly Gly Val Pro Ser Arg Phe Ser Gly
    50                  55                  60

Ser Gly Ser Gly Thr Asp Phe Thr Leu Thr Ile Arg Thr Leu Gln Pro
65                  70                  75                  80

Glu Asp Phe Ala Thr Tyr Tyr Cys Gln Gln Ser Ser Asn Thr Pro Phe
                85                  90                  95

Thr Phe Gly Pro Gly Thr Val Val Asp Phe Arg Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210>  120
<211>  5
<212>  PRT
<213>  Artificial sequence

<220>
<223>  Peptide linker

<400>  120

Gly Gly Gly Gly Ser
1               5

<210>  121
<211>  10
<212>  PRT
<213>  Artificial sequence

<220>
<223>  Peptide linker


<400>  121

Gly Gly Gly Gly Ser Gly Gly Gly Gly Ser
1               5                   10


<210>  122
<211>  15
<212>  PRT
<213>  Artificial sequence

<220>
<223>  Peptide linker


<400>  122

Gly Gly Gly Gly Ser Gly Gly Gly Gly Ser Gly Gly Gly Gly Ser
1               5                   10                  15


<210>  123
<211>  20
<212>  PRT
<213>  Artificial sequence

<220>
<223>  Peptide linker


<400>  123

Gly Gly Gly Gly Ser Gly Gly Gly Gly Ser Gly Gly Gly Gly Ser Gly
1               5                   10                  15


Gly Gly Gly Ser
            20


<210>  124
<211>  600
<212>  PRT
<213>  Artificial sequence

<220>
<223>  Heavy chain amino acid sequence of Control protein 1

<400>  124

Gln Val Gln Leu Gln Glu Ser Gly Pro Gly Leu Val Lys Pro Ser Glu
1               5                   10                  15

Thr Leu Ser Leu Thr Cys Thr Val Ser Gly Phe Ser Leu Ser Arg Tyr
            20                  25                  30

Ser Val His Trp Ile Arg Gln Pro Pro Gly Lys Gly Leu Glu Trp Leu
        35                  40                  45

Gly Met Ile Trp Gly Val Gly Thr Thr Asp Tyr Asn Ser Ala Leu Lys
    50                  55                  60

Ser Arg Leu Thr Ile Ser Lys Asp Thr Ser Lys Asn Gln Phe Ser Leu
65                  70                  75                  80

Lys Leu Ser Ser Val Thr Ala Ala Asp Thr Ala Val Tyr Tyr Cys Ala
                85                  90                  95

Arg Asn Trp Gly Thr Ala Asp Tyr Phe Asp Tyr Trp Gly Gln Gly Thr
            100                 105                 110

Thr Val Thr Val Ser Ser Ala Ser Thr Lys Gly Pro Ser Val Phe Pro
        115                 120                 125

Leu Ala Pro Ser Ser Lys Ser Thr Ser Gly Gly Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Gln Thr Tyr Ile Cys Asn Val Asn His Lys Pro Ser
        195                 200                 205

Asn Thr Lys Val Asp Lys Lys Val Glu Pro Lys Ser Cys Asp Lys Thr
    210                 215                 220

His Thr Cys Pro Pro Cys Pro Ala Pro Glu Leu Leu Gly Gly Pro Ser
225                 230                 235                 240

Val Phe Leu Phe Pro Pro Lys Pro Lys Asp Thr Leu Met Ile Ser Arg
                245                 250                 255

Thr Pro Glu Val Thr Cys Val Val Val Asp Val Ser His Glu Asp Pro
            260                 265                 270

Glu Val Lys Phe Asn Trp Tyr Val Asp Gly Val Glu Val His Asn Ala
        275                 280                 285

Lys Thr Lys Pro Arg Glu Glu Gln Tyr Asn Ser Thr Tyr Arg Val Val
    290                 295                 300

Ser Val Leu Thr Val Leu His Gln Asp Trp Leu Asn Gly Lys Glu Tyr
305                 310                 315                 320

Lys Cys Lys Val Ser Asn Lys Ala Leu Pro Ala Pro Ile Glu Lys Thr
                325                 330                 335

Ile Ser Lys Ala Lys Gly Gln Pro Arg Glu Pro Gln Val Tyr Thr Leu
            340                 345                 350

Pro Pro Ser Arg Glu Glu Met Thr Lys Asn Gln Val Ser Leu Thr Cys
        355                 360                 365

Leu Val Lys Gly Phe Tyr Pro Ser Asp Ile Ala Val Glu Trp Glu Ser
    370                 375                 380

Asn Gly Gln Pro Glu Asn Asn Tyr Lys Thr Thr Pro Pro Val Leu Asp
385                 390                 395                 400

Ser Asp Gly Ser Phe Phe Leu Tyr Ser Lys Leu Thr Val Asp Lys Ser
                405                 410                 415

Arg Trp Gln Gln Gly Asn Val Phe Ser Cys Ser Val Met His Glu Ala
            420                 425                 430

Leu His Asn His Tyr Thr Gln Lys Ser Leu Ser Leu Ser Pro Gly Lys
        435                 440                 445

Gly Gly Gly Gly Ser Gly Gly Gly Gly Ser Gly Gly Gly Gly Ser Thr
    450                 455                 460

Ile Pro Pro His Val Gln Lys Ser Val Asn Asn Asp Met Ile Val Thr
465                 470                 475                 480

Asp Asn Asn Gly Ala Val Lys Phe Pro Gln Leu Cys Lys Phe Cys Asp
                485                 490                 495

Val Arg Phe Ser Thr Cys Asp Asn Gln Lys Ser Cys Met Ser Asn Cys
            500                 505                 510

Ser Ile Thr Ser Ile Cys Glu Lys Pro Gln Glu Val Cys Val Ala Val
        515                 520                 525

Trp Arg Lys Asn Asp Glu Asn Ile Thr Leu Glu Thr Val Cys His Asp
    530                 535                 540

Pro Lys Leu Pro Tyr His Asp Phe Ile Leu Glu Asp Ala Ala Ser Pro
545                 550                 555                 560

Lys Cys Ile Met Lys Glu Lys Lys Lys Pro Gly Glu Thr Phe Phe Met
                565                 570                 575

Cys Ser Cys Ser Ser Asp Glu Cys Asn Asp Asn Ile Ile Phe Ser Glu
            580                 585                 590

Glu Tyr Asn Thr Ser Asn Pro Asp
        595                 600

<210>  125
<211>  218
<212>  PRT
<213>  Artificial sequence

<220>
<223>  Light chain amino acid sequence of Control protein 1

<400>  125

Asp Ile Val Leu Thr Gln Ser Pro Ala Ser Leu Ala Val Ser Pro Gly
1               5                   10                  15

Gln Arg Ala Thr Ile Thr Cys Arg Ala Ser Lys Ser Val His Thr Ser
            20                  25                  30

Gly Tyr Ser Tyr Met His Trp Tyr Gln Gln Lys Pro Gly Gln Pro Pro
        35                  40                  45

Lys Leu Leu Ile Tyr Leu Ala Ser Asn Leu Glu Ser Gly Val Pro Ala
    50                  55                  60

Arg Phe Ser Gly Ser Gly Ser Gly Thr Asp Phe Thr Leu Thr Ile Asn
65                  70                  75                  80

Pro Val Glu Ala Asn Asp Thr Ala Asn Tyr Tyr Cys Gln His Ser Gly
                85                  90                  95

Glu Leu Pro Tyr Thr Phe Gly Gly Gly Thr Lys Val Glu Ile Lys Arg
            100                 105                 110

Thr Val Ala Ala Pro Ser Val Phe Ile Phe Pro Pro Ser Asp Glu Gln
        115                 120                 125

Leu Lys Ser Gly Thr Ala Ser Val Val Cys Leu Leu Asn Asn Phe Tyr
    130                 135                 140

Pro Arg Glu Ala Lys Val Gln Trp Lys Val Asp Asn Ala Leu Gln Ser
145                 150                 155                 160

Gly Asn Ser Gln Glu Ser Val Thr Glu Gln Asp Ser Lys Asp Ser Thr
                165                 170                 175

Tyr Ser Leu Ser Ser Thr Leu Thr Leu Ser Lys Ala Asp Tyr Glu Lys
            180                 185                 190

His Lys Val Tyr Ala Cys Glu Val Thr His Gln Gly Leu Ser Ser Pro
        195                 200                 205

Val Thr Lys Ser Phe Asn Arg Gly Glu Cys
    210                 215

<210>  126
<211>  653
<212>  PRT
<213>  Artificial sequence

<220>
<223>  Heavy chain amino acid sequence of Control protein 2

<400>  126

Gln Val Gln Leu Val Glu Ser Gly Gly Gly Val Val Gln Pro Gly Arg
1               5                   10                  15

Ser Leu Arg Leu Asp Cys Lys Ala Ser Gly Ile Thr Phe Ser Asn Ser
            20                  25                  30

Gly Met His Trp Val Arg Gln Ala Pro Gly Lys Gly Leu Glu Trp Val
        35                  40                  45

Ala Val Ile Trp Tyr Asp Gly Ser Lys Arg Tyr Tyr Ala Asp Ser Val
    50                  55                  60

Lys Gly Arg Phe Thr Ile Ser Arg Asp Asn Ser Lys Asn Thr Leu Phe
65                  70                  75                  80

Leu Gln Met Asn Ser Leu Arg Ala Glu Asp Thr Ala Val Tyr Tyr Cys
                85                  90                  95

Ala Thr Asn Asp Asp Tyr Trp Gly Gln Gly Thr Leu Val Thr Val Ser
            100                 105                 110

Ser Ala Ser Thr Lys Gly Pro Ser Val Phe Pro Leu Ala Pro Cys Ser
        115                 120                 125

Arg Ser Thr Ser Glu Ser Thr Ala Ala Leu Gly Cys Leu Val Lys Asp
    130                 135                 140

Tyr Phe Pro Glu Pro Val Thr Val Ser Trp Asn Ser Gly Ala Leu Thr
145                 150                 155                 160

Ser Gly Val His Thr Phe Pro Ala Val Leu Gln Ser Ser Gly Leu Tyr
                165                 170                 175

Ser Leu Ser Ser Val Val Thr Val Pro Ser Ser Ser Leu Gly Thr Lys
            180                 185                 190

Thr Tyr Thr Cys Asn Val Asp His Lys Pro Ser Asn Thr Lys Val Asp
        195                 200                 205

Lys Arg Val Glu Ser Lys Tyr Gly Pro Pro Cys Pro Pro Cys Pro Ala
    210                 215                 220

Pro Glu Phe Leu Gly Gly Pro Ser Val Phe Leu Phe Pro Pro Lys Pro
225                 230                 235                 240

Lys Asp Thr Leu Met Ile Ser Arg Thr Pro Glu Val Thr Cys Val Val
                245                 250                 255

Val Asp Val Ser Gln Glu Asp Pro Glu Val Gln Phe Asn Trp Tyr Val
            260                 265                 270

Asp Gly Val Glu Val His Asn Ala Lys Thr Lys Pro Arg Glu Glu Gln
        275                 280                 285

Phe Asn Ser Thr Tyr Arg Val Val Ser Val Leu Thr Val Leu His Gln
    290                 295                 300

Asp Trp Leu Asn Gly Lys Glu Tyr Lys Cys Lys Val Ser Asn Lys Gly
305                 310                 315                 320

Leu Pro Ser Ser Ile Glu Lys Thr Ile Ser Lys Ala Lys Gly Gln Pro
                325                 330                 335

Arg Glu Pro Gln Val Tyr Thr Leu Pro Pro Ser Gln Glu Glu Met Thr
            340                 345                 350

Lys Asn Gln Val Ser Leu Thr Cys Leu Val Lys Gly Phe Tyr Pro Ser
        355                 360                 365

Asp Ile Ala Val Glu Trp Glu Ser Asn Gly Gln Pro Glu Asn Asn Tyr
    370                 375                 380

Lys Thr Thr Pro Pro Val Leu Asp Ser Asp Gly Ser Phe Phe Leu Tyr
385                 390                 395                 400

Ser Arg Leu Thr Val Asp Lys Ser Arg Trp Gln Glu Gly Asn Val Phe
                405                 410                 415

Ser Cys Ser Val Met His Glu Ala Leu His Asn His Tyr Thr Gln Lys
            420                 425                 430

Ser Leu Ser Leu Ser Leu Gly Lys Gly Gly Gly Gly Ser Gly Gly Gly
        435                 440                 445

Gly Ser Gly Gly Gly Gly Ser Val Ile His Val Thr Lys Glu Val Lys
    450                 455                 460

Glu Val Ala Thr Leu Ser Cys Gly His Asn Val Ser Val Glu Glu Leu
465                 470                 475                 480

Ala Gln Thr Arg Ile Tyr Trp Gln Lys Glu Lys Lys Met Val Leu Thr
                485                 490                 495

Met Met Ser Gly Asp Met Asn Ile Trp Pro Glu Tyr Lys Asn Arg Thr
            500                 505                 510

Ile Phe Asp Ile Thr Asn Asn Leu Ser Ile Val Ile Leu Ala Leu Arg
        515                 520                 525

Pro Ser Asp Glu Gly Thr Tyr Glu Cys Val Val Leu Lys Tyr Glu Lys
    530                 535                 540

Asp Ala Phe Lys Arg Glu His Leu Ala Glu Val Thr Leu Ser Val Lys
545                 550                 555                 560

Ala Asp Phe Pro Thr Pro Ser Ile Ser Asp Phe Glu Ile Pro Thr Ser
                565                 570                 575

Asn Ile Arg Arg Ile Ile Cys Ser Thr Ser Gly Gly Phe Pro Glu Pro
            580                 585                 590

His Leu Ser Trp Leu Glu Asn Gly Glu Glu Leu Asn Ala Ile Asn Thr
        595                 600                 605

Thr Val Ser Gln Asp Pro Glu Thr Glu Leu Tyr Ala Val Ser Ser Lys
    610                 615                 620

Leu Asp Phe Asn Met Thr Thr Asn His Ser Phe Met Cys Leu Ile Lys
625                 630                 635                 640

Tyr Gly His Leu Arg Val Asn Gln Thr Phe Asn Trp Asn
                645                 650

<210>  127
<211>  214
<212>  PRT
<213>  Artificial sequence

<220>
<223>  Light chain amino acid sequence of Control protein 2

<400>  127

Glu Ile Val Leu Thr Gln Ser Pro Ala Thr Leu Ser Leu Ser Pro Gly
1               5                   10                  15

Glu Arg Ala Thr Leu Ser Cys Arg Ala Ser Gln Ser Val Ser Ser Tyr
            20                  25                  30

Leu Ala Trp Tyr Gln Gln Lys Pro Gly Gln Ala Pro Arg Leu Leu Ile
        35                  40                  45

Tyr Asp Ala Ser Asn Arg Ala Thr Gly Ile Pro Ala Arg Phe Ser Gly
    50                  55                  60

Ser Gly Ser Gly Thr Asp Phe Thr Leu Thr Ile Ser Ser Leu Glu Pro
65                  70                  75                  80

Glu Asp Phe Ala Val Tyr Tyr Cys Gln Gln Ser Ser Asn Trp Pro Arg
                85                  90                  95

Thr Phe Gly Gln Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210>  128
<211>  657
<212>  PRT
<213>  Artificial sequence

<220>
<223>  Heavy chain amino acid sequence of Control protein 3

<400>  128

Gln Val Gln Leu Val Glu Ser Gly Gly Gly Val Val Gln Pro Gly Arg
1               5                   10                  15

Ser Leu Arg Leu Asp Cys Lys Ala Ser Gly Ile Thr Phe Ser Asn Ser
            20                  25                  30

Gly Met His Trp Val Arg Gln Ala Pro Gly Lys Gly Leu Glu Trp Val
        35                  40                  45

Ala Val Ile Trp Tyr Asp Gly Ser Lys Arg Tyr Tyr Ala Asp Ser Val
    50                  55                  60

Lys Gly Arg Phe Thr Ile Ser Arg Asp Asn Ser Lys Asn Thr Leu Phe
65                  70                  75                  80

Leu Gln Met Asn Ser Leu Arg Ala Glu Asp Thr Ala Val Tyr Tyr Cys
                85                  90                  95

Ala Thr Asn Asp Asp Tyr Trp Gly Gln Gly Thr Leu Val Thr Val Ser
            100                 105                 110

Ser Ala Ser Thr Lys Gly Pro Ser Val Phe Pro Leu Ala Pro Cys Ser
        115                 120                 125

Arg Ser Thr Ser Glu Ser Thr Ala Ala Leu Gly Cys Leu Val Lys Asp
    130                 135                 140

Tyr Phe Pro Glu Pro Val Thr Val Ser Trp Asn Ser Gly Ala Leu Thr
145                 150                 155                 160

Ser Gly Val His Thr Phe Pro Ala Val Leu Gln Ser Ser Gly Leu Tyr
                165                 170                 175

Ser Leu Ser Ser Val Val Thr Val Pro Ser Ser Ser Leu Gly Thr Lys
            180                 185                 190

Thr Tyr Thr Cys Asn Val Asp His Lys Pro Ser Asn Thr Lys Val Asp
        195                 200                 205

Lys Arg Val Glu Ser Lys Tyr Gly Pro Pro Cys Pro Pro Cys Pro Ala
    210                 215                 220

Pro Glu Phe Leu Gly Gly Pro Ser Val Phe Leu Phe Pro Pro Lys Pro
225                 230                 235                 240

Lys Asp Thr Leu Met Ile Ser Arg Thr Pro Glu Val Thr Cys Val Val
                245                 250                 255

Val Asp Val Ser Gln Glu Asp Pro Glu Val Gln Phe Asn Trp Tyr Val
            260                 265                 270

Asp Gly Val Glu Val His Asn Ala Lys Thr Lys Pro Arg Glu Glu Gln
        275                 280                 285

Phe Asn Ser Thr Tyr Arg Val Val Ser Val Leu Thr Val Leu His Gln
    290                 295                 300

Asp Trp Leu Asn Gly Lys Glu Tyr Lys Cys Lys Val Ser Asn Lys Gly
305                 310                 315                 320

Leu Pro Ser Ser Ile Glu Lys Thr Ile Ser Lys Ala Lys Gly Gln Pro
                325                 330                 335

Arg Glu Pro Gln Val Tyr Thr Leu Pro Pro Ser Gln Glu Glu Met Thr
            340                 345                 350

Lys Asn Gln Val Ser Leu Thr Cys Leu Val Lys Gly Phe Tyr Pro Ser
        355                 360                 365

Asp Ile Ala Val Glu Trp Glu Ser Asn Gly Gln Pro Glu Asn Asn Tyr
    370                 375                 380

Lys Thr Thr Pro Pro Val Leu Asp Ser Asp Gly Ser Phe Phe Leu Tyr
385                 390                 395                 400

Ser Arg Leu Thr Val Asp Lys Ser Arg Trp Gln Glu Gly Asn Val Phe
                405                 410                 415

Ser Cys Ser Val Met His Glu Ala Leu His Asn His Tyr Thr Gln Lys
            420                 425                 430

Ser Leu Ser Leu Ser Leu Gly Lys Gly Gly Gly Gly Ser Gly Gly Gly
        435                 440                 445

Gly Ser Gly Gly Gly Gly Ser Gly Arg Pro Phe Val Glu Met Tyr Ser
    450                 455                 460

Glu Ile Pro Glu Ile Ile His Met Thr Glu Gly Arg Glu Leu Val Ile
465                 470                 475                 480

Pro Cys Arg Val Thr Ser Pro Asn Ile Thr Val Thr Leu Lys Lys Phe
                485                 490                 495

Pro Leu Asp Thr Leu Ile Pro Asp Gly Lys Arg Ile Ile Trp Asp Ser
            500                 505                 510

Arg Lys Gly Phe Ile Ile Ser Asn Ala Thr Tyr Lys Glu Ile Gly Leu
        515                 520                 525

Leu Thr Cys Glu Ala Thr Val Asn Gly His Leu Tyr Lys Thr Asn Tyr
    530                 535                 540

Leu Thr His Arg Gln Thr Asn Thr Ile Ile Asp Val Val Leu Ser Pro
545                 550                 555                 560

Ser His Gly Ile Glu Leu Ser Val Gly Glu Lys Leu Val Leu Asn Cys
                565                 570                 575

Thr Ala Arg Thr Glu Leu Asn Val Gly Ile Asp Phe Asn Trp Glu Tyr
            580                 585                 590

Pro Ser Ser Lys His Gln His Lys Lys Leu Val Asn Arg Asp Leu Lys
        595                 600                 605

Thr Gln Ser Gly Ser Glu Met Lys Lys Phe Leu Ser Thr Leu Thr Ile
    610                 615                 620

Asp Gly Val Thr Arg Ser Asp Gln Gly Leu Tyr Thr Cys Ala Ala Ser
625                 630                 635                 640

Ser Gly Leu Met Thr Lys Lys Asn Ser Thr Phe Val Arg Val His Glu
                645                 650                 655

Pro

<210>  129
<211>  214
<212>  PRT
<213>  Artificial sequence

<220>
<223>  Light chain amino acid sequence of Control protein 3

<400>  129

Glu Ile Val Leu Thr Gln Ser Pro Ala Thr Leu Ser Leu Ser Pro Gly
1               5                   10                  15

Glu Arg Ala Thr Leu Ser Cys Arg Ala Ser Gln Ser Val Ser Ser Tyr
            20                  25                  30

Leu Ala Trp Tyr Gln Gln Lys Pro Gly Gln Ala Pro Arg Leu Leu Ile
        35                  40                  45

Tyr Asp Ala Ser Asn Arg Ala Thr Gly Ile Pro Ala Arg Phe Ser Gly
    50                  55                  60

Ser Gly Ser Gly Thr Asp Phe Thr Leu Thr Ile Ser Ser Leu Glu Pro
65                  70                  75                  80

Glu Asp Phe Ala Val Tyr Tyr Cys Gln Gln Ser Ser Asn Trp Pro Arg
                85                  90                  95

Thr Phe Gly Gln Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210>  130
<211>  202
<212>  PRT
<213>  Artificial sequence

<220>
<223>  VEGF-R fragment

<400>  130

Gly Arg Pro Phe Val Glu Met Tyr Ser Glu Ile Pro Glu Ile Ile His
1               5                   10                  15

Met Thr Glu Gly Arg Glu Leu Val Ile Pro Cys Arg Val Thr Ser Pro
            20                  25                  30

Asn Ile Thr Val Thr Leu Lys Lys Phe Pro Leu Asp Thr Leu Ile Pro
        35                  40                  45

Asp Gly Lys Arg Ile Ile Trp Asp Ser Arg Lys Gly Phe Ile Ile Ser
    50                  55                  60

Asn Ala Thr Tyr Lys Glu Ile Gly Leu Leu Thr Cys Glu Ala Thr Val
65                  70                  75                  80

Asn Gly His Leu Tyr Lys Thr Asn Tyr Leu Thr His Arg Gln Thr Asn
                85                  90                  95

Thr Ile Ile Asp Val Val Leu Ser Pro Ser His Gly Ile Glu Leu Ser
            100                 105                 110

Val Gly Glu Lys Leu Val Leu Asn Cys Thr Ala Arg Thr Glu Leu Asn
        115                 120                 125

Val Gly Ile Asp Phe Asn Trp Glu Tyr Pro Ser Ser Lys His Gln His
    130                 135                 140

Lys Lys Leu Val Asn Arg Asp Leu Lys Thr Gln Ser Gly Ser Glu Met
145                 150                 155                 160

Lys Lys Phe Leu Ser Thr Leu Thr Ile Asp Gly Val Thr Arg Ser Asp
                165                 170                 175

Gln Gly Leu Tyr Thr Cys Ala Ala Ser Ser Gly Leu Met Thr Lys Lys
            180                 185                 190

Asn Ser Thr Phe Val Arg Val His Glu Pro
        195                 200

<210>  131
<211>  117
<212>  PRT
<213>  Artificial sequence

<220>
<223>  Human TGF-βRII

<400>  131

Gly Ala Val Lys Phe Pro Gln Leu Cys Lys Phe Cys Asp Val Arg Phe
1               5                   10                  15

Ser Thr Cys Asp Asn Gln Lys Ser Cys Met Ser Asn Cys Ser Ile Thr
            20                  25                  30

Ser Ile Cys Glu Lys Pro Gln Glu Val Cys Val Ala Val Trp Arg Lys
        35                  40                  45

Asn Asp Glu Asn Ile Thr Leu Glu Thr Val Cys His Asp Pro Lys Leu
    50                  55                  60

Pro Tyr His Asp Phe Ile Leu Glu Asp Ala Ala Ser Pro Lys Cys Ile
65                  70                  75                  80

Met Lys Glu Lys Lys Lys Pro Gly Glu Thr Phe Phe Met Cys Ser Cys
                85                  90                  95

Ser Ser Asp Glu Cys Asn Asp Asn Ile Ile Phe Ser Glu Glu Tyr Asn
            100                 105                 110

Thr Ser Asn Pro Asp
        115

<210>  132
<211>  115

<212>  PRT
<213>  Artificial sequence

<220>
<223>  Human TGF-βRII

<400>  132

Val Lys Phe Pro Gln Leu Cys Lys Phe Cys Asp Val Arg Phe Ser Thr
1               5                   10                  15

Cys Asp Asn Gln Lys Ser Cys Met Ser Asn Cys Ser Ile Thr Ser Ile
            20                  25                  30

Cys Glu Lys Pro Gln Glu Val Cys Val Ala Val Trp Arg Lys Asn Asp
        35                  40                  45

Glu Asn Ile Thr Leu Glu Thr Val Cys His Asp Pro Lys Leu Pro Tyr
    50                  55                  60

His Asp Phe Ile Leu Glu Asp Ala Ala Ser Pro Lys Cys Ile Met Lys
65                  70                  75                  80

Glu Lys Lys Lys Pro Gly Glu Thr Phe Phe Met Cys Ser Cys Ser Ser
                85                  90                  95

Asp Glu Cys Asn Asp Asn Ile Ile Phe Ser Glu Glu Tyr Asn Thr Ser
            100                 105                 110

Asn Pro Asp
        115


<210>  133
<211>  112
<212>  PRT
<213>  Artificial sequence

<220>
<223>  CD80  ECD

<400>  133

Val Ile His Val Thr Lys Glu Val Lys Glu Val Ala Thr Leu Ser Cys
1               5                   10                  15

Gly His Asn Val Ser Val Glu Glu Leu Ala Gln Thr Arg Ile Tyr Trp
            20                  25                  30

Gln Lys Glu Lys Lys Met Val Leu Thr Met Met Ser Gly Asp Met Asn
        35                  40                  45

Ile Trp Pro Glu Tyr Lys Asn Arg Thr Ile Phe Asp Ile Thr Asn Asn
    50                  55                  60

Leu Ser Ile Val Ile Leu Ala Leu Arg Pro Ser Asp Glu Gly Thr Tyr
65                  70                  75                  80

Glu Cys Val Val Leu Lys Tyr Glu Lys Asp Ala Phe Lys Arg Glu His
                85                  90                  95

Leu Ala Glu Val Thr Leu Ser Val Lys Ala Asp Phe Pro Thr Pro Ser
            100                 105                 110

<210>  134
<211>  288
<212>  PRT
<213>  Artificial sequence

<220>
<223>  CD80

<400>  134

Met Gly His Thr Arg Arg Gln Gly Thr Ser Pro Ser Lys Cys Pro Tyr
1               5                   10                  15

Leu Asn Phe Phe Gln Leu Leu Val Leu Ala Gly Leu Ser His Phe Cys
            20                  25                  30

Ser Gly Val Ile His Val Thr Lys Glu Val Lys Glu Val Ala Thr Leu
        35                  40                  45

Ser Cys Gly His Asn Val Ser Val Glu Glu Leu Ala Gln Thr Arg Ile
    50                  55                  60

Tyr Trp Gln Lys Glu Lys Lys Met Val Leu Thr Met Met Ser Gly Asp
65                  70                  75                  80

Met Asn Ile Trp Pro Glu Tyr Lys Asn Arg Thr Ile Phe Asp Ile Thr
                85                  90                  95

Asn Asn Leu Ser Ile Val Ile Leu Ala Leu Arg Pro Ser Asp Glu Gly
            100                 105                 110

Thr Tyr Glu Cys Val Val Leu Lys Tyr Glu Lys Asp Ala Phe Lys Arg
        115                 120                 125

Glu His Leu Ala Glu Val Thr Leu Ser Val Lys Ala Asp Phe Pro Thr
    130                 135                 140

Pro Ser Ile Ser Asp Phe Glu Ile Pro Thr Ser Asn Ile Arg Arg Ile
145                 150                 155                 160

Ile Cys Ser Thr Ser Gly Gly Phe Pro Glu Pro His Leu Ser Trp Leu
                165                 170                 175

Glu Asn Gly Glu Glu Leu Asn Ala Ile Asn Thr Thr Val Ser Gln Asp
            180                 185                 190

Pro Glu Thr Glu Leu Tyr Ala Val Ser Ser Lys Leu Asp Phe Asn Met
        195                 200                 205

Thr Thr Asn His Ser Phe Met Cys Leu Ile Lys Tyr Gly His Leu Arg
    210                 215                 220

Val Asn Gln Thr Phe Asn Trp Asn Thr Thr Lys Gln Glu His Phe Pro
225                 230                 235                 240

Asp Asn Leu Leu Pro Ser Trp Ala Ile Thr Leu Ile Ser Val Asn Gly
                245                 250                 255

Ile Phe Val Ile Cys Cys Leu Thr Tyr Cys Phe Ala Pro Arg Cys Arg
            260                 265                 270

Glu Arg Arg Arg Asn Glu Arg Leu Arg Arg Glu Ser Val Arg Pro Val
        275                 280                 285

<210>  135
<211>  256
<212>  PRT
<213>  Artificial sequence

<220>
<223>  CD80

<400>  135

Met Gly His Thr Arg Arg Gln Gly Thr Ser Pro Ser Lys Cys Pro Tyr
1               5                   10                  15

Leu Asn Phe Phe Gln Leu Leu Val Leu Ala Gly Leu Ser His Phe Cys
                20                  25                  30

Ser Gly Val Ile His Val Thr Lys Glu Val Lys Glu Val Ala Thr Leu
            35                  40                  45

Ser Cys Gly His Asn Val Ser Val Glu Glu Leu Ala Gln Thr Arg Ile
    50                  55                  60

Tyr Trp Gln Lys Glu Lys Lys Met Val Leu Thr Met Met Ser Gly Asp
65                  70                  75                  80

Met Asn Ile Trp Pro Glu Tyr Lys Asn Arg Thr Ile Phe Asp Ile Thr
                85                  90                  95

Asn Asn Leu Ser Ile Val Ile Leu Ala Leu Arg Pro Ser Asp Glu Gly
                100                 105                 110

Thr Tyr Glu Cys Val Val Leu Lys Tyr Glu Lys Asp Ala Phe Lys Arg
            115                 120                 125

Glu His Leu Ala Glu Val Thr Leu Ser Val Lys Ala Asp Phe Pro Thr
    130                 135                 140

Pro Ser Ile Ser Asp Phe Glu Ile Pro Thr Ser Asn Ile Arg Arg Ile
145                 150                 155                 160

Ile Cys Ser Thr Ser Gly Gly Phe Pro Glu Pro His Leu Ser Trp Leu
                165                 170                 175

Glu Asn Gly Glu Glu Leu Asn Ala Ile Asn Thr Thr Val Ser Gln Asp
            180                 185                 190

Pro Glu Thr Glu Leu Tyr Ala Val Ser Ser Lys Leu Asp Phe Asn Met
        195                 200                 205

Thr Thr Asn His Ser Phe Met Cys Leu Ile Lys Tyr Gly His Leu Arg
    210                 215                 220

Val Asn Gln Thr Phe Asn Trp Asn Thr Ser Phe Ala Pro Arg Cys Arg
225                 230                 235                 240

Glu Arg Arg Arg Asn Glu Arg Leu Arg Arg Glu Ser Val Arg Pro Val
                245                 250                 255

<210>  136
<211>  162
<212>  PRT
<213>  Artificial sequence

<220>
<223>  CD80

<400>  136

Met Gly His Thr Arg Arg Gln Gly Thr Ser Pro Ser Lys Cys Pro Tyr
1               5                   10                  15

Leu Asn Phe Phe Gln Leu Leu Val Leu Ala Gly Leu Ser His Phe Cys
            20                  25                  30

Ser Gly Val Ile His Val Thr Lys Glu Val Lys Glu Val Ala Thr Leu
        35                  40                  45

Ser Cys Gly His Asn Val Ser Val Glu Glu Leu Ala Gln Thr Arg Ile
    50                  55                  60

Tyr Trp Gln Lys Glu Lys Lys Met Val Leu Thr Met Met Ser Gly Asp
65                  70                  75                  80

Met Asn Ile Trp Pro Glu Tyr Lys Asn Arg Thr Ile Phe Asp Ile Thr
                85                  90                  95

Asn Asn Leu Ser Ile Val Ile Leu Ala Leu Arg Pro Ser Asp Glu Gly
            100                 105                 110

Thr Tyr Glu Cys Val Val Leu Lys Tyr Glu Lys Asp Ala Phe Lys Arg
        115                 120                 125

Glu His Leu Ala Glu Val Thr Leu Ser Val Lys Gly Phe Ala Pro Arg
    130                 135                 140

Cys Arg Glu Arg Arg Arg Asn Glu Arg Leu Arg Arg Glu Ser Val Arg
145                 150                 155                 160

Pro Val

<210>  137
<211>  137

<212>  PRT
<213>  Artificial sequence

<220>
<223>  TGF-βRII extracellular region fragment

<400>  137

Thr Ile Pro Pro His Val Gln Lys Ser Val Asn Asn Asp Met Ile Val
1               5                   10                  15

Thr Asp Asn Asn Gly Ala Val Lys Phe Pro Gln Leu Cys Lys Phe Cys
            20                  25                  30

Asp Val Arg Phe Ser Thr Cys Asp Asn Gln Lys Ser Cys Met Ser Asn
        35                  40                  45

Cys Ser Ile Thr Ser Ile Cys Glu Lys Pro Gln Glu Val Cys Val Ala
    50                  55                  60

Val Trp Arg Lys Asn Asp Glu Asn Ile Thr Leu Glu Thr Val Cys His
65                  70                  75                  80

Asp Pro Lys Leu Pro Tyr His Asp Phe Ile Leu Glu Asp Ala Ala Ser
                85                  90                  95

Pro Lys Cys Ile Met Lys Glu Lys Lys Lys Pro Gly Glu Thr Phe Phe
            100                 105                 110

Met Cys Ser Cys Ser Ser Asp Glu Cys Asn Asp Asn Ile Ile Phe Ser
        115                 120                 125

Glu Glu Tyr Asn Thr Ser Asn Pro Asp
    130                 135

## Description

### FIELD

The present disclosure relates to a bispecific fusion protein with a novel structure and application thereof. The present disclosure also relates to a polynucleotide, a nucleic acid construct and a host cell encoding or preparing the fusion protein.

### BACKGROUND

With the development of biotechnology, antibodies, as mature therapeutic drugs, play an important role in the treatment of tumors and immune diseases. Currently, most of the antibody drugs on the market target a single antigen and can only act on one target molecule. However, complex diseases are often multifactorial in nature, involving the overexpression or synergy of disease mediators, or the upregulation of different receptors, including molecular interactions in their signaling networks. Therefore, regulating multiple signal pathways can improve the efficacy of therapeutic antibodies. Bispecific antibody (BsAb) therapy using dual targeting strategies has become one of the effective treatments for tumors and immune diseases (Kontermann R. Dual targeting strategies with bispecific antibodies [C]//MAbs. Taylor & Francis, 2012, 4(2): 182-197.).

Bispecific antibodies can specifically bind to two different antigens or epitopes (Carter P. Bispecific human IgG by design [J]. Journal of immunological methods, 2001, 248(1-2): 7-15.). As early as the 1980s, Morrison et al. first prepared the first truly tetravalent bispecific antibody against dextran and dansyl group by linking single-chain antibodies with different specificities using flexible peptides for fusion expression (Coloma M J, Morrison S L. Design and production of novel tetravalent bispecific antibodies [J]. Nature biotechnology, 1997, 15(2): 159.). However, due to the bottleneck of antibody preparation technology and the lack of basic research on signal pathways, the development of bispecific antibodies has been hindered. With the in-depth understanding of disease pathogenesis and the rapid development of therapeutic monoclonal antibodies (McAbs)-related technologies, the technologies of antibody's construction, expression and purification has greatly improved, enabling the development of bispecific antibodies has the technology and motivation to overcome the limiting factors. At present, the mechanisms of bispecific or multispecific antibodies are mainly as follows: (1) bridging cells (in trans); (2) receptor inhibition (in-cis) or receptor activation (in-cis); (3) co-factor mimetic; (4) PIgGyback approaches (Aran F. Labrijn, et al. Bispecific antibodies: a mechanistic review of the pipeline, Nature Reviews Drug Discovery volume 18, pages 585-608(2019)).

In the development of bispecific antibodies, the choice of molecular structure is very important. Different bispecific antibody designs have their own advantages and disadvantages, but the design of bispecific antibodies for clinical treatment purposes must solve the following problems: first, ensuring the correct coupling or pairing of two pairs (or more) of different light chains and heavy chains; second, maintaining the independence of each binding domain of each monoclonal antibody, and when binding different epitopes at the same time, there will be no steric interference between each other; third, the antibody molecules should be easy to express in mammalian cells without complicated protein modification process; fourthly, good druggability is required, such as high thermal stability, high chemical stability, high solubility, not easy to polymerize, low viscosity, high expression, suitable half-life, and the like (Spiess C, Zhai Q, Carter P J. Alternative molecular formats and therapeutic applications for bispecific antibodies [J]. Molecular immunology, 2015, 67(2): 95-106.).

According to different molecular structure forms (i.e., components and construction methods), bispecific antibodies can be divided into many types. For example, according to the left-right symmetry of the structure, they are divided into symmetrical and asymmetrical structures; according to the presence or absence of Fc region, they can be divided into bispecific antibodies containing Fc region and bispecific antibodies devoid of Fc region; and according to the number of antigen-binding regions, they are divided into bivalent, trivalent, tetravalent or multivalent configurations, etc.. At present, bispecific antibodies have developed dozens of structures (Spiess C, Zhai Q, Carter P J. Alternative molecular formats and therapeutic applications for bispecific antibodies [J]. Molecular immunology, 2015, 67(2): 95-106.), these dozens of bispecific antibodies have diverse structures, roughly divided into 5 categories, including IgG-like bispecific antibodies, IgG-like bispecific antibodies with additional functional regions, bispecific antibodies with different antigen-binding fragments linked in series, fusion protein-type bispecific antibodies, and chemically coupled bispecific antibodies. Among them, the construction of IgG-like bispecific antibodies mainly employs recombinant DNA technology for structural modification. The constructed IgG-like bispecific antibodies have complete crystallizable fragments (i.e., Fc fragments), and thus they can bind to different antigens while still retaining Fc fragment-mediated ADCC (antibody-dependent cell-mediated cytotoxicity) and CDC (complement-mediated cytotoxicity, complement-dependent cytotoxicity) and the like, and at the same time, this type of antibody also retains the feature of extending the *in vivo* half-life of the antibody by binding to the neonatal Fc receptor (FcRn) (Ridgway J B B, Presta L G, Carter P. 'Knobs-into-holes' engineering of antibody CH3 domains for heavy chain heterodimerization [J]. Protein Engineering, Design and Selection, 1996, 9(7): 617-621.); IgG-like bispecific antibodies with additional functional regions are generally based on traditional IgG antibodies and generated by adding other specific antigen-binding fragments (i.e., additional functional regions, such as single domain antibodies, single chain antibodies, etc.) to the heavy chain and/or light chain by means of fusion proteins (LaFleur D, Abramyan D, Kanakaraj P, et al. Monoclonal antibody therapeutics with up to five specificities: functional enhancement through fusion of target-specific peptides [C]//MAbs. Taylor & Francis, 2013, 5(2): 208-218.); bispecific antibodies with different antigen-binding fragments linked in series are obtained by linking different Fabs, single-chain antibodies, single-domain antibodies, or antigen-binding fragments in a certain sequence through a linking peptide (Stork R, Müller D, Kontermann R E. A novel tri-functional antibody fusion protein with improved pharmacokinetic properties generated by fusing a bispecific single-chain diabody with an albumin-binding domain from streptococcal protein G [J]. Protein Engineering, Design & Selection, 2007, 20(11): 569-576.); fusion protein-type bispecific antibodies are protein molecules that can bind two or more antigens at the same time, formed by linking antibodies/antibody fragments (IgG, Fab, scFv, etc.) with different specificities through a linker such as a polypeptide fragment; and chemically coupled bispecific antibodies are similar to fusion protein-type bispecific antibodies, while for the chemically coupled bispecific antibodies, two antibodies/antibody fragments must be prepared separately first, and then the two are coupled through chemical bonds or both are coupled to a carrier protein.

In addition, with the development of basic research on signal pathways and the expansion of bi-antibody mechanisms, there are more and more targets and combinations available for bispecific antibody selection, such as PD-1, PD-L1, CTLA-4, LAG-3, FGL1, TIM-3, Galectin-9, TIGIT, CD155 and TGF-β receptor (TGF-βR), CD80, CD86, VEGFR, VEGF-trap, FGL1, CD70, 4-1BBL, OX40L, and SIRPα; and tumor antigens including Claudin 18.2, HER-2, Mesothelin, BCMA, SSTR2, GPRC5D, PSMA, FCRH5, CD33, CD123, CD20, A33, CEA, CD28, DLL3, EGFR, VEGFR, VEGFR2, VEGF-A, Nectin-4, FGFR, C-met, RANKL, PDGF, PDGFR, PDGFRα, DLL4, Ang-1, and Ang-2, etc. Combinations of immune checkpoint targets or tumor targets with other targets relating to resisting the development of cancer cells or activating T-cells, or the combination of multiple immune checkpoint targets greatly improves the possibility of treating malignant diseases such as tumors. In the early stage, the combination of two separate checkpoint antibodies was used clinically to improve the efficacy. For example, compared with treatment with ipilimumab alone, the treatment with the combination of ipilimumab (anti-CTLA4) and nivolumab (anti-PD1) can improve the survival rate of patients with melanoma. Currently, the safety and early efficacy of the four PD-1×CTLA4 bsAbs are being evaluated in early clinical trials. The concept of inhibitors blocking two immune checkpoints is also used clinically for other target combinations, such as PD-1×LAG 3, PD-1×TIM3, and PD-L1×CTLA 4. (Wolchok, J. D. et al. Overall Survival with Combined Nivolumab and Ipilimumab in Advanced Melanoma, N.Engl. J. Med. 377(14): 1345-1356, (2017); Dovedi, S. J. et al. MEDI5752: A novel bispecific antibody that preferentially targets CTLA-4 on PD-1 expressing T-cells. Cancer Res.78(13), Supplement.Abstract 2776: (2018). Hedvat, M. et al. Simultaneous checkpoint -checkpoint or checkpoint - costimulatory receptortargeting with bispecific antibodies promotes enhanced human T cell activation [abstract P664]. Presented at the 2018 Society for Immunotherapyof Cancer (SITC) (2018); Aran F. Labrijn, et al. Bispecific antibodies: a mechanistic review of the pipeline, Nature Reviews Drug Discovery volume 18, pages 585-608(2019).

At present, most of the bispecific antibodies are still in the clinical or preclinical research stage, and only three bispecific antibodies have been approved for marketing. They are Trion Pharma's Catumaxomab (delisted due to commercial factors in 2017), Amgen's Blinatumomab and Roche's Emicizumab. Catumaxomab is an IgG-like bispecific antibody in the form of Triomab. It targets T cells and tumor cells on which epithelial cell adhesion molecules (EpCAMs) overexpress, and inhibits tumor cells through T cell activation, antibody-dependent cell-mediated cytotoxicity (ADCC), and complement-dependent cytotoxicity (CDC). It was approved by EMA in 2009 for the treatment of malignant ascites caused by EpCAM-positive tumors that were ineffective or unfeasible against the standard treatment, and it is also the first bispecific antibody to be approved for marketing. Blinatumomab is a bispecific antibody without Fc region and adopts the structure form of BiTE (bispecific T cell engager). Blinatumomab binds to T cells through the anti-CD3 part, and to malignant and normal B cells through the anti-CD 19 part, thereby inducing T cell-mediated tumor cell killing effect. In 2014, it was approved by FDA for the treatment of Philadelphia chromosome-negative precursor B-cell acute lymphoblastic leukemia. Emicizumab is a modified humanized bispecific IgG4 monoclonal antibody that can bind to factor IXa and factor X. This antibody adopts "Knobs-into-Holes (KiH)" for Fc design, "Common Light Chain-IgG (CLC-IgG)" for LC design, and "Multidimensional Optimization" for variable region optimization, and was approved by FDA in 2017 for routine prevention of hemophilia A in the presence of factor VIII inhibitors.

**Table 1. Bispecific antibodies that have been approved for marketing (including delisted)**

| **Bispecific antibody** | **Trade name** | **Research unit** | **Time of first approval** |
|---|---|---|---|
| Catumaxomab | Removab | Trion Pharma | 2009 (EMA) (delisted in 2017) |
| Blinatumomab | Blincyto | Amgen | 2014 (FDA) |
| Emicizumab | Hemlibra | Roche | 2017 (FDA) |

Although there are currently dozens of bispecific antibodies/platforms and research and development experience from 3 marketed drugs, there are more problems and challenges in the development of bispecific antibodies compared to general antibodies due to their complex structures and diverse functional characteristics. For example, IgG-like bispecific antibodies face the problem of mismatches of heavy chain-heavy chain or heavy chain-light chain during the preparation process, the most typical of which is Catumaxomab. Although Catumaxomab is the first bispecific antibody approved for marketing, there are some very obvious limitations, which are mainly reflected in the complex production process of Triomab antibody and the immunogenicity problem that heterologous antibodies are relatively prone to emerge, and in 2017, the drug was delisted due to commercial factors; for another example, bispecific antibodies with different antigen-binding fragments linked in series have a short half-life due to the lack of constant regions, especially the lack of Fc fragments, and their expression level and intracellular stability after expression are generally lower than traditional IgG (Stork R, Müller D, Kontermann R E. A novel tri-functional antibody fusion protein with improved pharmacokinetic properties generated by fusing a bispecific single-chain diabody with an albumin-binding domain from streptococcal protein G [J]. Protein Engineering, Design & Selection, 2007, 20(11): 569-576.). In addition, most of this type of antibody is difficult to adopt affinity purification means in the purification process, so the purification process and the analysis of impurities in the finished product are also more complicated than traditional IgG (Tan P H, Sandmaier B M, Stayton P S. Contributions of a highly conserved VH/VL hydrogen bonding interaction to scFv folding stability and refolding efficiency [J]. Biophysical journal, 1998, 75(3): 1473-1482.), the most typical of which is Blinatumomab. Blinatumomab has a small molecular weight because of lacking Fc fragments, and its half-life in the blood is only 1.25±0.63 h, which is much shorter than the half-life of an intact antibody, so it requires continuous intravenous injection for 4 weeks to reach the effective dose, and the reagent needs to be additionally equipped with a continuous infusion device when used. Besides, it has poor stability and easily forms aggregates during production, which also affects the quality of the product and increases production costs. The current bispecific antibodies have many problems, such as low expression, poor stability, complex production processes, and significantly higher research and development costs than monoclonal antibodies, all of which limit the development of bispecific antibodies. Therefore, there is an urgent need to develop bispecific antibodies with new structures to provide more clinical options.

### SUMMARY

In response to the above problems, the present disclosure provides a bispecific fusion protein with a novel structure, which belongs to an IgG-like bispecific fusion protein with additional functional regions. Specifically, the fusion protein has a structure in which a second binding domain is inserted in the hinge region of a full-length immunoglobulin G (IgG) through an optional peptide linker, and the Fab region of the IgG is a first binding domain of the fusion protein; after inserting the second binding domain, the heavy chain of the IgG is the heavy chain of the fusion protein, and the light chain of the IgG is the light chain of the fusion protein; the second binding domain is selected from a human receptor or ligand, an extracellular region fragment of the receptor or ligand, a binding domain fragment, and a fragment combination of the receptor or ligand; wherein the receptor or ligand forms a dimerization or multimerization structure in a natural signaling pathway to activate or inhibit the signaling pathway, and the second binding domain and the first binding domain target different targets. The bispecific fusion protein with the above structure was named as Hibody (Hinge-insertion bispecific antibody) structure fusion protein (hereinafter referred to as Hibody structure fusion protein or Hibody).

Further, the first binding domain targets and binds to an immune checkpoint molecule or a tumor antigen.

Further, the immune checkpoint molecule includes PD-1 (*Programmed cell death protein 1),* PD-L1 (*Programmed cell death 1 ligand 1*)*,* CTLA-4 (*Cytotoxic T lymphocyte-associated antigen-4*), LAG-3 (*Lymphocyte activation gene-3*)*,* FGL1 (*Fibrinogen-like protein 1*), TIM-3 (*T cell immunoglobulin-3*)*,* Galectin-9, TIGIT (*T-cell immunoreceptor with Ig and ITIM domains*)*,* CD155, CD47; the tumor antigen includes Claudin 18.2, Her-2 (*Human epidermalgrowth factor receptor-2*)*,* Mesothelin, BCMA (*B Cell Maturation Antigen*)*,* SSTR2 (*Somatostatin receptor 2*)*,* GPRC5D (*G-protein coupled receptor family C group 5 member D*)*,* PSMA (*Prostate specific membrane antigen*)*,* FcRH5 (*Fc receptor-like protein 5*), CD33, CD123, CD20, A33, CEA (*Carcino-embryonic antigen*)*,* CD28, DLL3 (*Delta-like protein 3*)*,* EGFR (*Epidermal growth factor receptor*)*,* VEGFR (*Vascular Endothelial Growth Factor Receptor*)*,* VEGFR2 (*Vascular Endothelial Growth Factor Receptor 2*)*,* VEGF-A (*Vascular endothelial growth factor- A*)*,* Nectin-4, FGFR (*Fibroblast growth factor receptors*)*,* C-met, RANKL (*Receptor activator of nuclear factor kappa-B ligand*)*,* PDGF (*Platelet Derived Growth Factor*)*,* PDGFR (*Platelet Derived Growth Factor Receptor*)*,* PDGFRα (*Platelet Derived Growth Factor Receptor α*), DLL4 (*Delta-like ligand 4*)*,* Ang-1 (*Angiopoietin-1*), and Ang-2 (*Angiopoietin-2*)*.*

Further, the second binding domain targets and binds to human TGF-β (*Transforming growth factor-β*), CTLA-4, VEGF, LAG3, CD27, 4-1BB (*CD137*), OX40 (*CD134*), CD47, FGL1 (*Fibrinogen Like Protein 1*)*,* TLT-2 (*Trem-like transcript 2*)*,* CD28, HGF (Hepatocyte growth factor), CSF1 (*Colony-stimulating factor 1*)*,* CXCL1 (*CXC chemokine ligand 1*)*,* CXCL2 (*CXC chemokine ligand 2*)*,* CXCL3 (*CXC chemokine ligand 3*)*,* CXCL5 (*CXC chemokine ligand 5*)*,* CXCL6 (*CXC chemokine ligand 6*)*,* CXCL7 (*CXC chemokine ligand 7*), CXCL8 (*CXC chemokine ligand 8*)*,* CXCL9 (*CXC chemokine ligand 9*)*,* CXCL10 (*CXC chemokine ligand 10*)*,* CXCL12 (*CXC chemokine ligand 12*)*,* GITR (*Glucocorticoid-induced tumor necrosis factor receptor*)*,* EGF (*Epidermal Growth Factor*)*,* and ICOSL (*Inducible co-stimulator ligand*)*.*

Further, the receptor or ligand is human TGF-β receptor (TGF-βR), CD80, CD86, VEGFR, VEGF-trap, FGL1, CD70, 4-1BBL, OX40L, SIRPα (*Signal regulatory protein α*), B7-H3 (*CD276*), C-met, CSF1R (*Colony-stimulating factor 1 receptor*)*,* CXCR2 (*CXC chemokine receptor 2*)*,* CXCR3 (*CXC chemokine receptor 3*)*,* CXCR4 (*CXC chemokine receptor* 4), GITRL (*Glucocorticoid-induced TNF receptor ligand*)*,* EGFR, and ICOS (*Inducible co-stimulator*).

Among them, CD80 and CD86 are transmembrane glycoproteins, which are members of the immunoglobulin superfamily (IgSF). The mature CD80 molecule consists of 254 amino acids, including 208 amino acids in the extracellular domain (ECD), 25 amino acids in the transmembrane domain, and 21 amino acids in the intracellular domain. Similarly, the mature CD86 molecule consists of 303 amino acids, including 222 amino acids in the extracellular domain, 20 amino acids in the transmembrane domain, and 61 amino acids in the intracellular domain. The extracellular domains of CD80 and CD86 include immunoglobulin V (IgV) and immunoglobulin C (IgC) regions. CD80 and CD86 bind to their ligands CD28 and CTLA-4 through the immunoglobulin V (IgV) region. In the case of CD80 and CD86 binding to CD28, CD80 and CD86 have important regulatory effects on antigen-induced T cell activation, proliferation, and production of effector function, acting as positive regulators; while in the case of CD80 and CD86 binding to CTLA-4, CD80 and CD86 down-regulates the immune response, acting as negative regulators. Therefore, CD80 and CD86 are co-stimulatory factors when T lymphocytes are activated, and they play an important role in autoimmune monitoring, humoral immune response and transplantation response. Different from using agonistic anti-CD28 antibodies to activate T cells, using CD28's ligand CD80 as an activating factor to activate T cells does not trigger a severe cytokine storm, thereby greatly reducing the possibility of endangering the life of the patient.

In some embodiments, CD80 ECD is selected from human CD80 (such as the human CD80 of SEQ ID NO: 134) or human CD80 ECD derived from CD80 isotype 2 or isotype 3 (SEQ ID NO: 135 and 136). The CD80 ECD comprises CD80 immunoglobulin V (IgV) region (CD80-IgV, SEQ ID NO: 133). In one embodiment, the CD80 ECD comprises human CD80 immunoglobulin V region and C region (CD80-IgVIgC, SEQ ID NO: 32). In one embodiment, the CD80 ECD is human CD80 ECD. In one embodiment, the CD80 ECD comprises human CD80 IgV.

CD80-IgV (SEQ ID NO: 133):

VIHVTKEVKE VATLSCGHNV SVEELAQTRI YWQKEKKMVL TMMSGDMNIW PEYKNRTIFD 60

ITNNLSIVIL ALRPSDEGTY ECVVLKYEKD AFKREHLAEV TLSVKADFPT PS 112

CD80-IgVIgC (SEQ ID NO: 32):

VIHVTKEVKE VATLSCGHNV SVEELAQTRI YWQKEKKMVL TMMSGDMNIW PEYKNRTIFD 60

ITNNLSIVIL ALRPSDEGTY ECVVLKYEKD AFKREHLAEV TLSVKADFPT PSISDFEIPT 120

SNIRRIICST SGGFPEPHLS WLENGEELNA INTTVSQDPE TELYAVSSKL DFNMTTNHSF 180

MCLIKYGHLR VNQTFNWN 198

CD80 isotype 1 (SEQ ID NO: 134):

MGHTRRQGTS PSKCPYLNFF QLLVLAGLSH FCSGVIHVTK EVKEVATLSC GHNVSVEELA 60

QTRIYWQKEK KMVLTMMSGD MNIWPEYKNR TIFDITNNLS IVILALRPSD EGTYECVVLK 120

YEKDAFKREH LAEVTLSVKA DFPTPSISDF EIPTSNIRRI ICSTSGGFPE PHLSWLENGE 180

ELNAINTTVS QDPETELYAV SSKLDFNMTT NHSFMCLIKY GHLRVNQTFN WNTTKQEHFP 240

DNLLPSWAIT LISVNGIFVI CCLTYCFAPR CRERRRNERL RRESVRPV 288

CD80 isotype 2 (SEQ ID NO: 135):

MGHTRRQGTS PSKCPYLNFF QLLVLAGLSH FCSGVIHVTK EVKEVATLSC GHNVSVEELA 60

QTRIYWQKEK KMVLTMMSGD MNIWPEYKNR TIFDITNNLS IVILALRPSD EGTYECVVLK 120

YEKDAFKREH LAEVTLSVKA DFPTPSISDF EIPTSNIRRI ICSTSGGFPE PHLSWLENGE 180

ELNAINTTVS QDPETELYAV SSKLDFNMTT NHSFMCLIKY GHLRVNQTFN WNTSFAPRCR 240

ERRRNERLRR ESVRPV 256

CD80 isotype 3 (SEQ ID NO: 136):

MGHTRRQGTS PSKCPYLNFF QLLVLAGLSH FCSGVIHVTK EVKEVATLSC GHNVSVEELA 60

QTRIYWQKEK KMVLTMMSGD MNIWPEYKNR TIFDITNNLS IVILALRPSD EGTYECVVLK 120

YEKDAFKREH LAEVTLSVKG FAPRCRERRR NERLRRESVR PV 162

Further, the first binding domain and the second binding domain are selected from the following combinations:
(1) The first binding domain targets and binds to PD-L1, and the second binding domain targets and binds to human TGF-β, VEGF, FGL1, CD47, CD155, HGF, CSF1, CXCL1, CXCL2, CXCL3, CXCL5, CXCL6, CXCL7, CXCL8, CXCL9, CXCL10, CXCL12, EGF or ICOSL; or
(2) The first binding domain targets and binds to PD-1, and the second binding domain targets and binds to human CTLA-4, VEGF, HGF, EGF, CD28, LAG3, CD27, 4-1BB or OX40; or
(3) The first binding domain targets and binds to CTLA-4, and the second binding domain targets and binds to human CD28, VEGF, HGF, EGF, LAG3, CD27, 4-1BB or OX40; or
(4) The first binding domain targets and binds to LAG-3, and the second binding domain targets and binds to human CD28, VEGF, HGF, EGF, CTLA-4, CD27, 4-1BB or OX40; or
(5) The first binding domain targets and binds to FGL1, and the second binding domain targets and binds to human TGF-β, VEGF, CD47, CD155, HGF, CSF1, CXCL1, CXCL2, CXCL3, CXCL5, CXCL6, CXCL7, CXCL8, CXCL9, CXCL10, CXCL12, EGF or ICOSL; or
(6) The first binding domain targets and binds to TIM-3, and the second binding domain targets and binds to human VEGF, HGF, EGF, LAG3, CD27, 4-1BB or OX40; or
(7) The first binding domain targets and binds to Galectin-9, and the second binding domain targets and binds to human TGF-β, VEGF, CD47, CD155, HGF, CSF1, CXCL1, CXCL2, CXCL3, CXCL5, CXCL6, CXCL7, CXCL8, CXCL9, CXCL10, CXCL12, EGF or ICOSL; or
(8) The first binding domain targets and binds to TIGIT, and the second binding domain targets and binds to human TGF-β, HGF, EGF or VEGF; or
(9) The first binding domain targets and binds to CD155, and the second binding domain targets and binds to human TGF-β, VEGF, HGF, EGF, CD47 or CD155; or
(10) The first binding domain targets and binds to Claudin 18.2, HER-2, mesothelin, BCMA, SSTR2, GPRC5D, PSMA, FCRH5, CD33, CD123, CD20, A33, CEA, CD28, DLL3, EGFR, VEGFR, VEGFR2, VEGF-A, Nectin-4, FGFR, C-met, RANKL, PDGF, PDGFR, PDGFRα, DLL-4, Ang-1 or Ang-2, and the second binding domain targets and binds to human CTLA-4, TGF-β, VEGF, FGL1, LAG3, 4-1BB, OX40, CD27, CD28, CD47, CD155, HGF, CSF1, CXCL1, CXCL2, CXCL3, CXCL5, CXCL6, CXCL7, CXCL8, CXCL9, CXCL10, CXCL12, EGF or ICOSL.

Further, the first binding domain and the receptor or ligand are selected from the following combinations:
(1) The first binding domain targets and binds to PD-L1, and the receptor or ligand is selected from TGF-βRII, VEGFR, LAG-3, SIRPα, TIGIT, C-MET, CSF1R, CXCR2, CXCR3, CXCR4, EGFR or ICOS; or
(2) The first binding domain targets and binds to PD-1, and the receptor or ligand is selected from human CD80, CD86, VEGFR, c-MET, EGFR, FGL1, CD70, 4-1BBL or OX40L; or
(3) The first binding domain targets and binds to CTLA-4, and the receptor or ligand is selected from human CD80, CD86, VEGFR, c-MET, EGFR, FGL1, CD70, 4-1BBL or OX40L; or
(4) The first binding domain targets and binds to LAG-3, and the receptor or ligand is selected from human VEGFR, c-MET, EGFR, CD80, CD86, CD70, 4-1BBL or OX40L; or
(5) The first binding domain targets and binds to FGL1, and the receptor or ligand is selected from human TGF-βRII, VEGFR, SIRPα, TIGIT, c-MET, CSF1R, CXCR2, CXCR3, CXCR4, EGFR or ICOS; or
(6) The first binding domain targets and binds to TIM-3, and the receptor or ligand is selected from human VEGFR, c-MET, EGFR, FGL1, CD70, 4-1BBL or OX40L; or
(7) The first binding domain targets and binds to Galectin-9, and the receptor or ligand is selected from human TGF-βRII, VEGFR, SIRPα, TIGIT, c-MET, CSF1R, CXCR2, CXCR3, CXCR4, EGFR or ICOS; or
(8) The first binding domain targets and binds to TIGIT, and the receptor or ligand is selected from human TGF-βRII, c-MET, EGFR or VEGFR; or
(9) The first binding domain targets and binds to CD155, and the receptor or ligand is selected from human TGF-βRII, VEGFR, c-MET, EGFR, SIRPα or TIGIT; or
(10) The first binding domain targets and binds to Claudin 18.2, HER-2, mesothelin, BCMA, SSTR2, GPRC5D, PSMA, FCRH5, CD33, CD123, CD20, A33, CEA, CD28, DLL3, EGFR, VEGFR, VEGFR2, Nectin-4, FGFR, c-MET, RANKL, PDGF, PDGFR, PDGFRα, DLL4, Ang-1 or Ang-2, and the receptor or ligand is selected from human CTLA-4, CD80, CD86, TGF-βRII, VEGFR, FGL1, LAG3, 4-1BB, OX40, CD27, CD28, CD70, c-MET, SIRPα, TIGIT, CSF1R, CXCR2, CXCR3, CXCR4, EGFR or ICOS.

Further, the fragment of the receptor or ligand comprises a fragment of the extracellular region and a fragment of the binding domain of the receptor or ligand.

Further, the first binding domain targets and binds to PD-L1; the second binding domain is a fragment of human TGF-βRII; or the first binding domain targets and binds to PD-1, and the second binding domain comprises human CD80 ECD, CD80 IgV region, human CD80 IgVIgC, VEGFR1 ECD, VEGFR2 ECD or a combination of the second extracellular region of VEGFR1 and the third extracellular region of VEGFR2; or the first binding domain targets and binds to Claudin 18.2, HER-2 or EGFR, and the second binding domain is selected from human CD80 ECD, CD80 IgV region, human CD80 IgVIgC, VEGFR1 ECD, VEGFR2 ECD or a combination of the second extracellular region of VEGFR1 and the third extracellular region of VEGFR2.

Further, the second binding domain is selected from:
(1) Human TGF-βRII comprising the sequence as shown in SEQ ID NO: 31, 131 or 132, or a sequence having an identity of greater than 80%, 85%, 90%, 95%, 96%, 97%, 98%, 99% compared with the sequence as shown in SEQ ID NO: 31, 131 or 132, or an amino acid sequence having 1, 2, 3, 4, 5, 6, 7, 8, 9, or 10 amino acid substitutions or deletions on the sequence as shown in SEQ ID NO: 31, 131 or 132; or
(2) CD80 ECD comprising the sequence as shown in SEQ ID NO: 32 or 133, or a sequence having an identity of greater than 80%, 85%, 90%, 95%, 96%, 97%, 98%, 99% compared with the sequence as shown in SEQ ID NO: 32 or 133, or an amino acid sequence having 1, 2, 3, 4, 5, 6, 7, 8, 9, or 10 amino acid substitutions or deletions on the sequence as shown in SEQ ID NO: 32 or 133; or
(3) A combination of the second extracellular region of VEGFR1 and the third extracellular region of VEGFR2, comprising the sequence as shown in SEQ ID NO: 33, or a sequence having an identity of greater than 80%, 85%, 90%, 95%, 96%, 97%, 98%, 99% compared with the sequence as shown in SEQ ID NO: 33, or an amino acid sequence having 1, 2, 3, 4, 5, 6, 7, 8, 9, or 10 amino acid substitutions or deletions on the sequence as shown in SEQ ID NO: 33.

Further, the C-terminus or/and N-terminus of the second binding domain has a peptide linker, and the peptide linker consists of 2-30 amino acids.

Further, the peptide linker may be:
(1) (GGGGS)n; or
(2) AKTTPKLEEGEFSEAR (SEQ ID NO: 80); or
(3) AKTTPKLEEGEFSEARV (SEQ ID NO: 81); or
(4) AKTTPKLGG (SEQ ID NO: 82); or
(5) SAKTTPKLGG (SEQ ID NO: 83); or
(6) SAKTTP (SEQ ID NO: 84); or
(7) RADAAP (SEQ ID NO: 85); or
(8) RADAAPTVS (SEQ ID NO: 86); or
(9) RADAAAAGGPGS (SEQ ID NO: 87); or
(10) RADAAAA (SEQ ID NO: 88); or
(11) SAKTTPKLEEGEFSEARV (SEQ ID NO: 89); or
(12) ADAAP (SEQ ID NO: 90); or
(13) DAAPTVSIFPP (SEQ ID NO: 91); or
(14) TVAAP (SEQ ID NO: 92); or
(15) TVAAPSVFIFPP (SEQ ID NO: 93); or
(16) QPKAAP (SEQ ID NO: 94); or
(17) QPKAAPSVTLFPP (SEQ ID NO: 95); or
(18) AKTTPP (SEQ ID NO: 96); or
(19) AKTTPPSVTPLAP (SEQ ID NO: 97); or
(20) AKTTAP (SEQ ID NO: 98); or
(21) AKTTAPSVYPLAP (SEQ ID NO: 99); or
(22) ASTKGP (SEQ ID NO: 100); or
(23) ASTKGPSVFPLAP (SEQ ID NO: 101); or
(24) GENKVEYAPALMALS (SEQ ID NO: 102); or
(25) GPAKELTPLKEAKVS (SEQ ID NO: 103); or
(26) GHEAAAVMQVQYPAS (SEQ ID NO: 104); or
(27) GGGGSGGGGSGGGGSA (SEQ ID NO: 105),

wherein n is equal to 1, 2, 3 or 4;
when n=1, the (GGGGS)n is GGGGS (SEQ ID NO: 120);
when n=2, the (GGGGS)n is GGGGSGGGGS (SEQ ID NO: 121) or (GGGGS)₂;
when n=3, the (GGGGS)n is GGGGSGGGGSGGGGS (SEQ ID NO: 122) or (GGGGS)₃;
when n=4, the (GGGGS)n is GGGGSGGGGSGGGGSGGGGS (SEQ ID NO: 123) or (GGGGS)₄.

Further, the size of the inserted second binding domain does not exceed 235, 240, 250, 260, 270, 280, 290 or 300 amino acids.

Further, the insertion site of the second binding domain is located in a middle front part of the hinge region, and the insertion site does not affect the formation of disulfide bonds of the immunoglobulin, wherein the middle front part of the hinge region generally refers to the part before 231A. Further, part of the amino acids in the hinge region before and after the insertion site are substituted or deleted. For example, the hinge region contains D221G and/or C220V mutations.

Further, the IgG is selected from mammalian IgG, humanized IgG, and human IgG, and the mammal includes mouse and rat; preferably, the IgG is IgG1, IgG2, IgG3 or IgG4.

Further, the Fc region of the fusion protein is aglycosylated or deglycosylated, or has reduced fucosylation or is afucosylated.

Further, the IgG is Atezolizumab, Avelumab, Durvalumab, Nivolumab, Pembrolizumab, Cemiplimab or Ipilimumab. Preferably, the IgG is Atezolizumab, the amino acid sequence of the heavy chain of the IgG is shown in SEQ ID NO: 106, and the amino acid sequence of the light chain of the IgG is shown in SEQ ID NO: 107; or the IgG is Avelumab, the amino acid sequence of the heavy chain of the IgG is shown in SEQ ID NO: 108, and the amino acid sequence of the light chain of the IgG is shown in SEQ ID NO: 109; or the IgG is Durvalumab, the amino acid sequence of the heavy chain of the IgG is shown in SEQ ID NO: 110, and the amino acid sequence of the light chain of the IgG is shown in SEQ ID NO: 111; or the IgG is Nivolumab, the amino acid sequence of the heavy chain of the IgG is shown in SEQ ID NO: 112, and the amino acid sequence of the light chain of the IgG is shown in SEQ ID NO: 113; or the IgG is Pembrolizumab, the amino acid sequence of the heavy chain of the IgG is shown in SEQ ID NO: 114, and the amino acid sequence of the light chain of the IgG is shown in SEQ ID NO: 115; or the IgG is Ipilimumab, the amino acid sequence of the heavy chain of the IgG is shown in SEQ ID NO: 116, and the amino acid sequence of the light chain of the IgG is shown in SEQ ID NO: 117; or the IgG is Cemiplimab, the amino acid sequence of the heavy chain of the IgG is shown in SEQ ID NO: 118, and the amino acid sequence of the light chain of the IgG is shown in SEQ ID NO: 119.

Specifically, the present disclosure provides a Hibody structure fusion protein with a first binding domain targeting human PD-L1, the CDRs of the heavy chain variable region and/or the CDRs of the light chain variable region in the Fab of the IgG have the same CDR sequence as the antibody defined by the following sequence, or has 1-2 amino acid substitutions on the CDRs of the antibody defined by the following sequence, the antibody is defined as follows:
(1) The amino acid sequence of the heavy chain variable region is shown in SEQ ID NO: 66; and/or
(2) The amino acid sequence of the light chain variable region is shown in SEQ ID NO: 67.

Further, the CDRs of the heavy chain variable region and/or the CDRs of the light chain variable region in the Fab of the IgG are as follows:
(1) For the heavy chain variable region, the amino acid sequence of CDR1 is selected from SEQ ID NOs: 1-5 and amino acid sequences having 1 or 2 amino acid substitutions on SEQ ID NOs: 1-5; the amino acid sequence of CDR2 is selected from SEQ ID NOs: 6-10 and amino acid sequences having 1 or 2 amino acid substitutions on SEQ ID NOs: 6-10; the amino acid sequence of CDR3 is selected from SEQ ID NOs: 11-15 and amino acid sequences having 1 or 2 amino acid substitutions on SEQ ID NOs: 11-15; and/or
(2) For the light chain variable region, the amino acid sequence of CDR1 is selected from SEQ ID NOs: 16-20 and amino acid sequences having 1 or 2 amino acid substitutions on SEQ ID NOs: 16-20; the amino acid sequence of CDR2 is selected from SEQ ID NOs: 21-25 and amino acid sequences having 1 or 2 amino acid substitutions on SEQ ID NOs: 21-25; the amino acid sequence of CDR3 is selected from SEQ ID NOs: 26-30 and amino acid sequences having 1 or 2 amino acid substitutions on SEQ ID NOs: 26-30.

In a particular embodiment, according to different measurement methods or system identifications used, the complementarity determining regions CDRs 1-3 of the corresponding heavy chain and light chain variable regions are shown in Table 2.

**Table 2. Amino acid sequences of heavy chain and light chain variable region CDRs 1-3 of the anti-PD-L1 antibody**

| **Category** | **System** | **CDR1** | **CDR2** | **CDR3** |
|---|---|---|---|---|
| Heavy chain | Chothia | SEQ ID NO: 1 GFSLSRY | SEQ ID NO: 6 WGVGT | SEQ ID NO: 11 NWGTADYFDY |
| | AbM | SEQ ID NO: 2 GFSLSRYSVH | SEQ ID NO: 7 MIWGVGTTD | SEQ ID NO: 12 NWGTADYFDY |
| | Kabat | SEQ ID NO: 3 RYSVH | SEQ ID NO: 8 MIWGVGTTDYNSALKS | SEQ ID NO: 13 NWGTADYFDY |
| | Contact | SEQ ID NO: 4 SRYSVH | SEQ ID NO: 9 WLGMIWGVGTTD | SEQ ID NO: 14 ARNWGTADYFD |
| | IMGT | SEQ ID NO: 5 GFSLSRYS | SEQ ID NO: 10 IWGVGTT | SEQ ID NO: 15 ARNWGTADYFDY |
| Light chain | Chothia | SEQ ID NO: 16 RASKSVHTSGYSYMH | SEQ ID NO: 21 LASNLES | SEQ ID NO: 26 QHSGELPYT |
| | AbM | SEQ ID NO: 17 RASKSVHTSGYSYMH | SEQ ID NO: 22 LASNLES | SEQ ID NO: 27 QHSGELPYT |
| | Kabat | SEQ ID NO: 18 RASKSVHTSGYSYMH | SEQ ID NO: 23 LASNLES | SEQ ID NO: 28 QHSGELPYT |
| | Contact | SEQ ID NO: 19 HTSGYSYMHWY | SEQ ID NO: 24 LLIYLASNLE | SEQ ID NO: 29 QHSGELPY |
| | IMGT | SEQ ID NO: 20 KSVHTSGYSY | SEQ ID NO: 25 LAS | SEQ ID NO: 30 QHSGELPYT |

Further, the fusion protein comprises the following CDR sequences,
(1) The amino acid sequences of CDRs 1-3 of the heavy chain variable region are SEQ ID NOs: 1, 6, 11, or an amino acid sequence having 1 or 2 amino acid substitutions on SEQ ID NOs: 1, 6, 11; and/or an amino acid sequence of CDRs 1-3 of the light chain variable region are SEQ ID NOs: 16, 21, 26, or an amino acid sequence having 1 or 2 amino acid substitutions on SEQ ID NOs: 16, 21, 26; or
(2) The amino acid sequences of CDRs 1-3 of the heavy chain variable region are SEQ ID NOs: 2, 7, 12, or an amino acid sequence having 1 or 2 amino acid substitutions on SEQ ID NOs: 2, 7, 12; and/or an amino acid sequence of CDRs 1-3 of the light chain variable region are SEQ ID NOs: 17, 22, 27, or an amino acid sequence having 1 or 2 amino acid substitutions on SEQ ID NOs: 17, 22, 27; or
(3) The amino acid sequences of CDRs 1-3 of the heavy chain variable region are SEQ ID NOs: 3, 8, 13, or an amino acid sequence having 1 or 2 amino acid substitutions on SEQ ID NOs: 3, 8, 13; and/or an amino acid sequence of CDRs 1-3 of the light chain variable region are SEQ ID NOs: 18, 23, 28, or an amino acid sequence having 1 or 2 amino acid substitutions on SEQ ID NOs: 18, 23, 28; or
(4) The amino acid sequences of CDRs 1-3 of the heavy chain variable region are SEQ ID NOs: 4, 9, 14, or an amino acid sequence having 1 or 2 amino acid substitutions on SEQ ID NOs: 4, 9, 14; and/or an amino acid sequence of CDRs 1-3 of the light chain variable region are SEQ ID NOs: 19, 24, 29, or an amino acid sequence having 1 or 2 amino acid substitutions on SEQ ID NOs: 19, 24, 29; or
(5) The amino acid sequences of CDRs 1-3 of the heavy chain variable region are SEQ ID NOs: 5, 10, 15, or an amino acid sequence having 1 or 2 amino acid substitutions on SEQ ID NOs: 5, 10, 15; and/or an amino acid sequence of CDRs 1-3 of the light chain variable region are SEQ ID NOs: 20, 25, 30, or an amino acid sequence having 1 or 2 amino acid substitutions on SEQ ID NOs: 20, 25, 30.

Further, in the heavy chain of the fusion protein, the amino acid sequences of CDRs 1-3 of the heavy chain variable region are SEQ ID NOs: 3, 8, 13; and/or an amino acid sequence of CDRs 1-3 of the light chain variable region are SEQ ID NOs: 18, 23, 28.

Further, the sequences of the heavy chain variable region and the light chain variable region of the fusion protein are as follows,
(1) The heavy chain variable region has a sequence as shown in SEQ ID NO: 66, or a sequence that has the same CDRs 1-3 as SEQ ID NO: 66 and has an identity of greater than 80%, 85%, 90%, 95%, 96%, 97%, 98%, 99% compared with SEQ ID NO: 66; and/or
(2) The light chain variable region has a sequence as shown in SEQ ID NO: 67, or a sequence that has the same CDRs 1-3 as SEQ ID NO: 67 and has an identity of greater than 80%, 85%, 90%, 95%, 96%, 97%, 98%, 99% compared with SEQ ID NO: 67.

More specifically, the amino acid sequences of the heavy chain and light chain of the fusion protein are as follows,
(1) The heavy chain of the fusion protein has an amino acid sequence as shown in SEQ ID NO: 72, or a sequence having an identity of greater than 80%, 85%, 90%, 95%, 96%, 97%, 98%, 99% compared with SEQ ID NO: 72;
(2) The light chain of the fusion protein has an amino acid sequence as shown in SEQ ID NO: 73, or a sequence having an identity of greater than 80%, 85%, 90%, 95%, 96%, 97%, 98%, 99% compared with SEQ ID NO: 73.

Further, the amino acid sequences of the heavy chain and light chain of the fusion protein are as follows,
(1) The amino acid sequence of the heavy chain of the fusion protein has 1-15 amino acid site mutations or has an alternative peptide linker compared with SEQ ID NO: 72;
(2) The amino acid sequence of the light chain of the fusion protein has 1-10 amino acid site mutations compared with SEQ ID NO: 73.

Specifically, the present disclosure provides a Hibody structure fusion protein with a first binding domain targeting human PD-1, wherein the CDRs of the heavy chain variable region and/or the CDRs of the light chain variable region in the Fab have the same CDR sequence as the antibody defined by the following sequence, or has 1-2 amino acid substitutions on the CDRs of the antibody defined by the following sequence, the antibody is defined as follows:
(1) The amino acid sequence of the heavy chain variable region is shown in SEQ ID NO: 64; and/or
(2) The amino acid sequence of the light chain variable region is shown in SEQ ID NO: 65.

Further, the CDRs of the heavy chain variable region and/or the CDRs of the light chain variable region in the Fab in the fusion protein are as follows:
(1) For the heavy chain variable region, the amino acid sequence of CDR1 is selected from SEQ ID NOs: 34-38 and amino acid sequences having 1 or 2 amino acid substitutions on SEQ ID NOs: 34-38; the amino acid sequence of CDR2 is selected from SEQ ID NOs: 39-43 and amino acid sequences having 1 or 2 amino acid substitutions on SEQ ID NOs: 39-43; the amino acid sequence of CDR3 is selected from SEQ ID NOs: 44-48 and amino acid sequences having 1 or 2 amino acid substitutions on SEQ ID NOs: 44-48; and/or
(2) For the light chain variable region, the amino acid sequence of CDR1 is selected from SEQ ID NOs: 49-53 and amino acid sequences having 1 or 2 amino acid substitutions on SEQ ID NOs: 49-53; the amino acid sequence of CDR2 is selected from SEQ ID NOs: 54-58 and amino acid sequences having 1 or 2 amino acid substitutions on SEQ ID NOs: 54-58; the amino acid sequence of CDR3 is selected from SEQ ID NOs: 59-63 and amino acid sequences having 1 or 2 amino acid substitutions on SEQ ID NOs: 59-63

In a particular embodiment, according to different measurement methods or system identifications used, the complementarity determining regions CDRs 1-3 of the corresponding heavy chain and light chain variable regions are shown in Table 3.

**Table 3. Amino acid sequences of heavy chain and light chain variable region CDRs 1-3 of the anti-PD-1 antibody**

| **Category** | **System** | **CDR1** | **CDR2** | **CDR3** |
|---|---|---|---|---|
| Heavy chain | Chothia | SEQ ID NO: 34 GITFSNS | SEQ ID NO: 39 WYDGSK | SEQ ID NO: 44 NDDY |
| | AbM | SEQ ID NO: 35 GITFSNSGMH | SEQ ID NO: 40 VIWYDGSKRY | SEQ ID NO: 45 NDDY |
| | Kabat | SEQ ID NO: 36 NSGMH | SEQ ID NO: 41 VIWYDGSKRYYADSVKG | SEQ ID NO: 46 NDDY |
| | Contact | SEQ ID NO: 37 SNSGMH | SEQ ID NO: 42 WVAVIWYDGSKRY | SEQ ID NO: 47 ATNDD |
| | IMGT | SEQ ID NO: 38 GITFSNSG | SEQ ID NO: 43 IWYDGSKR | SEQ ID NO: 48 ATNDDY |
| Light chain | Chothia | SEQ ID NO: 49 RASQSVSSYLA | SEQ ID NO: 54 DASNRAT | SEQ ID NO: 59 QQSSNWPRT |
| | AbM | SEQ ID NO: 50 RASQSVSSYLA | SEQ ID NO: 55 DASNRAT | SEQ ID NO: 60 QQSSNWPRT |
| | Kabat | SEQ ID NO: 51 RASQSVSSYLA | SEQ ID NO: 56 DASNRAT | SEQ ID NO: 61 QQSSNWPRT |
| | Contact | SEQ ID NO: 52 SSYLAWY | SEQ ID NO: 57 LLIYDASNRA | SEQ ID NO: 62 QQSSNWPR |
| | IMGT | SEQ ID NO: 53 QSVSSY | SEQ ID NO: 58 DAS | SEQ ID NO: 63 QQSSNWPRT |

Further, the CDRs 1-3 of the variable region of the fusion protein is defined as follows,
(1) The amino acid sequences of CDRs 1-3 of the heavy chain variable region are SEQ ID NOs: 34, 39, 44, or an amino acid sequence having 1 or 2 amino acid substitutions on SEQ ID NOs: 34, 39, 44; and/or an amino acid sequence of CDRs 1-3 of the light chain variable region are SEQ ID NOs: 49, 54, 59, or an amino acid sequence having 1 or 2 amino acid substitutions on SEQ ID NOs: 49, 54, 59; or
(2) The amino acid sequences of CDRs 1-3 of the heavy chain variable region are SEQ ID NOs: 35, 40, 45, or an amino acid sequence having 1 or 2 amino acid substitutions on SEQ ID NOs: 35, 40, 45; and/or an amino acid sequence of CDRs 1-3 of the light chain variable region are SEQ ID NOs: 50, 55, 60, or an amino acid sequence having 1 or 2 amino acid substitutions on SEQ ID NOs: 50, 55, 60; or
(3) The amino acid sequences of CDRs 1-3 of the heavy chain variable region are SEQ ID NOs: 36, 41, 46, or an amino acid sequence having 1 or 2 amino acid substitutions on SEQ ID NOs: 36, 41, 46; and/or an amino acid sequence of CDRs 1-3 of the light chain variable region are SEQ ID NOs: 51, 56, 61, or an amino acid sequence having 1 or 2 amino acid substitutions on SEQ ID NOs: 51, 56, 61; or
(4) The amino acid sequences of CDRs 1-3 of the heavy chain variable region are SEQ ID NOs: 37, 42, 47, or an amino acid sequence having 1 or 2 amino acid substitutions on SEQ ID NOs: 37, 42, 47; and/or an amino acid sequence of CDRs 1-3 of the light chain variable region are SEQ ID NOs: 52, 57, 62, or an amino acid sequence having 1 or 2 amino acid substitutions on SEQ ID NOs: 52, 57, 62; or
(5) The amino acid sequences of CDRs 1-3 of the heavy chain variable region are SEQ ID NOs: 38, 43, 48, or an amino acid sequence having 1 or 2 amino acid substitutions on SEQ ID NOs: 38, 43, 48; and/or an amino acid sequence of CDRs 1-3 of the light chain variable region are SEQ ID NOs: 53, 58, 63, or an amino acid sequence having 1 or 2 amino acid substitutions on SEQ ID NOs: 53, 58, 63.

Further, the CDRs 1-3 of the variable region of the fusion protein is defined as follows: the amino acid sequences of CDRs 1-3 of the heavy chain variable region are SEQ ID NOs: 36, 41, 46; and/or an amino acid sequence of CDRs 1-3 of the light chain variable region are SEQ ID NOs: 51, 56, 61.

Further, the amino acid sequences of the heavy chain variable region and the light chain variable region of the fusion protein are as follows,
(1) The heavy chain variable region has a sequence as shown in SEQ ID NO: 64, or a sequence that has the same CDRs 1-3 as SEQ ID NO: 64 and has an identity of greater than 80%, 85%, 90%, 95%, 96%, 97%, 98%, 99% compared with SEQ ID NO: 64; and/or
(2) The light chain variable region has a sequence as shown in SEQ ID NO: 65, or a sequence that has the same CDRs 1-3 as SEQ ID NO: 65 and has an identity of greater than 80%, 85%, 90%, 95%, 96%, 97%, 98%, 99% compared with SEQ ID NO: 65.

More specifically, the amino acid sequences of the heavy chain and light chain of the fusion protein are defined as follows:
(1) The heavy chain of the fusion protein has an amino acid sequence as shown in SEQ ID NO: 68, or a sequence having an identity of greater than 80%, 85%, 90%, 95%, 96%, 97%, 98%, 99% compared with SEQ ID NO: 68;
(2) The light chain of the fusion protein has an amino acid sequence as shown in SEQ ID NO: 69, or a sequence having an identity of greater than 80%, 85%, 90%, 95%, 96%, 97%, 98%, 99% compared with SEQ ID NO: 69.

Further, the amino acid sequences of the heavy chain and light chain of the fusion protein are defined as follows:
(1) The amino acid sequence of the heavy chain of the fusion protein has 1-15 amino acid site mutations or has an alternative peptide linker compared with SEQ ID NO: 68;
(2) The amino acid sequence of the light chain of the fusion protein has 1-10 amino acid site mutations compared with SEQ ID NO: 69.

More specifically, the amino acid sequences of the heavy chain and light chain of the fusion protein are defined as follows:
(1) The heavy chain of the fusion protein has an amino acid sequence as shown in SEQ ID NO: 70, or a sequence having an identity of greater than 80%, 85%, 90%, 95%, 96%, 97%, 98%, 99% compared with SEQ ID NO: 70;
(2) The light chain of the fusion protein has an amino acid sequence as shown in SEQ ID NO: 71, or a sequence having an identity of greater than 80%, 85%, 90%, 95%, 96%, 97%, 98%, 99% compared with SEQ ID NO: 71.

Further, the amino acid sequences of the heavy chain and light chain of the fusion protein are defined as follows:
(1) The amino acid sequence of the heavy chain of the fusion protein has 1-15 amino acid site mutations or has an alternative peptide linker compared with SEQ ID NO: 70;
(2) The amino acid sequence of the light chain of the fusion protein has 1-10 amino acid site mutations compared with SEQ ID NO: 71.

The present disclosure also provides an isolated polynucleotide encoding the above fusion protein.

The present disclosure also provides a nucleic acid construct comprising the above polynucleotide, preferably the nucleic acid construct is a vector.

The present disclosure also provides a host cell comprising the above polynucleotide or the above nucleic acid construct or the vector, preferably the cell is a prokaryotic cell, eukaryotic cell, yeast cell, mammal cell, E. coli cell or CHO cell, NS0 cell, Sp2/0 cell, or BHK cell.

The present disclosure also provides a pharmaceutical composition comprising the above fusion protein and a pharmaceutically acceptable carrier.

The present disclosure also provides a method for treating tumor or cancer, comprising the step of administering the above fusion protein or the pharmaceutical composition to a subject in need of a treatment or relief.

The present disclosure also provides use of the above fusion protein, the polynucleotide, the nucleic acid construct or the vector, or the pharmaceutical composition in the manufacture of a medicament for the treatment or prevention of a tumor or cancer. The tumor or cancer includes a solid tumor or a non-solid tumor.

The present disclosure also provides a diagnostic kit comprising the above fusion protein.

The present disclosure also provides a method of manufacturing the fusion protein, comprising culturing the host cell under conditions allowing the expression of the above nucleic acid construct, and recovering the produced fusion protein from the culture.

The Hibody structure fusion protein provided in the present disclosure has the same advantages of expression and production as IgG because the second binding domain is inserted in the hinge region, and can be seamlessly applicable to the existing IgG expression platform and purification platform, which greatly reduces the subsequent development cost; compared with the bispecific antibody constructed by the existing bispecific antibody platform, the bispecific antibody constructed by the Hibody platform has a higher expression level and does not have the problem of light and heavy chain mismatches, which also greatly reduces the cost of antibody production. In addition, it is surprising to find that inserting a second binding domain of a certain size in the two hinge regions not only does not affect the binding activity of the Fab region of the fusion protein and the stability of the protein, but also further improves the stability and obtains a longer half-life. More notably, the binding activity of the second binding domain to the target binding site is significantly improved compared to that of the corresponding soluble natural binding fragment monomer to the corresponding target, which may lie in that since the receptor or ligand of the selected second binding domain will form a dimerization or multimerization structure in the natural signaling pathway to activate or inhibit the signaling pathway, when the second binding domain is inserted into the hinge region, its spatial positional relationship in the hinge region promotes the formation of the dimerization of the corresponding receptor or ligand or its fragment, so that by imitating the natural signal pathway, a more significantly improved binding activity is generated, which makes the fusion protein have a greatly increased activation or inhibition ability compared with a soluble receptor or ligand or its fragment. Besides, through a reasonable combination of the two binding domains, the target of receptor inhibition or receptor activation can be flexibly selected to achieve an effective synergistic effect. The bispecific fusion protein provided in the present disclosure has a more significant disease treatment effect to effectively treat or control the disease process, especially in various tumors and cancers, compared with the separate administration of two target drugs or the bispecific antibody of the prior art.

### BRIEF DESCRIPTION OF DRAWINGS

FIG. 1 Schematic diagram of Hibody structure fusion protein.
FIG. 2 shows the inhibition curve of Hib-PDV and its control group on mouse colon cancer model tumor.
FIG. 3 shows the inhibition curve of Hib-PDC and its control group on mouse colon cancer tumor.
FIG. 4 is a comparison chart of Hib-PLT and its control group on promoting cell secretion of IFN-γ factor at the level of 10-100 nM.

### DETAILED DESCRIPTION

### Definitions

Unless otherwise defined, all technical and scientific terms used herein have the same meaning as commonly understood by those of ordinary skill in the art related to the present disclosure. Those skilled in the art can refer to the Dictionary of Cell and Molecular Biology, third edition, 1999, Academic Press; the Concise Dictionary of Biomedicine and Molecular Biology, Juo, Pei-Show, second edition, 2002, CRC Press; and the Oxford Dictionary of Biochemistry and Molecular Biology, revised edition, 2000 , Oxford University Press.

The amino acids involved in the present disclosure can be represented by their commonly known three-letter symbols or by the single-letter symbols recommended by the IUPAC-IUB Biochemical Nomenclature Commission. Likewise, nucleotides can be represented by their generally recognized one-letter codes.

In the present disclosure, the determination or numbering method of the complementarity determining region (CDR) of the antibody variable domain includes IMGT, Kabat (such as stated in Kabat et al., Sequences of Proteins of Immunological Interest, 5th edition, Public Health Service, National Institutes of Health, Bethesda MD. (1991)), Chothia, AbM and Contact method.

For the purpose of the present invention, "identity", "consistency" or "similarity" between two nucleic acid or amino acid sequences refers to the percentage of the same nucleotides or amino acid residues between the two sequences to be compared obtained after the optimal alignment. The percentage is purely statistical and the differences between the two sequences are randomly distributed and cover their full length. Comparisons between two nucleic acid or amino acid sequences are usually performed by comparing these sequences after aligning them in an optimal manner, and can be performed over a segment or over a "comparison window". In addition to manual implementation, the optimal alignment for comparing sequences can also performed through the local homology algorithm of Smith and Waterman (1981) [Ad. App. Math. 2: 482], through the local homology algorithm of Neddleman and Wunsch (1970) [J. Mol. Biol. 48: 443], through the similarity search method of Pearson and Lipman (1988) [Proc. Natl. Acad. Sci. USA 85: 2444), and through the computer software using these algorithms (GAP, BESTFIT, FASTA and TFASTA in the Wisconsin Genetics Software Package, Genetics Computer Group, 575 Science Dr., Madison, WI, or through BLAST N or BLAST P comparison software).

As broadly defined, the immunoglobulin involved in the present disclosure refers to an animal protein with antibody activity, consisting of two identical light chains and two identical heavy chains. It is an important class of immune effector molecules and a protein produced by lymphocytes of higher animal immune system, and can be transformed into antibodies by induction of antigens. Due to different structures, it can be divided into 5 types: IgG, IgA, IgM, IgD and IgE. Preferably, the immunoglobulin involved in the present disclosure is IgG.

As broadly defined, the receptor involved in the present disclosure is a type of special protein that exists in the cell membrane or in the cell and can bind to a specific signal molecule outside the cell to activate a series of biochemical reactions in the cell to cause the cell to produce a corresponding effect under external stimuli. The biologically active substances that bind to receptors are collectively referred to as ligands. The binding of the receptor and the ligand results in a molecular conformation change, which causes cellular responses, such as mediating intercellular signal transduction, intercellular adhesion, endocytosis, and the like.

The bispecific fusion protein involved in the present disclosure is based on the structure of an immunoglobulin with a second binding domain inserted in its hinge region. Therefore, the bispecific fusion protein involved in the present disclosure has two binding domains.

### Examples

The present disclosure will be further illustrated by way of the following examples. It should be noted that the following examples are for further elaboration and explanation of the present disclosure, and should not be regarded as limiting the present disclosure.

### Example 1 Construction of proteins

In this example, the following protein molecules were constructed using conventional methods in the art, and expressed transiently or stably according to conventional methods in the art:
(1) Three Hibody bispecific fusion proteins: Hib-PLT, Hib-PDC, and Hib-PDV;
(2) Control proteins 1-3: constructed according to the bifunctional molecular structure in WO2015/118175;
(3) Other control proteins: TGF-β-R-His, VEGF-R-His, CD80-His, PD-L1 control antibodies.

**Table 4. Target combinations of fusion protein**

| **Fusion protein code** | **First binding domain target** | **Second binding domain target** | **Second binding domain fragment inserted** | **Peptide linker** |
|---|---|---|---|---|
| Hib-PLT | PD-L1 | TGF-β | TGF-βRII extracellular region fragment | (GGGGS)₃ |
| Hib-PDC | PD-1 | CD28 | CD80 | (GGGGS)₃ |
| Hib-PDV | PD-1 | VEGF | The combination of the second extracellular region of VEGFR1 and the third extracellular region of VEGFR2 | (GGGGS)₃ |
| Control protein 1 | PD-L1 | TGF-β | TGF-βRII extracellular region fragment | (GGGGS)₃ |
| Control protein 2 | PD-1 | CD28 | CD80 | (GGGGS)₃ |
| Control protein 3 | PD-1 | VEGF | The combination of the second extracellular region of VEGFR1 and the third extracellular region of VEGFR2 | (GGGGS)₃ |

- The nucleic acid sequences and amino acid sequences of the heavy chain and light chain of the fusion protein Hib-PLT are as follows:
   Nucleic acid sequence of fusion protein Hib-PLT heavy chain (SEQ ID NO: 78): 
CAGGTGCAGCTGCAGGAGTCCGGACCAGGACTGGTGAAGCCATCCGAGACCCTGAGCCTGAC
CTGTACAGTGTCCGGCTTCAGCCTGTCTAGGTACAGCGTGCACTGGATCAGACAGCCACCTGG
CAAGGGACTGGAGTGGCTGGGCATGATCTGGGGCGTGGGCACCACAGACTACAACTCTGCTC
TGAAGTCCAGACTGACCATCAGCAAGGATACATCTAAGAATCAGTTCAGCCTGAAGCTGTCCA
GCGTGACCGCCGCTGACACAGCCGTGTACTATTGCGCTCGCAACTGGGGCACCGCCGACTACT
TCGACTATTGGGGCCAGGGCACCACAGTGACAGTGTCTTCCGCTAGCACCAAGGGCCCATCG
GTCTTCCCCCTGGCACCCTCCTCCAAGAGCACCTCTGGGGGCACAGCGGCCCTGGGCTGCCT
GGTCAAGGACTACTTCCCCGAACCGGTGACGGTGTCGTGGAACTCAGGCGCCCTGACCAGCG
GCGTGCACACCTTCCCGGCTGTCCTACAGTCCTCAGGACTCTACTCCCTCAGCAGCGTGGTGA
CCGTGCCCTCCAGCAGCTTGGGCACCCAGACCTACATCTGCAACGTGAATCACAAGCCCAGC
AACACCAAGGTGGACAAGAAAGTTGAGCCCAAATCTTGTGACAAAGGCGGGGGAGGCAGCG
GCGGCGGAGGAAGCGGGGGCGGAGGTAGCACCATCCCTCCACACGTGCAGAAGAGCGTG
AACAATGACATGATCGTCACCGACAACAACGGCGCCGTCAAGTTCCCCCAGCTGTGCAA
ATTCTGCGACGTGAGGTTCTCCACGTGCGACAACCAGAAGAGCTGTATGAGCAACTGCA
GCATCACATCCATCTGCGAAAAACCCCAGGAAGTGTGCGTCGCCGTCTGGCGGAAGAA
CGACGAGAACATCACACTGGAGACCGTGTGCCACGACCCCAAACTGCCCTACCACGACT
TCATCCTGGAGGACGCCGCCAGCCCAAAGTGCATCATGAAAGAGAAGAAGAAGCCGGG
CGAGACTTTCTTCATGTGCTCCTGCAGCTCCGACGAGTGCAACGATAATATCATCTTCAG
CGAAGAATACAACACATCTAACCCAGACGGAGGGGGCGGATCCGGGGGCGGCGGAAGCGG
CGGGGGGGCAGCACTCACACATGCCCACCGTGCCCAGCACCTGAACTCCTGGGGGGACCGT
CAGTCTTCCTCTTCCCCCCAAAACCCAAGGACACCCTCATGATCTCCCGGACCCCTGAGGTCA
CATGCGTGGTGGTGGACGTGAGCCACGAAGACCCTGAGGTCAAGTTCAACTGGTACGTGGAC
GGCGTGGAGGTGCATAATGCCAAGACAAAGCCGCGGGAGGAGCAGTACAACAGCACGTACC
 
GTGTGGTCAGCGTCCTCACCGTCCTGCACCAGGACTGGCTGAATGGCAAGGAGTACAAGTGC
AAGGTCTCCAACAAAGCCCTCCCAGCCCCCATCGAGAAAACCATCTCCAAAGCCAAAGGGCA
GCCCCGAGAACCACAGGTGTACACCCTGCCCCCATCCCGGGAGGAGATGACCAAGAACCAGG
TCAGCCTGACCTGCCTGGTCAAAGGCTTCTATCCCAGCGACATCGCCGTGGAGTGGGAGAGC
AATGGGCAGCCGGAGAACAACTACAAGACCACGCCTCCCGTGCTGGACTCCGACGGCTCCTT
CTTCCTCTACAGCAAGCTCACCGTGGACAAGAGCAGGTGGCAGCAGGGGAACGTCTTCTCAT
GCTCCGTGATGCATGAGGCTCTGCACAACCACTACACGCAGAAGAGCCTCTCCCTGTCTCCGG
GTAAATGA
   Note: The underlined and bold sequence is the TGF-βRII nucleic acid sequence.
   Amino acid sequence of fusion protein Hib-PLT heavy chain (SEQ ID NO: 72): 
QVQLQESGPG LVKPSETLSL TCTVSGFSLS RYSVHWIRQP PGKGLEWLGM IWGVGTTDYN 60

SALKSRLTIS KDTSKNQFSL KLSSVTAADT AVYYCARNWG TADYFDYWGQ GTTVTVSSAS 120

TKGPSVFPLA PSSKSTSGGT AALGCLVKDY FPEPVTVSWN SGALTSGVHT FPAVLQSSGL 180

YSLSSVVTVP SSSLGTQTYI CNVNHKPSNT KVDKKVEPKS CDKGGGGSGG GGSGGGGSTI 240

PPHVQKSVNN DMIVTDNNGA VKFPQLCKFC DVRFSTCDNQ KSCMSNCSIT SICEKPQEVC 300

VAVWRKNDEN ITLETVCHDP KLPYHDFILE DAASPKCIMK EKKKPGETFF MCSCSSDECN 360

DNIIFSEEYN TSNPDGGGGS GGGGSGGGGS THTCPPCPAP ELLGGPSVFL FPPKPKDTLM 420

ISRTPEVTCV VVDVSHEDPE VKFNWYVDGV EVHNAKTKPR EEQYNSTYRV VSVLTVLHQD 480

WLNGKEYKCK VSNKALPAPI EKTISKAKGQ PREPQVYTLP PSREEMTKNQ VSLTCLVKGF 540

YPSDIAVEWE SNGQPENNYK TTPPVLDSDG SFFLYSKLTV DKSRWQQGNV FSCSVMHEAL 600

HNHYTQKSLS LSPGK 615
   Note: The bold and framed fragment is the hinge region sequence, the peptide linker is in italics, and the underlined and bold sequence is the TGF-βRII amino acid sequence.
   Nucleic acid sequence of fusion protein Hib-PLT light chain (SEQ ID NO: 79): 
GATATCGTGCTGACCCAGTCTCCAGCTTCCCTGGCCGTGTCCCCAGGACAGAGGGCCACCATC
ACATGTCGGGCTTCCAAGAGCGTGCACACAAGCGGCTACTCTTATATGCATTGGTACCAGCAG
AAGCCCGGCCAGCCCCCTAAGCTGCTGATCTATCTGGCTTCCAACCTGGAGAGCGGAGTGCCA
GCTAGGTTCTCTGGCTCCGGCAGCGGCACCGACTTTACCCTGACAATCAATCCTGTGGAGGCC
 
AACGATACAGCTAATTACTATTGCCAGCACTCCGGAGAGCTGCCATACACCTTCGGCGGAGGC
ACAAAGGTGGAGATCAAGCGTACGGTGGCTGCACCATCTGTCTTCATCTTCCCGCCATCTGAT
GAGCAGTTGAAATCTGGAACTGCCTCTGTTGTGTGCCTGCTGAATAACTTCTATCCCAGAGAG
GCCAAAGTACAGTGGAAGGTGGATAACGCCCTCCAATCGGGTAACTCCCAGGAGAGTGTCAC
AGAGCAGGACAGCAAGGACAGCACCTACAGCCTCAGCAGCACCCTGACGCTGAGCAAAGCA
GACTACGAGAAACACAAAGTCTACGCCTGCGAAGTCACCCATCAGGGCCTGAGCTCGCCCGT
CACAAAGAGCTTCAACAGGGGAGAGTGTTAG
   Amino acid sequence of fusion protein Hib-PLT light chain (SEQ ID NO: 73): 
DIVLTQSPAS LAVSPGQRAT ITCRASKSVH TSGYSYMHWY QQKPGQPPKL LIYLASNLES 60

GVPARFSGSG SGTDFTLTIN PVEANDTANY YCQHSGELPY TFGGGTKVEI KRTVAAPSVF 120

IFPPSDEQLK SGTASVVCLL NNFYPREAKV QWKVDNALQS GNSQESVTEQ DSKDSTYSLS 180

STLTLSKADY EKHKVYACEV THQGLSSPVT KSFNRGEC 218
- The nucleic acid sequences and amino acid sequences of the heavy chain and light chain of the fusion protein Hib-PDC are as follows:
   Nucleic acid sequence of fusion protein Hib-PDC heavy chain (SEQ ID NO: 74): 
CAGGTGCAGCTCGTGGAGAGCGGGGGAGGCGTCGTCCAGCCTGGCCGTAGCCTGCGGCTGGA
CTGTAAGGCTAGCGGAATCACGTTCAGCAACAGCGGAATGCACTGGGTCAGACAGGCTCCTG
GCAAAGGCCTCGAATGGGTCGCCGTGATCTGGTACGACGGAGCAAGATACTACGCAGAT
TCAGTGAAGGGCCGTTTCACAATCAGCCGTGACAACTCGAAGAACACACTGTTCCTGCAGAT
GAACAGCCTGAGGGCAGAAGACACAGCAGTCTACTACTGCGCTACAAATGACGACTACTGGG
GCCAGGGAACACTGGTCACCGTGAGCTCAGCTTCCACCAAGGGCCCATCCGTCTTCCCCCTG
GCGCCCTGCTCCAGGAGCACCTCCGAGAGCACAGCCGCCCTGGGCTGCCTGGTCAAGGACTA
CTTCCCCGAACCGGTGACGGTGTCGTGGAACTCAGGCGCCCTGACCAGCGGCGTGCACACCT
TCCCGGCTGTCCTACAGTCCTCAGGACTCTACTCCCTCAGCAGCGTGGTGACCGTGCCCTCCA
GCAGCTTGGGCACGAAGACCTACACCTGCAACGTAGATCACAAGCCCAGCAACACCAAGGTG
GACAAGAGAGTTGAGTCCGGTGGCGGCGGAAGCGGCGGCGGAGGCAGCGGCGGAGGCGGC
AGCGTGATCCATGTCACGAAGGAGGTCAAAGAAGTGGCCACCCTCAGCTGCGGTCACA
ACGTCAGCGTGGAAGAGTTGGCCCAGACCAGAATCTACTGGCAGAAGGAGAAGAAGAT
GGTCTTGACCATGATGAGCGGCGACATGAACATCTGGCCAGAGTACAAGAATAGAACTA
 
TCTTCGACATCACCAATAACCTGAGCATCGTGATCCTCGCTTTGCGTCCGAGCGACGAA
GGCACCTACGAGTGCGTAGTGCTAAAGTACGAGAAAGACGCCTTCAAGCGGGAGCACC
TGGCAGAAGTAACCCTGAGCGTGAAGGCAGACTTCCCAACGCCTAGCATCTCCGACTTC
GAGATCCCCACAAGCAACATCCGGCGGATCATCTGTAGCACCTCCGGGGGTTTCCCCGA
GCCTCACCTGTCCTGGCTCGAGAACGGCGAGGAGCTGAACGCCATCAACACCACCGTG
AGCCAGGACCCCGAGACAGAACTGTACGCCGTGAGCTCCAAGCTCGACTTCAACATGA
CCACAAATCACAGCTTCATGTGCCTCATCAAGTACGGACACCTGCGGGTGAATCAGACG
TTCAACTGGAACGGCGGGGGGGTAGCGGCGGCGGGGGCTCAGGTGGGGGGGCTCAAAAT
ATGGTCCCCCATGCCCACCATGCCCAGCACCTGAGTTCCTGGGGGGACCATCAGTCTTCCTGT
TCCCCCCAAAACCCAAGGACACTCTCATGATCTCCCGGACCCCTGAGGTCACGTGCGTGGTGG
TGGACGTGAGCCAGGAAGACCCCGAGGTCCAGTTCAACTGGTACGTGGATGGCGTGGAGGTG
CATAATGCCAAGACAAAGCCGCGGGAGGAGCAGTTCAACAGCACGTACCGTGTGGTCAGCGT
CCTCACCGTCCTGCACCAGGACTGGCTGAACGGCAAGGAGTACAAGTGCAAGGTCTCCAACA
AAGGCCTCCCGTCCTCCATCGAGAAAACCATCTCCAAAGCCAAAGGGCAGCCCCGAGAGCCA
CAGGTGTACACCCTGCCCCCATCCCAGGAGGAGATGACCAAGAACCAGGTCAGCCTGACCTG
CCTGGTCAAAGGCTTCTACCCCAGCGACATCGCCGTGGAGTGGGAGAGCAATGGGCAGCCGG
AGAACAACTACAAGACCACGCCTCCCGTGCTGGACTCCGACGGCTCCTTCTTCCTCTACAGCA
GGCTAACCGTGGACAAGAGCAGGTGGCAGGAGGGGAATGTCTTCTCATGCTCCGTGATGCAT
GAGGCTCTGCACAACCACTACACACAGAAGAGCCTCTCCCTGTCTCTGGGTAAATAG
   Note: The underlined and bold sequence is the CD80 nucleic acid sequence.
   Amino acid sequence of fusion protein Hib-PDC heavy chain (SEQ ID NO: 68): 
QVQLVESGGG VVQPGRSLRL DCKASGITFS NSGMHWVRQA PGKGLEWVAV IWYDGSKRYY 60

ADSVKGRFTI SRDNSKNTLF LQMNSLRAED TAVYYCATND DYWGQGTLVT VSSASTKGPS 120

VFPLAPCSRS TSESTAALGC LVKDYFPEPV TVSWNSGALT SGVHTFPAVL QSSGLYSLSS 180

VVTVPSSSLG TKTYTCNVDH KPSNTKVDKR VESGGGGSGG GGSGGGGSVI HVTKEVKEVA 240

TLSCGHNVSV EELAQTRIYW QKEKKMVLTM MSGDMNIWPE YKNRTIFDIT NNLSIVILAL 300

RPSDEGTYEC VVLKYEKDAF KREHLAEVTL SVKADFPTPS ISDFEIPTSN IRRIICSTSG 360

GFPEPHLSWL ENGEELNAIN TTVSQDPETE LYAVSSKLDF NMTTNHSFMC LIKYGHLRVN 420

QTFNWNGGGG SGGGGSGGGG SKYGPPCPPC PAPEFLGGPS VFLFPPKPKD TLMISRTPEV 480

TCVVVDVSQE DPEVQFNWYV DGVEVHNAKT KPREEQFNST YRVVSVLTVL HQDWLNGKEY 540
 
KCKVSNKGLP SSIEKTISKA KGQPREPQVY TLPPSQEEMT KNQVSLTCLV KGFYPSDIAV 600

EWESNGQPEN NYKTTPPVLD SDGSFFLYSR LTVDKSRWQE GNVFSCSVMH EALHNHYTQK 660

SLSLSLGK 668
   Note: The peptide linker is in italics, and the underlined and bold sequence is the CD80 amino acid sequence.
   Nucleic acid sequence of fusion protein Hib-PDC light chain (SEQ ID NO: 75): 
GAGATCGTGCTGACACAGAGCCCAGCCACTCTGTCACTGTCCCCAGGAGAAAGGGCTACTCT
GTCTTGCCGGGCAAGCCAGTCTGTCTCCAGCTACCTGGCCTGGTATCAGCAGAAGCCCGGACA
GGCTCCTAGACTGCTGATCTACGACGCAAGTAACAGAGCCACCGGCATCCCCGCACGCTTCAG
TGGCTCAGGCTCCGGAACAGACTTTACTCTGACCATCTCTAGTCTGGAGCCTGAAGATTTCGC
CGTGTACTATTGTCAGCAGAGCTCTAATTGGCCTAGAACCTTCGGCCAGGGCACCAAAGTCGA
GATCAAGCGTACGGTGGCTGCACCATCTGTCTTCATCTTCCCGCCATCTGATGAGCAGTTGAAA
TCTGGAACTGCCTCTGTTGTGTGCCTGCTGAATAACTTCTATCCCAGAGAGGCCAAAGTACAG
TGGAAGGTGGATAACGCCCTCCAATCGGGTAACTCCCAGGAGAGTGTCACAGAGCAGGACAG
CAAGGACAGCACCTACAGCCTCAGCAGCACCCTGACGCTGAGCAAAGCAGACTACGAGAAA
CACAAAGTCTACGCCTGCGAAGTCACCCATCAGGGCCTGAGCTCGCCCGTCACAAAGAGCTT
CAACAGGGGAGAGTGTTAG
   Amino acid sequence of fusion protein Hib-PDC light chain (SEQ ID NO: 69): 
EIVLTQSPAT LSLSPGERAT LSCRASQSVS SYLAWYQQKP GQAPRLLIYD ASNRATGIPA 60

RFSGSGSGTD FTLTISSLEP EDFAVYYCQQ SSNWPRTFGQ GTKVEIKRTV AAPSVFIFPP 120

SDEQLKSGTA SVVCLLNNFY PREAKVQWKV DNALQSGNSQ ESVTEQDSKD STYSLSSTLT 180

LSKADYEKHK VYACEVTHQG LSSPVTKSFN RGEC 214
- The nucleic acid sequences and amino acid sequences of the heavy chain and light chain of the fusion protein Hib-PDV are as follows:
   Nucleic acid sequence of fusion protein Hib-PDV heavy chain (SEQ ID NO: 76): 
CAGGTGCAGCTCGTGGAGAGCGGGGGAGGCGTCGTCCAGCCTGGCCGTAGCCTGCGGCTGGA
CTGTAAGGCTAGCGGAATCACGTTCAGCAACAGCGGAATGCACTGGGTCAGACAGGCTCCTG
 
GCAAAGGCCTCGAATGGGTCGCCGTGATCTGGTACGACGGAGCAAGATACTACGCAGAT
TCAGTGAAGGGCCGTTTCACAATCAGCCGTGACAACTCGAAGAACACACTGTTCCTGCAGAT
GAACAGCCTGAGGGCAGAAGACACAGCAGTCTACTACTGCGCTACAAATGACGACTACTGGG
GCCAGGGAACACTGGTCACCGTGAGCTCAGCTTCCACCAAGGGCCCATCCGTCTTCCCCCTG
GCGCCCTGCTCCAGGAGCACCTCCGAGAGCACAGCCGCCCTGGGCTGCCTGGTCAAGGACTA
CTTCCCCGAACCGGTGACGGTGTCGTGGAACTCAGGCGCCCTGACCAGCGGCGTGCACACCT
TCCCGGCTGTCCTACAGTCCTCAGGACTCTACTCCCTCAGCAGCGTGGTGACCGTGCCCTCCA
GCAGCTTGGGCACGAAGACCTACACCTGCAACGTAGATCACAAGCCCAGCAACACCAAGGTG
GACAAGAGAGTTGAGTCCGGTGGCGGCGGAAGCGGCGGCGGAGGCAGCGGCGGAGGCGGC
AGCGGTAGACCATTCGTAGAGATGTACAGCGAAATCCCCGAAATCATCCACATGACTGAA
GGAAGGGAGCTCGTCATCCCCTGCCGGGTTACGTCACCTAACATCACTGTTACTCTGAA
GAAGTTCCCACTCGACACTTTGATCCCTGATGGAAAACGCATCATCTGGGACAGCAGAA
AGGGCTTCATCATCTCAAATGCAACGTACAAAGAAATCGGACTCCTGACCTGTGAAGCA
ACAGTCAATGGACATTTGTATAAGACAAACTATCTCACACATCGACAGACCAATACAATC
ATCGATGTGGTTCTGAGCCCGTCTCATGGAATCGAACTATCTGTTGGAGAAAAGCTCGT
CCTGAATTGTACAGCAAGAACTGAACTGAATGTGGGAATCGACTTCAACTGGGAATACC
CTTCTTCGAAGCATCAGCATAAGAAACTCGTAAACCGAGACCTAAAGACCCAGTCTGGG
AGCGAGATGAAGAAATTCTTGAGCACCCTGACTATCGATGGTGTAACCCGGAGCGACCA
GGGATTGTACACCTGTGCAGCATCCAGCGGGCTGATGACCAAGAAGAACAGCACATTCG
TCAGGGTCCATGAACCCGGCGGGGGGGGTAGCGGCGGCGGGGGCTCAGGTGGGGGGGGCT
CAAAATATGGTCCCCCATGCCCACCATGCCCAGCACCTGAGTTCCTGGGGGGACCATCAGTCT
TCCTGTTCCCCCCAAAACCCAAGGACACTCTCATGATCTCCCGGACCCCTGAGGTCACGTGCG
TGGTGGTGGACGTGAGCCAGGAAGACCCCGAGGTCCAGTTCAACTGGTACGTGGATGGCGTG
GAGGTGCATAATGCCAAGACAAAGCCGCGGGAGGAGCAGTTCAACAGCACGTACCGTGTGGT
CAGCGTCCTCACCGTCCTGCACCAGGACTGGCTGAACGGCAAGGAGTACAAGTGCAAGGTCT
CCAACAAAGGCCTCCCGTCCTCCATCGAGAAAACCATCTCCAAAGCCAAAGGGCAGCCCCGA
GAGCCACAGGTGTACACCCTGCCCCCATCCCAGGAGGAGATGACCAAGAACCAGGTCAGCCT
GACCTGCCTGGTCAAAGGCTTCTACCCCAGCGACATCGCCGTGGAGTGGGAGAGCAATGGGC
AGCCGGAGAACAACTACAAGACCACGCCTCCCGTGCTGGACTCCGACGGCTCCTTCTTCCTCT
ACAGCAGGCTAACCGTGGACAAGAGCAGGTGGCAGGAGGGGAATGTCTTCTCATGCTCCGTG
ATGCATGAGGCTCTGCACAACCACTACACACAGAAGAGCCTCTCCCTGTCTCTGGGTAAATAG
   Note: The underlined and bold sequence is the VEGFR nucleic acid sequence.
   Amino acid sequence of fusion protein Hib-PDV heavy chain (SEQ ID NO: 70): 
QVQLVESGGG VVQPGRSLRL DCKASGITFS NSGMHWVRQA PGKGLEWVAV IWYDGSKRYY 60

ADSVKGRFTI SRDNSKNTLF LQMNSLRAED TAVYYCATND DYWGQGTLVT VSSASTKGPS 120

VFPLAPCSRS TSESTAALGC LVKDYFPEPV TVSWNSGALT SGVHTFPAVL QSSGLYSLSS 180

VVTVPSSSLG TKTYTCNVDH KPSNTKVDKR VESGGGGSGG GGSGGGGSGR PFVEMYSEIP 240

EIIHMTEGRE LVIPCRVTSP NITVTLKKFP LDTLIPDGKR IIWDSRKGFI ISNATYKEIG 300

LLTCEATVNG HLYKTNYLTH RQTNTIIDVV LSPSHGIELS VGEKLVLNCT ARTELNVGID 360

FNWEYPSSKH QHKKLVNRDL KTQSGSEMKK FLSTLTIDGV TRSDQGLYTC AASSGLMTKK 420

NSTFVRVHEP GGGGSGGGGS GGGGSKYGPP CPPCPAPEFL GGPSVFLFPP KPKDTLMISR 480

TPEVTCVVVD VSQEDPEVQF NWYVDGVEVH NAKTKPREEQ FNSTYRVVSV LTVLHQDWLN 540

GKEYKCKVSN KGLPSSIEKT ISKAKGQPRE PQVYTLPPSQ EEMTKNQVSL TCLVKGFYPS 600

DIAVEWESNG QPENNYKTTP PVLDSDGSFF LYSRLTVDKS RWQEGNVFSC SVMHEALHNH 660

YTQKSLSLSL GK 672
   Note: The peptide linker is in italics, and the underlined and bold sequence is the VEGF amino acid sequence.
   Nucleic acid sequence of fusion protein Hib-PDV light chain (SEQ ID NO: 77): 
GAGATCGTGCTGACACAGAGCCCAGCCACTCTGTCACTGTCCCCAGGAGAAAGGGCTACTCT
GTCTTGCCGGGCAAGCCAGTCTGTCTCCAGCTACCTGGCCTGGTATCAGCAGAAGCCCGGACA
GGCTCCTAGACTGCTGATCTACGACGCAAGTAACAGAGCCACCGGCATCCCCGCACGCTTCAG
TGGCTCAGGCTCCGGAACAGACTTTACTCTGACCATCTCTAGTCTGGAGCCTGAAGATTTCGC
CGTGTACTATTGTCAGCAGAGCTCTAATTGGCCTAGAACCTTCGGCCAGGGCACCAAAGTCGA
GATCAAGCGTACGGTGGCTGCACCATCTGTCTTCATCTTCCCGCCATCTGATGAGCAGTTGAAA
TCTGGAACTGCCTCTGTTGTGTGCCTGCTGAATAACTTCTATCCCAGAGAGGCCAAAGTACAG
TGGAAGGTGGATAACGCCCTCCAATCGGGTAACTCCCAGGAGAGTGTCACAGAGCAGGACAG
CAAGGACAGCACCTACAGCCTCAGCAGCACCCTGACGCTGAGCAAAGCAGACTACGAGAAA
CACAAAGTCTACGCCTGCGAAGTCACCCATCAGGGCCTGAGCTCGCCCGTCACAAAGAGCTT
CAACAGGGGAGAGTGTTAG
   Amino acid sequence of fusion protein Hib-PDV light chain (SEQ ID NO: 71): EIVLTQSPAT LSLSPGERAT LSCRASQSVS SYLAWYQQKP GQAPRLLIYD ASNRATGIPA 60 
RFSGSGSGTD FTLTISSLEP EDFAVYYCQQ SSNWPRTFGQ GTKVEIKRTV AAPSVFIFPP 120

SDEQLKSGTA SVVCLLNNFY PREAKVQWKV DNALQSGNSQ ESVTEQDSKD STYSLSSTLT 180

LSKADYEKHK VYACEVTHQG LSSPVTKSFN RGEC 214
- The amino acid sequences of the heavy chain and light chain of the Control protein 1
   Amino acid sequence of Control protein 1 heavy chain (SEQ ID NO: 124): 
QVQLQESGPG LVKPSETLSL TCTVSGFSLS RYSVHWIRQP PGKGLEWLGM IWGVGTTDYN 60

SALKSRLTIS KDTSKNQFSL KLSSVTAADT AVYYCARNWG TADYFDYWGQ GTTVTVSSAS 120

TKGPSVFPLA PSSKSTSGGT AALGCLVKDY FPEPVTVSWN SGALTSGVHT FPAVLQSSGL 180

YSLSSVVTVP SSSLGTQTYI CNVNHKPSNT KVDKKVEPKS CDKTHTCPPC PAPELLGGPS 240

VFLFPPKPKD TLMISRTPEV TCVVVDVSHE DPEVKFNWYV DGVEVHNAKT KPREEQYNST 300

YRVVSVLTVL HQDWLNGKEY KCKVSNKALP APIEKTISKA KGQPREPQVY TLPPSREEMT 360

KNQVSLTCLV KGFYPSDIAV EWESNGQPEN NYKTTPPVLD SDGSFFLYSK LTVDKSRWQQ 420

GNVFSCSVMH EALHNHYTQK SLSLSPGKGG GGSGGGGSGG GGSTIPPHVQ KSVNNDMIVT 480

DNNGAVKFPQ LCKFCDVRFS TCDNQKSCMS NCSITSICEK PQEVCVAVWR KNDENITLET 540

VCHDPKLPYH DFILEDAASP KCIMKEKKKP GETFFMCSCS SDECNDNIIF SEEYNTSNPD 600
   Note: The peptide linker is in italics, and the underlined and bold sequence is the TGF-βRII amino acid sequence.
   Amino acid sequence of Control protein 1 light chain (SEQ ID NO: 125): 
DIVLTQSPAS LAVSPGQRAT ITCRASKSVH TSGYSYMHWY QQKPGQPPKL LIYLASNLES 60

GVPARFSGSG SGTDFTLTIN PVEANDTANY YCQHSGELPY TFGGGTKVEI KRTVAAPSVF 120

IFPPSDEQLK SGTASVVCLL NNFYPREAKV QWKVDNALQS GNSQESVTEQ DSKDSTYSLS 180

STLTLSKADY EKHKVYACEV THQGLSSPVT KSFNRGEC 218
- The amino acid sequences of the heavy chain and light chain of the Control protein 2
   Amino acid sequence of Control protein 2 heavy chain (SEQ ID NO: 126): 
QVQLVESGGG VVQPGRSLRL DCKASGITFS NSGMHWVRQA PGKGLEWVAV IWYDGSKRYY 60

ADSVKGRFTI SRDNSKNTLF LQMNSLRAED TAVYYCATND DYWGQGTLVT VSSASTKGPS 120

VFPLAPCSRS TSESTAALGC LVKDYFPEPV TVSWNSGALT SGVHTFPAVL QSSGLYSLSS 180

VVTVPSSSLG TKTYTCNVDH KPSNTKVDKR VESKYGPPCP PCPAPEFLGG PSVFLFPPKP 240

KDTLMISRTP EVTCVVVDVS QEDPEVQFNW YVDGVEVHNA KTKPREEQFN STYRVVSVLT 300

VLHQDWLNGK EYKCKVSNKG LPSSIEKTIS KAKGQPREPQ VYTLPPSQEE MTKNQVSLTC 360

LVKGFYPSDI AVEWESNGQP ENNYKTTPPV LDSDGSFFLY SRLTVDKSRW QEGNVFSCSV 420

MHEALHNHYT QKSLSLSLGK GGGGSGGGGS GGGGSVIHVT KEVKEVATLS CGHNVSVEEL 480

AQTRIYWQKE KKMVLTMMSG DMNIWPEYKN RTIFDITNNL SIVILALRPS DEGTYECVVL 540

KYEKDAFKRE HLAEVTLSVK ADFPTPSISD FEIPTSNIRR IICSTSGGFP EPHLSWLENG 600

EELNAINTTV SQDPETELYA VSSKLDFNMT TNHSFMCLIK YGHLRVNQTF NWN 653
   Note: The peptide linker is in italics, and the underlined and bold sequence is the CD80 amino acid sequence.
   Amino acid sequence of Control protein 2 light chain (SEQ ID NO: 127): 
EIVLTQSPAT LSLSPGERAT LSCRASQSVS SYLAWYQQKP GQAPRLLIYD ASNRATGIPA 60

RFSGSGSGTD FTLTISSLEP EDFAVYYCQQ SSNWPRTFGQ GTKVEIKRTV AAPSVFIFPP 120

SDEQLKSGTA SVVCLLNNFY PREAKVQWKV DNALQSGNSQ ESVTEQDSKD STYSLSSTLT 180

LSKADYEKHK VYACEVTHQG LSSPVTKSFN RGEC 214
- The amino acid sequences of the heavy chain and light chain of the Control protein 3
   Amino acid sequence of Control protein 3 heavy chain (SEQ ID NO: 128): 
QVQLVESGGG VVQPGRSLRL DCKASGITFS NSGMHWVRQA PGKGLEWVAV IWYDGSKRYY 60

ADSVKGRFTI SRDNSKNTLF LQMNSLRAED TAVYYCATND DYWGQGTLVT VSSASTKGPS 120

VFPLAPCSRS TSESTAALGC LVKDYFPEPV TVSWNSGALT SGVHTFPAVL QSSGLYSLSS 180
 
VVTVPSSSLG TKTYTCNVDH KPSNTKVDKR VESKYGPPCP PCPAPEFLGG PSVFLFPPKP 240

KDTLMISRTP EVTCVVVDVS QEDPEVQFNW YVDGVEVHNA KTKPREEQFN STYRVVSVLT 300

VLHQDWLNGK EYKCKVSNKG LPSSIEKTIS KAKGQPREPQ VYTLPPSQEE MTKNQVSLTC 360

LVKGFYPSDI AVEWESNGQP ENNYKTTPPV LDSDGSFFLY SRLTVDKSRW QEGNVFSCSV 420

MHEALHNHYT QKSLSLSLGK GGGGSGGGGS GGGGSGRPFV EMYSEIPEII HMTEGRELVI 480

PCRVTSPNIT VTLKKFPLDT LIPDGKRIIW DSRKGFIISN ATYKEIGLLT CEATVNGHLY 540

KTNYLTHRQT NTIIDVVLSP SHGIELSVGE KLVLNCTART ELNVGIDFNW EYPSSKHQHK 600

KLVNRDLKTQ SGSEMKKFLS TLTIDGVTRS DQGLYTCAAS SGLMTKKNST FVRVHEP 657
   Note: The peptide linker is in italics, and the underlined and bold sequence is the VEGFR amino acid sequence.
   Amino acid sequence of Control protein 3 light chain (SEQ ID NO: 129): 
EIVLTQSPAT LSLSPGERAT LSCRASQSVS SYLAWYQQKP GQAPRLLIYD ASNRATGIPA 60

RFSGSGSGTD FTLTISSLEP EDFAVYYCQQ SSNWPRTFGQ GTKVEIKRTV AAPSVFIFPP 120

SDEQLKSGTA SVVCLLNNFY PREAKVQWKV DNALQSGNSQ ESVTEQDSKD STYSLSSTLT 180

LSKADYEKHK VYACEVTHQG LSSPVTKSFN RGEC 214
- TGF-β-R-His
   TGF-β-R-His is the His-tagged TGF-βRII extracellular region fragment, wherein the amino acid sequence of the TGF-βRII extracellular region fragment (SEQ ID NO: 137) is as follows: 
TIPPHVQKSV NNDMIVTDNN GAVKFPQLCK FCDVRFSTCD NQKSCMSNCS ITSICEKPQE 60

VCVAVWRKND ENITLETVCH DPKLPYHDFI LEDAASPKCI MKEKKKPGET FFMCSCSSDE 120

CNDNIIFSEE YNTSNPD 137
- VEGF-R-His amino acid sequence
   VEGF-R-His is the His-tagged combination fragment of the second extracellular region of VEGFR1 and the third extracellular region of VEGFR2, wherein the amino acid sequence of combination fragment of the second extracellular region of VEGFR1 and the third extracellular region of VEGFR2 (SEQ ID NO: 130) is as follows: 
GRPFVEMYSE IPEIIHMTEG RELVIPCRVT SPNITVTLKK FPLDTLIPDG KRIIWDSRKG 60

FIISNATYKE IGLLTCEATV NGHLYKTNYL THRQTNTIID VVLSPSHGIE LSVGEKLVLN 120

CTARTELNVG IDFNWEYPSS KHQHKKLVNR DLKTQSGSEM KKFLSTLTID GVTRSDQGLY 180

TCAASSGLMT KKNSTFVRVH EP 202
- CD80-His amino acid sequence
   CD80-His is the His-tagged CD80 fragment, wherein the amino acid sequence of the CD80 fragment (SEQ ID NO: 32) is as follows:

VIHVTKEVKE VATLSCGHNV SVEELAQTRI YWQKEKKMVL TMMSGDMNIW PEYKNRTIFD 60

ITNNLSIVIL ALRPSDEGTY ECVVLKYEKD AFKREHLAEV TLSVKADFPT PSISDFEIPT 120

SNIRRIICST SGGFPEPHLS WLENGEELNA INTTVSQDPE TELYAVSSKL DFNMTTNHSF 180

MCLIKYGHLR VNQTFNWN 198

### Example 2 CHO expression

A. Cell Thawing and Expansion:
CHO host cells were thawed with a culture volume of 10 mL and culture conditions of 37.0°C, 200 rpm, 8% CO₂, and 80% humidity. After thawing, the cell density was 2.82×10⁵ cells/mL, and the viability was 95.41%.

After 72 hours of cell expansion and culture, the density of viable cells was 2.22×10⁶ cells/mL, and the viability was 98.32%.

The cells were re-seeded at 5×10⁵ cells/mL for cell re-seeding operation. After 48 hours of continuous culture, the density of viable cells was 2×10⁶ cells/mL, and the viability was 98.68%.

The cells were seeded into a 6-well plate at 5×10⁵ cells/well, with a culture volume of 2 mL/well, and placed in a carbon dioxide incubator (culture conditions: 37°C, 8% CO₂) overnight for adherence and transfection the next day.

### B. Transfection

The transfection reagent used Lipofectamine^{®}LTX Reagent (Invitrogen, catalog No. 15338-100), containing LTX and Plus reagents.

Transfection procedure: Changing the medium in the 6-well plate and preparing the transfection complex: LTX: 9 µl/well + plasmid: 2.5 µg/well + plus: 2.5 µl/well. The transfection complex was added at 250 µl/well to the 6-well plate. The medium in each well of the 6-well plate was changed 5 hours after cell transfection. After 48 hours of transfection, the supernatant was taken to detect the antibody content of the supernatant by the double antigen ELISA method.

### C. Batch culture and fed-batch culture

After 48 hours of transfection, the cells in the six-well plate were digested, collected into a cell culture tube, and adapted to grow in suspension at a cell density of 3×10⁵ cells/mL. When the cell viability returned to about 90%, Hib-PLT expressing cell pool and the Control protein 1 expressing cell pool were cultured in batch culture, and Hib-PDC, Control protein 2, Hib-PDV, and Control protein 3 were cultured in fed-batch culture.

Batch culture: Cells were cultured in a TPP cell culture tube at a cell density of 3×10⁵ cells/mL, with a volume of 30 mL and culture conditions at 37.0°C, 200 rpm, 8% CO₂, and 80% humidity, and the cell viability and metabolism were monitored every day. The culture was terminated until the viability was <60%, and the cell supernatant after the completion of the culture was taken for concentration detection.

Fed-batch culture: Cells were cultured in a TPP cell culture tube at a cell density of 3×10⁵ cells/mL, with a volume of 30 mL and culture conditions at 37.0°C, 200 rpm, 8% CO₂, and 80% humidity. The cell concentrated medium was given according to a certain fed-batch strategy (see Table 5), during which Glc was controlled at 2-6 g/L. The culture was terminated until the viability was <60%, and the cell supernatant after the completion of the culture was taken for concentration detection.

**Table 5. Fed-batch strategy**

| | |
|---|---|
| Day 0 | 3×10⁵/ml, culture volume 30 ml |
| Day 4, 6, 8, 10, 12 | 900 µL CellBoost7A, 90 µL CellBoost7B |
| Day 16 | Detecting VCD and viability, stocking the sample and terminating the experiment |

Table 6 shows the expression level results of Hibody bispecific fusion proteins Hib-PLT, Hib-PDC and Hib-PDV and their corresponding control proteins. The results show that the relative value of the increase in Hib-PLT expression level increased by 52.85% compared with its corresponding control protein; the relative value of the increase in Hib-PDC expression level increased by 33.43% compared with its corresponding control protein; and the relative value of the increase in Hib-PDV expression level increased by 25.6% compared with its corresponding control protein, indicating the expression level of the Hibody bispecific fusion protein provided in the present disclosure is significantly superior to that of the corresponding control protein, and the bispecific antibody provided in the present disclosure has unexpected technical effects.

**Table 6 Expression level of bispecific antibodies**

| **Category** | **Method** | **Expression level** | **Relative value of increase** |
|---|---|---|---|
| Hib-PLT | Batch culture | 188 mg/L | 52.85% |
| Control protein 1 | Batch culture | 123 mg/L | |
| Hib-PDC | Fed-batch culture | 349.59 mg/L | 33.43% |
| Control protein 2 | Fed-batch culture | 262 mg/L | |
| Hib-PDV | Fed-batch culture | 243.95 mg/L | 25.6% |
| Control protein 3 | Fed-batch culture | 194.22 mg/L | |

| | | | |
|---|---|---|---|
| Note: Relative value of increase = (the expression level of the bispecific fusion protein provided in the present disclosure - the expression level of the corresponding control protein) / the expression level of the corresponding control protein. | | | |

### Example 3 Determination of binding activity

### 1. Determination of Hib-PLT binding activity:

(1) Coating antigen: TGF-β1 was diluted to 2 µg/ml with coating buffer, added to a 96-well plate at 100 µl/well, and allowed to stand overnight at 2-8°C;
(2) Washing the plate: The plate was automatically washed 3 times at 350 µl/well with PBST used as the eluent, and was patted to dryness after washed;
(3) Blocking the plate: The plate was blocked at 300 µl/well with 3% BSA-PBST used as the blocking solution, and incubated at 37°C for 2 hours;
(4) Washing the plate: The plate was automatically washed 3 times at 350 µl/well with PBST used as the eluent, and was patted to dryness after washed;
(5) Adding samples: Each concentration point of samples is as follows. The sample with each concentration point was sequentially added to a 96-well plate at 100 µl/well, and each concentration point was in parallel with 2 replicate wells, and the plate was incubated at 37°C for 2 hours;

| Test group | S01 | S02 | S03 | S04 | S05 | S06 | S07 | S08 | S09 | S10 | S11 |
|---|---|---|---|---|---|---|---|---|---|---|---|
| Hib-PLT TGF-β-R-His | 555.556 | 55.556 | 5.556 | 1.852 | 0.617 | 0.206 | 0.069 | 0.023 | 0.008 | 0.001 | 0.000 |
| | 64516.000 | 6451.600 | 645.160 | 215.053 | 71.684 | 23.895 | 7.965 | 2.655 | 0.885 | 0.088 | 0.009 |

| | | | | | | | | | | | |
|---|---|---|---|---|---|---|---|---|---|---|---|
| (Unit: nM) | | | | | | | | | | | |

(6) Washing the plate: The plate was automatically washed 3 times at 350 µl/well with PBST used as the eluent, and was patted to dryness after washed;
(7) Adding the secondary antibody:
- Hib-PLT sample well: Goat-anti-Human-Fc-HRP was diluted at 1:5000, and added at 100 µl/well. Then the plate was incubated at 37°C for 2 hours;
- TGF-β-R-His well: Anti-His-HRP was diluted at 1:5000, and added at 100 µl/well. Then the plate was incubated at 37°C for 2 hours;

(8) Washing the plate: The plate was automatically washed 5 times at 350 µl/well with PBST used as the eluent, and was patted to dryness after washed;
(9) Color development: The plate was subjected to color development for 2 minutes at 100 µl/well with TMB used as the color development solution;
(10) Terminating the color development: The plate was terminated for color development at 50 µl/well with 2M H₂SO₄ used as the stop solution;
(11) Reading: The absorbance values were read at 450/655 nm respectively using the MD microplate reader. The concentration of the sample to be tested was used as the abscissa and the absorbance value as the ordinate to plot making a 4-PL curve, and the software automatically gave the EC50 value.

### 2. Determination of Hib-PDV binding activity:

(1) Coating antigen: VEGF was diluted to 1 µg/ml with coating buffer, added to a 96-well plate at 100 µl/well, and allowed to stand overnight at 2-8°C;
(2) Washing the plate: The plate was automatically washed 3 times at 350 µl/well with PBST used as the eluent, and was patted to dryness after washed;
(3) Blocking the plate: The plate was blocked at 300 µl/well with 3% BSA-PBST used as the blocking solution, and incubated at 37°C for 2 hours;
(4) Washing the plate: The plate was automatically washed 3 times at 350 µl/well with PBST used as the eluent, and was patted to dryness after washed;
(5) Adding samples: Each concentration point of samples is as follows. The sample with each concentration point was sequentially added to a 96-well plate at 100 µl/well, and each concentration point was in parallel with 2 replicate wells, and the plate was incubated at 37°C for 2 hours;

| Test group | S01 | S02 | S03 | S04 | S05 | S06 | S07 | S08 | S09 | S10 | S11 |
|---|---|---|---|---|---|---|---|---|---|---|---|
| Hib-PDV | 555.556 | 55.556 | 5.556 | 1.852 | 0.617 | 0.206 | 0.069 | 0.023 | 0.005 | 0.001 | 0.000 |
| VEGFR-His | 43478.300 | 4347.830 | 434.783 | 144.928 | 48.309 | 16.103 | 5.368 | 1.789 | 0.358 | 0.036 | 0.004 |

| | | | | | | | | | | | |
|---|---|---|---|---|---|---|---|---|---|---|---|
| (Unit: nM) | | | | | | | | | | | |

(6) Washing the plate: The plate was automatically washed 3 times at 350 µl/well with PBST used as the eluent, and was patted to dryness after washed;
(7) Adding the secondary antibody:
- Hib-PDV sample well: Goat-anti-Human-Fc-HRP was diluted at 1:5000, and added at 100 µl/well. Then the plate was incubated at 37°C for 2 hours;
- VEGFR-His well: Anti-His-HRP was diluted at 1:5000, and added at 100 µl/well. Then the plate was incubated at 37°C for 2 hours;

(8) Washing the plate: The plate was automatically washed 5 times at 350 µl/well with PBST used as the eluent, and was patted to dryness after washed;
(9) Color development: The plate was subjected to color development for 2 minutes at 100 µl/well with TMB used as the color development solution;
(10) Terminating the color development: The plate was terminated for color development at 50 µl/well with 2M H₂SO₄ used as the stop solution;
(11) Reading: The absorbance values were read at 450/655 nm respectively using the MD microplate reader. The concentration of the sample to be tested was used as the abscissa and the absorbance value as the ordinate to plot making a 4-PL curve, and the software automatically gave the EC50 value.

### 3. Determination of Hib-PDC binding activity:

(1) Coating antigen: CTLA-4 Protein was redissolved with 1 mL of sterilized water at a concentration of 200 µg/mL, diluted to 20 µg/ml with coating buffer, added to a 96-well plate at 100 µl/well, and allowed to stand overnight at 2-8°C;
(2) Washing the plate: The plate was automatically washed 3 times at 350 µl/well with PBST used as the eluent, and was patted to dryness after washed;
(3) Blocking the plate: The plate was blocked at 300 µl/well with 3% BSA-PBST used as the blocking solution, and incubated at 37°C for 2h±20min;
(4) Washing the plate: The plate was automatically washed 3 times at 350 µl/well with PBST used as the eluent, and was patted to dryness after washed;
(5) Adding samples: The biotinylated sample was diluted with PBST. Each concentration point of samples was as follows. The sample with each concentration point was sequentially added to a 96-well plate at 100 µl/well, and each concentration point was in parallel with 2 replicate wells, with the blank well being PBST, and the plate was incubated at 37°C for 2h±20min;

| Test group | S01 | S02 | S03 | S04 | S05 | S06 | S07 | S08 | S09 | S10 | S11 |
|---|---|---|---|---|---|---|---|---|---|---|---|
| Hib-PDC | 1000000 | 100000 | 10000 | 3333.333 | 1111.111 | 370.370 | 123.457 | 41.152 | 13.717 | 1.372 | 0.137 |
| CD80-His | 1000000 | 100000 | 10000 | 3333.333 | 1111.111 | 370.370 | 123.457 | 41.152 | 13.717 | 1.372 | 0.137 |

| | | | | | | | | | | | |
|---|---|---|---|---|---|---|---|---|---|---|---|
| (Unit: nM) | | | | | | | | | | | |

(6) Washing the plate: The plate was automatically washed 3 times at 350 µl/well with PBST used as the eluent, and was patted to dryness after washed;
(7) Adding the secondary antibody:
- Hib-PDC sample well and CD80-His well: Streptavidin-HRP was diluted at 1:2000, and added at 100 µl/well. Then the plate was incubated at 37°C for 1h±10min;

(8) Washing the plate: The plate was automatically washed 5 times at 350 µl/well with PBST used as the eluent, and was patted to dryness after washed;
(9) Color development: TMB Double Slow substrate was added at 100 µl/well and the plate was subjected to color development for 6 minutes at room temperature shielded from light;
(10) Terminating the color development: The plate was terminated for color development at 50 µl/well with 2M H₂SO₄ used as the stop solution;
(11) Reading: The absorbance values were read at 450/655 nm respectively using the MD microplate reader. The concentration of the sample to be tested was used as the abscissa and the absorbance value as the ordinate to plot making a 4-PL curve, and the software automatically gave the EC50 value. (see table 7).

**Table 7. EC50 value of each test group**

| **Test group** | **EC50 value** |
|---|---|
| Hib-PLT | 0.164 nM |
| TGF-β-R-His | 11.60 nM |
| Hib-PDV | 0.061 nM |
| VEGFR-His | 2154 nM |
| Hib-PDC | 24.36 nM |
| CD80-His | 50.69 nM |

The results of determination of binding activity showed that, comparison between Hib-PLT and TGF-β receptor monomer: Hib-PLT has an EC50 value of 0.164 nM, TGF-β receptor monomer has an EC50 value of 11.60 nM, and the binding activity of Hib-PLT is about 70 times better than that of TGF-β receptor monomer; comparison between Hib-PDV and VEGF receptor monomer: Hib-PDV has an EC50 value of 0.061 nM, VEGF receptor monomer has an EC50 value of 2154 nM, and the binding activity of Hib-PDV binding activity is about 35,311 times better than that of VEGF receptor monomer; comparison between Hib-PDC and CD80 monomer: Hib-PDC has an EC50 value of 24.36 nM, CD80 monomer has an EC50 value of 50.69 nM, and the binding activity of Hib-PDC is about 2 times better than that of CD80 monomer. That is, the binding activities of Hib-PLT, Hib-PDV, and Hib-PDC constructed with the bi-antibody model provided by the present disclosure are much better than that of their corresponding monomers.

### Example 4 The efficacy of Hib-PDV on subcutaneously transplanted tumor of colon cancer MC38 cells in C57BL/6J-hPD-1 mice

Thirty-two 7-week-old, C57BL/6J humanized PD-1 transgenic mice (from Shanghai Model Organisms Center, Inc.) were collected. 0.1 mL of colon cancer MC38 cell suspension (1×10⁶ cells/mouse) was inoculated subcutaneously into the right flank of mice. When the tumor grew to about 100 mm³, mice were randomly divided into groups according to the tumor size, namely Vehicle (PBS) group, Hib-PDV (1.25 mg/kg) group, Hib-PDV (2.5 mg/kg) group and Hib-PDV (5 mg/kg) group, respectively, a total of 4 groups, 8 tumor-bearing mice in each group. They were administered intraperitoneally once a week, a total of 3 administrations, during the administration period, the long diameter and short diameter of the tumor and body weight were measured twice a week. On the 21^{st} day after administration, the tumor growth inhibition rate (TGI_{RTV}) was calculated for drug efficacy evaluation, as shown in Table 8.

**Table 8. Dosage regimen of Hib-PDV on tumor inhibitory activity of mouse colon cancer**

| **Group** | **Administration group** | **Dosage (mg/kg)** | **Administration route** | **Administration frequency** | **Administration volume** |
|---|---|---|---|---|---|
| 1 | Vehicle | - | i.p. | QW×3 | 10 mL/kg |
| 2 | Hib-PDV | 5 | i.p. | QW×3 | 10 mL/kg |
| 3 | Hib-PDV | 2.5 | i.p. | QW×3 | 10 mL/kg |
| 4 | Hib-PDV | 1.25 | i.p. | QW×3 | 10 mL/kg |

Calculation formula:
Tumor volume: TV=D₁×D₂²/2, where D1 and D2 represent the long diameter and short diameter of the tumor, respectively;
Relative tumor volume: RTV=T_{VT}/T_{V1}, where T_{V1} is the tumor volume before administration, and T_{VT} is the tumor volume at each measurement;
Relative tumor inhibition rate: TGI_{RTV}(%)=(1-T_{RTV}/C_{RTV})×100%, where T_{RTV} represents the RTV of the administration group or the positive control group, and C_{RTV} represents the RTV of the negative control group.

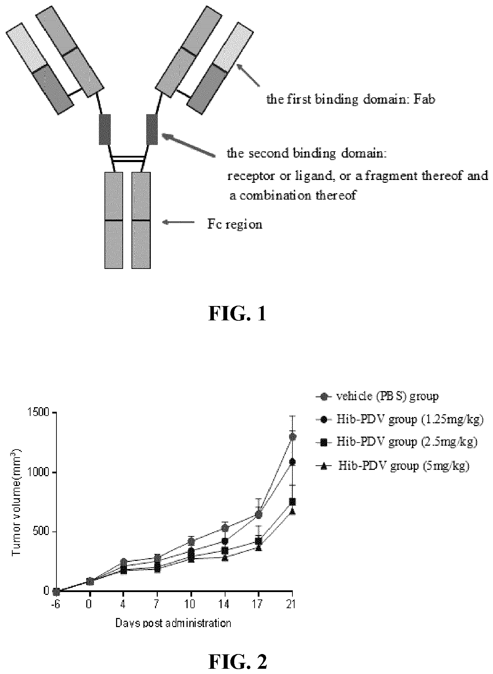

Results and conclusions: The tumor inhibitory effects of Hib-PDV on mouse colon cancer model are shown in Table 9 and FIG. 2, where Table 9 shows the tumor volume and tumor growth inhibition rate of Hib-PDV on mouse colon cancer model, and FIG. 2 is a graph of tumor growth after administration. Experimental results show that Hib-PDV has a significant inhibitory effect on mouse colon cancer model tumor.

**Table 9. effect of Hib-PDV on the tumor volume and tumor inhibition rate of mouse colon cancer model (mean+SEM)**

| **Administration group** | **Tumor volume (TV, mm³)** | | **TGIRTV (%)** |
|---|---|---|---|
| | D0 | D21 | |
| Vehicle | 86+10 | 1299+174 | - |
| Hib-PDV (5 mg/kg) | 86+10 | 675+218 | 46 |
| Hib-PDV (2.5 mg/kg) | 86+8 | 755+305 | 47 |
| Hib-PDV (1.25 mg/kg) | 85+8 | 1088+259 | 14 |

### Example 5 The efficacy of Hib-PDC on subcutaneously transplanted tumor of transgenic hPD-L1 MC38 cells in C57BL/6J-hPD-1 mice

Thirty-two 7-9 week-old, humanized C57BL/6J humanized PD-1 transgenic female mice (from Shanghai Model Organisms Center, Inc.) were collected. 0.1 mL of transgenic hPD-L1 MC38 cell suspension (1×10⁶ cells/mouse) was subcutaneously implanted in the back of the right forelimb of the mouse. When the tumor grew to about 100 mm³, mice were randomly divided into groups according to the tumor size, namely Vehicle (PBS) group, Hib-PDC (1.25 mg/kg) group, Hib-PDC (2.5 mg/kg) group and Hib-PDC (5 mg/kg) group, respectively, a total of 4 groups, 8 tumor-bearing mice in each group. They were administered intraperitoneally once a week, a total of 3 administrations, during the administration period, the long diameter and short diameter of the tumor and body weight were measured twice a week. On the 21st day after administration, the tumor growth inhibition rate (TGI_{RTV}) was calculated for drug efficacy evaluation, as shown in Table 10.

**Table 10. Dosage regimen of Hib-PDC on tumor inhibitory activity of mouse colon cancer**

| **Group** | **Administration group** | **Dosage (mg/kg)** | **Administration route** | **Administration volume** | **Administration frequency** |
|---|---|---|---|---|---|
| 1 | Vehicle(PBS) | -- | i.p. | 10 ml/kg | QW×3 |
| 2 | Hib-PDC | 1.25 | i.p. | 10 ml/kg | QW×3 |
| 3 | Hib-PDC | 2.5 | i.p. | 10 ml/kg | QW×3 |
| 4 | Hib-PDC | 5 | i.p. | 10 ml/kg | QW×3 |

Calculation formula:
Tumor volume: TV=D₁×D₂²/2, where D1 and D2 represent the long diameter and short diameter of the tumor, respectively;
Relative tumor volume: RTV=T_{VT}/T_{V1}, where T_{V1} is the tumor volume before administration, and T_{VT} is the tumor volume at each measurement;
Relative tumor inhibition rate: TGI_{RTV}(%)=(1-T_{RTV}/C_{RTV})×100%, where T_{RTV} represents the RTV of the administration group or the positive control group, and C_{RTV} represents the RTV of the negative control group.

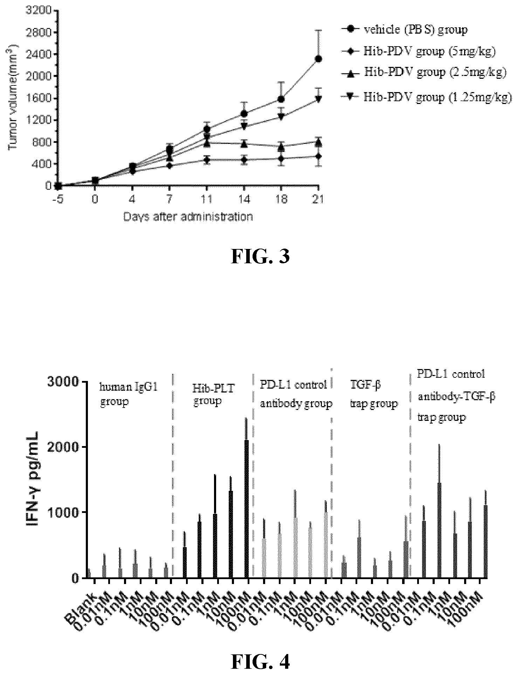

Results and conclusions: The tumor inhibitory effects of Hib-PDC on mouse colon cancer are shown in Table 11 and FIG. 3, where Table 11 shows the effect of Hib-PDC on the tumor volume and tumor inhibition rate on mouse colon cancer model, and FIG. 3 is a graph of tumor growth after administration. Experimental results show that Hib-PDC has a significant inhibitory effect on subcutaneously transplanted tumors of colorectal cancer in mice.

**Table 11. effect of Hib-PDC on the tumor volume and tumor inhibition rate of mouse colon cancer model (mean+SEM)**

| **Group** | **Dosage (mg/kg)** | **Tumor volume (TV, mm³)** | | **TGIRTV (%)** |
|---|---|---|---|---|
| | | **D0** | **D21** | |
| PBS | -- | 101±10 | 2319±517 | -- |
| Hib-PDC | 1.25 | 101±8 | 1577±211 | 31 |
| Hib-PDC | 2.5 | 101±9 | 813±74 | 63 |
| Hib-PDC | 5 | 101±9 | 541±179 | 77 |

### Example 6 Effect of Hib-PLT on the cytokine secretion in the mixed reaction of allogeneic lymphocytes

ELISA method was used to detect the concentration of cytokine IFN-γ in the cell supernatant to evaluate the role of Hib-PLT in activating CD4⁺ T cells. The mononuclear lymphocytes from donor 1 were induced into mature dendritic cells *in vitro* by DC cell rapid maturation in vitro kit (cat: CT-004, StemEry), the cells were collected after 48 h and diluted to 2×10⁵ cells/mL. The CD4⁺ T cells from donor 2 were enriched with Magnetic beads (EasySep^{™} Human CD4⁺ T Cell Enrichment Kit, StemCell, cat: 19052) and diluted to 1×10⁶ cells/mL. The mature dendritic cells and CD4⁺ T cells were mixed at a volume ratio of 1:1, and the mixed cell suspension was added to a 96-well plate at a volume of 100 µL/well. Hib-PLT, PD-L1 control antibody + TGF-β Trap (combination medication), PD-L1 control antibody, TGF-β Trap, and human IgG1 (Biolegend) were added to a 96-well plate containing mixed cell suspension at a final concentration of 0.01-100 nM. The plate was then placed in a 37°C, 5% CO₂ incubator for 120 h, centrifuged at 2000 rpm for 5 min, and 150-200 µL of the cell supernatant was collected. ELISA kits (Human IL-2 Quantikine ELISA Kit, R&D, cat: S2050; Human IFN-gamma Quantikine ELISA Kit, R&D, cat: SIF50C) were used to detect IFN-γ factor in the supernatant of the mixed reaction of allogeneic lymphocytes. One-way ANOVA was used to analysis the differences between groups.

Results and conclusions: As shown in FIG. 4, the level of Hib-PLT at a concentration of 10-100 nM promoting cell secretion of IFN-γ factor was significantly higher than that of the combination medication group of PD-L1 control antibody and TGF-β trap (p<0.05). The results show that at the concentration level of 10-100 nM, Hib-PLT is better than the combination medication group in promoting the activation of CD4⁺ T cells in the mixed lymphocytes reaction.

The present disclosure has been exemplified by various specific examples. However, a person of ordinary skill in the art can understand that the present disclosure is not limited to each specific embodiments, and a person of ordinary skill can make various changes or modifications within the scope of the present disclosure, and each technical feature mentioned in various places in this specification can be combined with each other without departing from the spirit and scope of the present disclosure. Such changes and modifications are within the scope of the present disclosure.

## Claims

1. A bispecific fusion protein, wherein the fusion protein has a structure in which a second binding domain is inserted in the hinge region of a full-length immunoglobulin G (IgG) through an optional peptide linker, and the Fab region of the IgG is a first binding domain; after inserting the second binding domain, the heavy chain of the IgG is the heavy chain of the fusion protein, and the light chain of the IgG is the light chain of the fusion protein; the second binding domain is selected from a human receptor or ligand, a fragment of the receptor or ligand, and a fragment combination of the receptor or ligand; wherein the receptor or ligand forms a dimerization or multimerization structure in a natural signaling pathway to activate or inhibit the signaling pathway, and the second binding domain and the first binding domain target different targets.

2. The fusion protein according to claim 1, wherein the first binding domain targets and binds to an immune checkpoint molecule or a tumor antigen.

3. The fusion protein according to claim 2, wherein the immune checkpoint molecule includes PD-1, PD-L1, CTLA-4, LAG-3, FGL1, TIM-3, Galectin-9, TIGIT, CD155 and CD47; the tumor antigen includes Claudin 18.2, HER-2, Mesothelin, BCMA, SSTR2, GPRC5D, PSMA, FCRH5, CD33, CD123, CD20, A33, CEA, CD28, DLL3, EGFR, VEGFR, VEGFR2, VEGF-A, Nectin-4, FGFR, C-met, RANKL, PDGF, PDGFR, PDGFRα, DLL4, Ang-1 and Ang-2.

4. The fusion protein according to claim 3, wherein the second binding domain targets and binds to human TGF-β, CTLA-4, VEGF, LAG3, CD27, 4-1BB, OX40, CD47, FGL1, TLT-2, CD28, HGF, CSF1, CXCL1, CXCL2, CXCL3, CXCL5, CXCL6, CXCL7, CXCL8, CXCL9, CXCL10, CXCL12, GITR, EGF and ICOSL.

5. The fusion protein according to claim 4, wherein the receptor or ligand is human TGF-β receptor (TGF-βR), CD80, CD86, VEGFR, VEGF-trap, FGL1, CD70, 4-1BBL, OX40L, SIRPα, B7-H3, C-met, CSF1R, CXCR2, CXCR3, CXCR4, GITRL, EGFR and ICOS.

6. The fusion protein according to claim 4, wherein the first binding domain and the second binding domain are selected from the following combinations:
(1) the first binding domain targets and binds to PD-L1, and the second binding domain targets and binds to human TGF-β, VEGF, FGL1, CD47, CD155, HGF, CSF1, CXCL1, CXCL2, CXCL3, CXCL5, CXCL6, CXCL7, CXCL8, CXCL9, CXCL10, CXCL12, EGF or ICOSL; or
(2) the first binding domain targets and binds to PD-1, and the second binding domain targets and binds to human CTLA-4, VEGF, HGF, EGF, CD28, LAG3, CD27, 4-1BB or OX40; or
(3) the first binding domain targets and binds to CTLA-4, and the second binding domain targets and binds to human CD28, VEGF, HGF, EGF, LAG3, CD27, 4-1BB or OX40; or
(4) the first binding domain targets and binds to LAG-3, and the second binding domain targets and binds to human CD28, VEGF, HGF, EGF, CTLA-4, CD27, 4-1BB or OX40; or
(5) the first binding domain targets and binds to FGL1, and the second binding domain targets and binds to human TGF-β, VEGF, CD47, CD155, HGF, CSF1, CXCL1, CXCL2, CXCL3, CXCL5, CXCL6, CXCL7, CXCL8, CXCL9, CXCL10, CXCL12, EGF or ICOSL; or
(6) the first binding domain targets and binds to TIM-3, and the second binding domain targets and binds to human VEGF, HGF, EGF, LAG3, CD27, 4-1BB or OX40; or
(7) the first binding domain targets and binds to Galectin-9, and the second binding domain targets and binds to human TGF-β, VEGF, CD47, CD155, HGF, CSF1, CXCL1, CXCL2, CXCL3, CXCL5, CXCL6, CXCL7, CXCL8, CXCL9, CXCL10, CXCL12, EGF or ICOSL; or
(8) the first binding domain targets and binds to TIGIT, and the second binding domain targets and binds to human TGF-β, HGF, EGF or VEGF; or
(9) the first binding domain targets and binds to CD155, and the second binding domain targets and binds to human TGF-β, VEGF, HGF, EGF, CD47 or CD155; or
(10) the first binding domain targets and binds to Claudin 18.2, HER-2, mesothelin, BCMA, SSTR2, GPRC5D, PSMA, FCRH5, CD33, CD123, CD20, A33, CEA, CD28, DLL3, EGFR, VEGFR, VEGFR2, VEGF-A, Nectin-4, FGFR, C-met, RANKL, PDGF, PDGFR, PDGFRα, DLL-4, Ang-1 or Ang-2, and the second binding domain targets and binds to human CTLA-4, TGF-β, VEGF, FGL1, LAG3, 4-1BB, OX40, CD27, CD28, CD47, CD155, HGF, CSF1, CXCL1, CXCL2, CXCL3, CXCL5, CXCL6, CXCL7, CXCL8, CXCL9, CXCL10, CXCL12, EGF or ICOSL.

7. The fusion protein according to claim 6, wherein the first binding domain and the receptor or ligand are selected from the following combinations:
(1) the first binding domain targets and binds to PD-L1, and the receptor or ligand is selected from TGF-βRII, VEGFR, LAG-3, SIRPα, TIGIT, C-MET, CSF1R, CXCR2, CXCR3, CXCR4, EGFR or ICOS; or
(2) the first binding domain targets and binds to PD-1, and the receptor or ligand is selected from human CD80, CD86, VEGFR, c-MET, EGFR, FGL1, CD70, 4-1BBL or OX40L; or
(3) the first binding domain targets and binds to CTLA-4, and the receptor or ligand is selected from human CD80, CD86, VEGFR, c-MET, EGFR, FGL1, CD70, 4-1BBL or OX40L; or
(4) the first binding domain targets and binds to LAG-3, and the receptor or ligand is selected from human VEGFR, c-MET, EGFR, CD80, CD86, CD70, 4-1BBL or OX40L; or
(5) the first binding domain targets and binds to FGL1, and the receptor or ligand is selected from human TGF-βRII, VEGFR, SIRPα, TIGIT, c-MET, CSF1R, CXCR2, CXCR3, CXCR4, EGFR or ICOS; or
(6) the first binding domain targets and binds to TIM-3, and the receptor or ligand is selected from human VEGFR, c-MET, EGFR, FGL1, CD70, 4-1BBL or OX40L; or
(7) the first binding domain targets and binds to Galectin-9, and the receptor or ligand is selected from human TGF-βRII, VEGFR, SIRPα, TIGIT, c-MET, CSF1R, CXCR2, CXCR3, CXCR4, EGFR or ICOS; or
(8) the first binding domain targets and binds to TIGIT, and the receptor or ligand is selected from human TGF-βRII, c-MET, EGFR or VEGFR; or
(9) the first binding domain targets and binds to CD155, and the receptor or ligand is selected from human TGF-βRII, VEGFR, c-MET, EGFR, SIRPα or TIGIT; or
(10) the first binding domain targets and binds to Claudin 18.2, HER-2, mesothelin, BCMA, SSTR2, GPRC5D, PSMA, FCRH5, CD33, CD123, CD20, A33, CEA, CD28, DLL3, EGFR, VEGFR, VEGFR2, Nectin-4, FGFR, c-MET, RANKL, PDGF, PDGFR, PDGFRα, DLL4, Ang-1 or Ang-2, and the receptor or ligand is selected from human CTLA-4, CD80, CD86, TGF-βRII, VEGFR, FGL1, LAG3, 4-1BB, OX40, CD27, CD28, CD70, c-MET, SIRPα, TIGIT, CSF1R, CXCR2, CXCR3, CXCR4, EGFR or ICOS.

8. The fusion protein according to any one of claims 1-7, wherein the fragment of the receptor or ligand comprises a fragment of the extracellular region and a fragment of the binding domain of the receptor or ligand.

9. The fusion protein according to claim 7, wherein the first binding domain targets and binds to PD-L1; the second binding domain is a fragment of human TGF-βRII; or the first binding domain targets and binds to PD-1, and the second binding domain is selected from human CD80 ECD, CD80 IgV region, human CD80 IgVIgC, VEGFR1 ECD, VEGFR2 ECD or a combination of the second extracellular region of VEGFR1 and the third extracellular region of VEGFR2; or the first binding domain targets and binds to Claudin 18.2, HER-2 or EGFR, and the second binding domain is selected from human CD80 ECD, CD80 IgV region, human CD80 IgVIgC, VEGFR1 ECD, VEGFR2 ECD or a combination of the second extracellular region of VEGFR1 and the third extracellular region of VEGFR2.

10. The fusion protein according to claim 9, wherein the second binding domain is selected from:
(1) human TGF-βRII comprising the sequence as shown in SEQ ID NO: 31, 131 or 132, or a sequence having an identity of greater than 80%, 85%, 90%, 95%, 96%, 97%, 98%, 99% compared with the sequence as shown in SEQ ID NO: 31, 131 or 132, or an amino acid sequence having 1, 2, 3, 4, 5, 6, 7, 8, 9, or 10 amino acid substitutions or deletions on the sequence as shown in SEQ ID NO: 31, 131 or 132; or
(2) CD80 ECD comprising the sequence as shown in SEQ ID NO: 32 or 133, or a sequence having an identity of greater than 80%, 85%, 90%, 95%, 96%, 97%, 98%, 99% compared with the sequence as shown in SEQ ID NO: 32 or 133, or an amino acid sequence having 1, 2, 3, 4, 5, 6, 7, 8, 9, or 10 amino acid substitutions or deletions on the sequence as shown in SEQ ID NO: 32 or 133; or
(3) a combination of the second extracellular region of VEGFR1 and the third extracellular region of VEGFR2, comprising the sequence as shown in SEQ ID NO: 33, or a sequence having an identity of greater than 80%, 85%, 90%, 95%, 96%, 97%, 98%, 99% compared with the sequence as shown in SEQ ID NO: 33, or an amino acid sequence having 1, 2, 3, 4, 5, 6, 7, 8, 9, or 10 amino acid substitutions or deletions on the sequence as shown in SEQ ID NO: 33.

11. The fusion protein according to claim 9 or 10, wherein the first binding domain targets and binds to PD-L1, and the CDRs of the heavy chain variable region and/or the CDRs of the light chain variable region in the Fab of the IgG have the same CDR sequence as the antibody defined by the following sequence, or have 1-2 amino acid substitutions on the CDRs of the antibody defined by the following sequence, wherein the antibody is defined as follows:
(1) the amino acid sequence of the heavy chain variable region is shown in SEQ ID NO: 66; and/or
(2) the amino acid sequence of the light chain variable region is shown in SEQ ID NO: 67.

12. The fusion protein according to claim 11, wherein the CDRs of the heavy chain variable region and/or the CDRs of the light chain variable region in the Fab of the IgG are as follows:
(1) for the heavy chain variable region, the amino acid sequence of CDR1 is selected from SEQ ID NOs: 1-5 and amino acid sequences having 1 or 2 amino acid substitutions on SEQ ID NOs: 1-5; the amino acid sequence of CDR2 is selected from SEQ ID NOs: 6-10 and amino acid sequences having 1 or 2 amino acid substitutions on SEQ ID NOs: 6-10; the amino acid sequence of CDR3 is selected from SEQ ID NOs: 11-15 and amino acid sequences having 1 or 2 amino acid substitutions on SEQ ID NOs: 11-15; and/or
(2) for the light chain variable region, the amino acid sequence of CDR1 is selected from SEQ ID NOs: 16-20 and amino acid sequences having 1 or 2 amino acid substitutions on SEQ ID NOs: 16-20; the amino acid sequence of CDR2 is selected from SEQ ID NOs: 21-25 and amino acid sequences having 1 or 2 amino acid substitutions on SEQ ID NOs: 21-25; the amino acid sequence of CDR3 is selected from SEQ ID NOs: 26-30 and amino acid sequences having 1 or 2 amino acid substitutions on SEQ ID NOs: 26-30.

13. The fusion protein according to claim 12, wherein:
(1) the amino acid sequences of CDRs 1-3 of the heavy chain variable region are SEQ ID NOs: 1, 6, 11, or an amino acid sequence having 1 or 2 amino acid substitutions on SEQ ID NOs: 1, 6, 11; and/or an amino acid sequence of CDRs 1-3 of the light chain variable region are SEQ ID NOs: 16, 21, 26, or an amino acid sequence having 1 or 2 amino acid substitutions on SEQ ID NOs: 16, 21, 26; or
(2) the amino acid sequences of CDRs 1-3 of the heavy chain variable region are SEQ ID NOs: 2, 7, 12, or an amino acid sequence having 1 or 2 amino acid substitutions on SEQ ID NOs: 2, 7, 12; and/or an amino acid sequence of CDRs 1-3 of the light chain variable region are SEQ ID NOs: 17, 22, 27, or an amino acid sequence having 1 or 2 amino acid substitutions on SEQ ID NOs: 17, 22, 27; or
(3) the amino acid sequences of CDRs 1-3 of the heavy chain variable region are SEQ ID NOs: 3, 8, 13, or an amino acid sequence having 1 or 2 amino acid substitutions on SEQ ID NOs: 3, 8, 13; and/or an amino acid sequence of CDRs 1-3 of the light chain variable region are SEQ ID NOs: 18, 23, 28, or an amino acid sequence having 1 or 2 amino acid substitutions on SEQ ID NOs: 18, 23, 28; or
(4) the amino acid sequences of CDRs 1-3 of the heavy chain variable region are SEQ ID NOs: 4, 9, 14, or an amino acid sequence having 1 or 2 amino acid substitutions on SEQ ID NOs: 4, 9, 14; and/or an amino acid sequence of CDRs 1-3 of the light chain variable region are SEQ ID NOs: 19, 24, 29, or an amino acid sequence having 1 or 2 amino acid substitutions on SEQ ID NOs: 19, 24, 29; or
(5) the amino acid sequences of CDRs 1-3 of the heavy chain variable region are SEQ ID NOs: 5, 10, 15, or an amino acid sequence having 1 or 2 amino acid substitutions on SEQ ID NOs: 5, 10, 15; and/or an amino acid sequence of CDRs 1-3 of the light chain variable region are SEQ ID NOs: 20, 25, 30, or an amino acid sequence having 1 or 2 amino acid substitutions on SEQ ID NOs: 20, 25, 30.

14. The fusion protein according to claim 13, wherein the amino acid sequences of CDRs 1-3 of the heavy chain variable region are SEQ ID NOs: 3, 8, 13; and/or an amino acid sequence of CDRs 1-3 of the light chain variable region are SEQ ID NOs: 18, 23, 28.

15. The fusion protein according to claim 14, wherein:
(1) the heavy chain variable region has a sequence as shown in SEQ ID NO: 66, or a sequence that has the same CDRs 1-3 as SEQ ID NO: 66 and has an identity of greater than 80%, 85%, 90%, 95%, 96%, 97%, 98%, 99% compared with SEQ ID NO: 66; and/or
(2) the heavy chain variable region has a sequence as shown in SEQ ID NO: 67, or a sequence that has the same CDRs 1-3 as SEQ ID NO: 67 and has an identity of greater than 80%, 85%, 90%, 95%, 96%, 97%, 98%, 99% compared with SEQ ID NO: 67.

16. The fusion protein according to claim 15, wherein:
(1) the heavy chain of the fusion protein has an amino acid sequence as shown in SEQ ID NO: 72, or a sequence having an identity of greater than 80%, 85%, 90%, 95%, 96%, 97%, 98%, 99% compared with SEQ ID NO: 72;
(2) the light chain of the fusion protein has an amino acid sequence as shown in SEQ ID NO: 73, or a sequence having an identity of greater than 80%, 85%, 90%, 95%, 96%, 97%, 98%, 99% compared with SEQ ID NO: 73.

17. The fusion protein according to claim 16, wherein:
(1) the amino acid sequence of the heavy chain of the fusion protein has 1-15 amino acid site mutations or has an alternative peptide linker compared with SEQ ID NO: 72;
(2) the amino acid sequence of the light chain of the fusion protein has 1-10 amino acid site mutations compared with SEQ ID NO: 73.

18. The fusion protein according to claim 9, wherein the first binding domain targets and binds to PD-1, and the CDRs of the heavy chain variable region and/or the CDRs of the light chain variable region in the Fab of the IgG have the same CDR sequence as the antibody defined by the following sequence, or have 1-2 amino acid substitutions on the CDRs of the antibody defined by the following sequence, wherein the antibody is defined as follows:
(1) the amino acid sequence of the heavy chain variable region is shown in SEQ ID NO: 64; and/or
(2) the amino acid sequence of the light chain variable region is shown in SEQ ID NO: 65.

19. The fusion protein according to claim 18, wherein the CDRs of the heavy chain variable region and/or the CDRs of the light chain variable region in the Fab of the IgG are as follows:
(1) for the heavy chain variable region, the amino acid sequence of CDR1 is selected from SEQ ID NOs: 34-38 and an amino acid sequence having 1 or 2 amino acid substitutions on SEQ ID NOs: 34-38; the amino acid sequence of CDR2 is selected from SEQ ID NOs: 39-43 and an amino acid sequence having 1 or 2 amino acid substitutions on SEQ ID NOs: 39-43; the amino acid sequence of CDR3 is selected from SEQ ID NOs: 44-48 and an amino acid sequence having 1 or 2 amino acid substitutions on SEQ ID NOs: 44-48; and/or
(2) for the light chain variable region, the amino acid sequence of CDR1 is selected from SEQ ID NOs: 49-53 and an amino acid sequence having 1 or 2 amino acid substitutions on SEQ ID NOs: 49-53; the amino acid sequence of CDR2 is selected from SEQ ID NOs: 54-58 and an amino acid sequence having 1 or 2 amino acid substitutions on SEQ ID NOs: 54-58; the amino acid sequence of CDR3 is selected from SEQ ID NOs: 59-63 and an amino acid sequence having 1 or 2 amino acid substitutions on SEQ ID NOs: 59-63.

20. The fusion protein according to claim 19, wherein:
(1) the amino acid sequences of CDRs 1-3 of the heavy chain variable region are SEQ ID NOs: 34, 39, 44, or an amino acid sequence having 1 or 2 amino acid substitutions on SEQ ID NOs: 34, 39, 44; and/or an amino acid sequence of CDRs 1-3 of the light chain variable region are SEQ ID NOs: 49, 54, 59, or an amino acid sequence having 1 or 2 amino acid substitutions on SEQ ID NOs: 49, 54, 59; or
(2) the amino acid sequences of CDRs 1-3 of the heavy chain variable region are SEQ ID NOs: 35, 40, 45, or an amino acid sequence having 1 or 2 amino acid substitutions on SEQ ID NOs: 35, 40, 45; and/or an amino acid sequence of CDRs 1-3 of the light chain variable region are SEQ ID NOs: 50, 55, 60, or an amino acid sequence having 1 or 2 amino acid substitutions on SEQ ID NOs: 50, 55, 60; or
(3) the amino acid sequences of CDRs 1-3 of the heavy chain variable region are SEQ ID NOs: 36, 41, 46, or an amino acid sequence having 1 or 2 amino acid substitutions on SEQ ID NOs: 36, 41, 46; and/or an amino acid sequence of CDRs 1-3 of the light chain variable region are SEQ ID NOs: 51, 56, 61, or an amino acid sequence having 1 or 2 amino acid substitutions on SEQ ID NOs: 51, 56, 61; or
(4) the amino acid sequences of CDRs 1-3 of the heavy chain variable region are SEQ ID NOs: 37, 42, 47, or an amino acid sequence having 1 or 2 amino acid substitutions on SEQ ID NOs: 37, 42, 47; and/or an amino acid sequence of CDRs 1-3 of the light chain variable region are SEQ ID NOs: 52, 57, 62, or an amino acid sequence having 1 or 2 amino acid substitutions on SEQ ID NOs: 52, 57, 62; or
(5) the amino acid sequences of CDRs 1-3 of the heavy chain variable region are SEQ ID NOs: 38, 43, 48, or an amino acid sequence having 1 or 2 amino acid substitutions on SEQ ID NOs: 38, 43, 48; and/or an amino acid sequence of CDRs 1-3 of the light chain variable region are SEQ ID NOs: 53, 58, 63, or an amino acid sequence having 1 or 2 amino acid substitutions on SEQ ID NOs: 53, 58, 63.

21. The fusion protein according to claim 20, wherein the amino acid sequences of CDRs 1-3 of the heavy chain variable region are SEQ ID NOs: 36, 41, 46; and/or an amino acid sequence of CDRs 1-3 of the light chain variable region are SEQ ID NOs: 51, 56, 61.

22. The fusion protein according to claim 21, wherein:
(1) the heavy chain variable region has a sequence as shown in SEQ ID NO: 64, or a sequence that has the same CDRs 1-3 as SEQ ID NO: 64 and has an identity of greater than 80%, 85%, 90%, 95%, 96%, 97%, 98%, 99% compared with SEQ ID NO: 64; and/or
(2) the light chain variable region has a sequence as shown in SEQ ID NO: 65, or a sequence that has the same CDRs 1-3 as SEQ ID NO: 65 and has an identity of greater than 80%, 85%, 90%, 95%, 96%, 97%, 98%, 99% compared with SEQ ID NO: 65.

23. The fusion protein according to claim 22, wherein:
(1) the heavy chain of the fusion protein has an amino acid sequence as shown in SEQ ID NO: 68, or a sequence having an identity of greater than 80%, 85%, 90%, 95%, 96%, 97%, 98%, 99% compared with SEQ ID NO: 68;
(2) the light chain of the fusion protein has an amino acid sequence as shown in SEQ ID NO: 69, or a sequence having an identity of greater than 80%, 85%, 90%, 95%, 96%, 97%, 98%, 99% compared with SEQ ID NO: 69.

24. The fusion protein according to claim 23, wherein:
(1) the amino acid sequence of the heavy chain of the fusion protein has 1-15 amino acid site mutations or has an alternative peptide linker compared with SEQ ID NO: 68;
(2) the amino acid sequence of the light chain of the fusion protein has 1-10 amino acid site mutations compared with SEQ ID NO: 69.

25. The fusion protein according to claim 22, wherein:
(1) the heavy chain of the fusion protein has an amino acid sequence as shown in SEQ ID NO: 70, or a sequence having an identity of greater than 80%, 85%, 90%, 95%, 96%, 97%, 98%, 99% compared with SEQ ID NO: 70;
(2) the light chain of the fusion protein has an amino acid sequence as shown in SEQ ID NO: 71, or a sequence having an identity of greater than 80%, 85%, 90%, 95%, 96%, 97%, 98%, 99% compared with SEQ ID NO: 71.

26. The fusion protein according to claim 25, wherein:
(1) the amino acid sequence of the heavy chain of the fusion protein has 1-15 amino acid site mutations or has an alternative peptide linker compared with SEQ ID NO: 70;
(2) the amino acid sequence of the light chain of the fusion protein has 1-10 amino acid site mutations compared with SEQ ID NO: 71.

27. The fusion protein according to any one of claims 1-10, wherein the IgG is Atezolizumab, Avelumab, Durvalumab, Nivolumab, Pembrolizumab, Cemiplimab or Ipilimumab.

28. The fusion protein according to any one of claims 1-27, wherein the C-terminus or/and N-terminus of the second binding domain has a peptide linker, and the peptide linker consists of 2-30 amino acids.

29. The fusion protein of claim 28, wherein the peptide linker is:
(1) (GGGGS)n; or
(2) AKTTPKLEEGEFSEAR (SEQ ID NO: 80); or
(3) AKTTPKLEEGEFSEARV (SEQ ID NO: 81); or
(4) AKTTPKLGG (SEQ ID NO: 82); or
(5) SAKTTPKLGG (SEQ ID NO: 83); or
(6) SAKTTP (SEQ ID NO: 84); or
(7) RADAAP (SEQ ID NO: 85); or
(8) RADAAPTVS (SEQ ID NO: 86); or
(9) RADAAAAGGPGS (SEQ ID NO: 87); or
(10) RADAAAA (SEQ ID NO: 88); or
(11) SAKTTPKLEEGEFSEARV (SEQ ID NO: 89); or
(12) ADAAP (SEQ ID NO: 90); or
(13) DAAPTVSIFPP (SEQ ID NO: 91); or
(14) TVAAP (SEQ ID NO: 92); or
(15) TVAAPSVFIFPP (SEQ ID NO: 93); or
(16) QPKAAP (SEQ ID NO: 94); or
(17) QPKAAPSVTLFPP (SEQ ID NO: 95); or
(18) AKTTPP (SEQ ID NO: 96); or
(19) AKTTPPSVTPLAP (SEQ ID NO: 97); or
(20) AKTTAP (SEQ ID NO: 98); or
(21) AKTTAPSVYPLAP (SEQ ID NO: 99); or
(22) ASTKGP (SEQ ID NO: 100); or
(23) ASTKGPSVFPLAP (SEQ ID NO: 101); or
(24) GENKVEYAPALMALS (SEQ ID NO: 102); or
(25) GPAKELTPLKEAKVS (SEQ ID NO: 103); or
(26) GHEAAAVMQVQYPAS (SEQ ID NO: 104); or
(27) GGGGSGGGGSGGGGSA (SEQ ID NO: 105),
wherein n is equal to 1, 2, 3 or 4.

30. The fusion protein according to any one of claims 1-29, wherein the size of the inserted second binding domain does not exceed 300 amino acids.

31. The fusion protein according to any one of claims 1-30, wherein the insertion site of the second binding domain is located in a middle front part of the hinge region, and the insertion site does not affect the formation of disulfide bonds of the immunoglobulin.

32. The fusion protein according to claim 31, wherein the middle front part of the hinge region refers to the part before 231A.

33. The fusion protein according to any one of claims 1-32, wherein part of the amino acids in the hinge region before and after the insertion site are substituted or deleted.

34. The fusion protein according to claim 33, wherein the hinge region contains D221G and/or C220V mutations.

35. The fusion protein according to any one of claims 1-34, wherein the IgG is selected from mammalian IgG, humanized IgG, and human IgG, and the mammal includes mouse, rat and rabbit; preferably, the IgG is IgG1, IgG2, IgG3 or IgG4.

36. The fusion protein according to any one of claims 1-35, wherein the Fc region of the fusion protein is aglycosylated or deglycosylated, or has reduced fucosylation or is afucosylated.

37. An isolated polynucleotide encoding the fusion protein according to any one of claims 1-36.

38. A nucleic acid construct comprising the polynucleotide according to claim 37, preferably the nucleic acid construct is a vector.

39. A host cell comprising the polynucleotide according to claim 37 or the nucleic acid construct or the vector according to claim 38, preferably the cell is a prokaryotic cell, eukaryotic cell, yeast cell, mammal cell, E. coli cell or CHO cell, NS0 cell, Sp2/0 cell, or BHK cell.

40. A pharmaceutical composition comprising the fusion protein according to any one of claims 1-36 and a pharmaceutically acceptable carrier.

41. A method for treating tumor or cancer, comprising the step of administering the fusion protein according to any one of claims 1-36 or the pharmaceutical composition according to claim 40 to a subject in need of a treatment or relief.

42. Use of the fusion protein according to any one of claims 1-36, the polynucleotide according to claim 37, the nucleic acid construct or the vector according to claim 38, or the pharmaceutical composition according to claim 40 in the manufacture of a medicament for the treatment or prevention of a tumor or cancer.

43. The use according to claim 42, wherein the tumor or cancer includes a solid tumor or a non-solid tumor.

44. A diagnostic kit comprising the fusion protein according to any one of claims 1-36.

45. A method of manufacturing the fusion protein according to any one of claims 1-36, comprising culturing the host cell according to claim 39 under a condition allowing the expression of the nucleic acid construct according to claim 38, and recovering the produced fusion protein from the culture.
